# EUROPEAN PATENT SPECIFICATION

(11) **EP 3 345 917 B1**
(45) Date of publication and mention of the grant of the patent: **17.11.2021**
(21) Application number: 17150353.5
(22) Date of filing: 05.01.2017
(51) Int. Cl.: C07K 7/02

(54) **ANTIPROTOZOAL COMPOUNDS**
ANTIPROTOZOENVERBINDUNG
COMPOSÉ ANTIPROTOZOAIRE

(43) Date of publication of application: 11.07.2018
(73) Proprietor: Bacoba AG, 4051 Basel (CH)
(72) Inventor: Agnello, Stefano, 8820 Wädenswil (CH); Adams, Michael, 8610 Uster (CH); Kaiser, Marcel, 4324 Obermumpf (CH); Mäser, Pascal, 4153 Reinach (CH); Urban, Alexander, 1030 Wien (AT); Raguz, Luka, 8207 Schaffhausen (CH); Riedl, Rainer, 8832 Wilen b. Wollerau (CH)
(74) Representative: Latscha Schöllhorn Partner AG

(56) References cited:
- AKI ISHIYAMA ET AL: "In vitro and in vivo antitrypanosomal activities of three peptide antibiotics: leucinostatin A and B, alamethicin I and tsushimycin", THE JOURNAL OF ANTIBIOTICS, vol. 62, no. 6, 1 June 2009 (2009-06-01), pages 303-308, XP055344637, GB ISSN: 0021-8820, DOI: 10.1038/ja.2009.32
- THOMAS DEGENKOLB ET AL: "Formation of New Lipoaminopeptides, Acremostatins A, B, and C, by Co-cultivation of Acremonium sp. Tbp-5 and Mycogone rosea DSM 12973", BIOSCIENCE BIOTECHNOLOGY BIOCHEMISTRY., vol. 66, no. 4, 22 January 2002 (2002-01-22), pages 883-886, XP055344837, TOKYO, JAPAN ISSN: 0916-8451, DOI: 10.1271/bbb.66.883
- ANA FLÁVIA CANOVAS MARTINEZ ET AL: "Liquid chromatography-tandem mass spectrometry characterization of five new leucinostatins produced by Paecilomyces lilacinus CG-189", THE JOURNAL OF ANTIBIOTICS, vol. 68, no. 3, 1 March 2015 (2015-03-01), pages 178-184, XP055344838, GB ISSN: 0021-8820, DOI: 10.1038/ja.2014.120
- MICHAELA STACH ET AL: "Improved Strategy for the Synthesis of the Anticancer Agent Culicinin D", EUROPEAN JOURNAL OF ORGANIC CHEMISTRY, vol. 2015, no. 28, 18 October 2015 (2015-10-18), pages 6341-6350, XP055344854, DE ISSN: 1434-193X, DOI: 10.1002/ejoc.201500872
- MARCEL KAISER ET AL: "Antiprotozoal Activity Profiling of Approved Drugs: A Starting Point toward Drug Repositioning", PLOS ONE, vol. 10, no. 8, 13 August 2015 (2015-08-13), page e0135556, XP055344644, DOI: 10.1371/journal.pone.0135556
- KAZUHIKO OTOGURO ET AL: "In Vitro Antimalarial Activities of the Microbial Metabolites.", THE JOURNAL OF ANTIBIOTICS, vol. 56, no. 3, 1 January 2003 (2003-01-01), pages 322-324, XP055344799, GB ISSN: 0021-8820, DOI: 10.7164/antibiotics.56.322

## Description

The present invention relates to compounds fo formula (I), pharmaceutical compositions comprising the same and the use of the compounds of formula (I) in methods of treatment of protozoan diseases, such as malaria, Human African Trypanosomiasis, Chagas disease, and leishmaniasis, and preferably in methods of treatment of Chagas disease or leishmaniasis.

### RELATED ART

Protozoan infections are parasitic diseases caused by organisms formerly classified in the Kingdom Protozoa. Protozoa are one of the three main classes of pathogens that cause diseases in humans. They are single-celled organism, and can only be seen under a microscope. When they invade a human they are able to multiply easily, which causes them to be at a great advantage and puts humans at a disadvantage. This helps them survive in the human body and causes a serious infection even with the arrival of a single protozoon. Protozoan infections include in particular the four major protozoan diseases, namely malaria, human African trypanosomiasis, Chagas disease, and leishmaniasis.

Leishmaniasis is caused by parasites of the genus Leishmania. These parasites are found mainly in southern Europe, the tropics and subtropics. The disease is transmitted by the bite of a female sandfly (genus *Phlebotomus)* during a blood meal on its host. Over 20 species and subspecies of *Leishmania* infect mammals, such as humans, causing a different spectrum of symptoms including *Leishmania donovani, Leishmania Mexicana* and *Leishmania tropica.*

Leishmaniasis occurs in 3 main forms: cutaneous leishmaniasis (CL), mucocutaneous leishmaniasis (MCL) and visceral leishmaniasis (VL), also called Kala Azar. CL and MCL infections can lead to severe scarring and permanent disfigurement. In patients with a MCL infection, the disease can destroy soft tissue in the mouth and nose, drastically deforming the face. VL is the most serious form and is caused by the parasites *Leishmania donovani* and *Leishmania infantum.* Patients who develop VL can die within months unless they receive treatment. The two main therapies for VL are the antimony derivatives sodium stibogluconate (Pentostam®) and meglumine antimoniate (Glucantim®) or Amphotericin B® for VL acquired in India (Sundar S et al. Clin. Infect. Dis. 31(4):1104-1107; Thakur CP et al. Indian J. Med. Res. 120(3): 166-172; Thakur CP et al. Trans. R. Soc. Trop. Med. Hyg. 93(3): 319-323; Thakur CP et al. Trans. R. Soc. Trop. Med. Hyg. 90(3):319-322; Sundar S et al. Clin. Infect. Dis. 42(5):608-613). Sodium stibogluconate has been 15 used for about 70 years and resistance to this drug is a growing problem. In addition, the treatment is relatively long and painful, and can cause undesirable and toxic side effects. Therefore, identification of alternative candidate compounds with anti-Leishmanial activities is of utmost urgency, and in particular there is a critical need to develop new therapeutic agents that have low cytotoxicity but high effectiveness against *Leishmania* parasites.

Chagas disease (also called American Trypanosomiasis) is another human parasitic disease that is endemic amongst poor populations on the American continent. The disease is caused by the protozoan parasite *Trypanosoma cruzi,* which is transmitted to humans by bloodsucking insects. The human disease occurs in two stages: the acute stage, which occurs shortly after infection and the chronic stage, which can develop over many years. Chronic infections result in various neurological disorders, including dementia, damage to the heart muscle and sometimes dilation of the digestive tract, as well as weight loss. Untreated, the chronic disease is often fatal. The drugs currently available for treating Chagas disease are Nifurtimox® and benznidazole®. However, problems with these current therapies include their diverse side effects, the length of treatment, and the requirement for medical supervision during treatment. Furthermore, treatment is really only effective when given during the acute stage of the disease. Resistance to the two frontline drugs has already occurred. The antifungal agent Amphotericin b has been proposed as a second-line drug, but this drug is costly and relatively toxic.

Human African Trypanosomiasis (HAT), also known as sleeping sickness, is a vector-borne parasitic disease caused by the protozoa. The parasites concerned are protozoa belonging to the *Trypanosoma* Genus. They are transmitted to humans by tsetse fly (*Glossina* Genus) bites which have acquired their infection from human beings or from animals harboring the human pathogenic parasites. Two sub-species of trypanosomes, *Trypanosoma brucei rhodesiense* (found in eastern and southern Africa) and *Trypanosoma brucei gambiense* (found in west and central Africa) infect humans and animals. Another sub-species, *Trypanosoma brucei brucei,* infect cattle, causing Nagana disease, which devastates livestock production and causes massive economic losses. *Trypanosoma brucei gambiense* currently accounts for over 98% of reported cases of sleeping sickness and causes a chronic infection. A person can be infected for months or even years without major signs or symptoms of the disease. When more evident symptoms emerge, the patient is often already in an advanced disease stage where the central nervous system is affected. *Trypanosoma brucei rhodesiense* represents under 2% of reported cases and causes an acute infection. First signs and symptoms are observed a few months or weeks after infection. The disease develops rapidly and invades the central nervous system. Currently, the drugs pentamidine® and suramin® are used in the early stage of *T. b. gambiense* and *T. b. rhodesiense* infections, and melarsoprol® is used in the late stage, whereas eflornithine® is only used in the late stage of *T. b. gambiense* infections. These drugs are old and highly unsatisfactory, as they cannot be given orally and can be dangerous because of their severe toxicity. Moreover, drug resistance in trypanosomes is increasing and treatment failures are becoming more common. Therefore, there is an urgent need for new antitrypanosomal drugs that are more effective and safer.

Malaria is an infectious disease caused by four protozoan parasites: *Plasmodium falciparum; Plasmodium vivax; Plasmodium ovale;* and *Plasmodium malaria.* These four parasites are typically transmitted by the bite of an infected female Anopheles mosquito. Malaria symptoms include headache, chills, tremors, aches and shaking. Malaria is a problem in many parts of the world and over the last few decades the malaria burden has steadily increased. An estimated 1-3 million people die every year from malaria - mostly children under the age of 5. This increase in malaria mortality is due in part to the fact that *Plasmodium falciparum,* the deadliest malaria parasite, has acquired resistance against nearly all available antimalarial drugs, with the exception of the artemisinin derivatives.

Leucinostatins are lipophilic peptide antibiotics produced as microbial metabolites. The nonapeptide leucinostatin A is the major antibiotic, cytotoxic, phytotoxic component isolated, as hydrochloride salt, from cultures filtrates of *Paecilomyces marquandii* or *Paecilomyces lilacinus.* The chemical structure of leucinostatin A has been worked out by several groups. It turned out to be composed by the unsaturated fatty acid (4*S*,2*E*)-4-methylhex-2-enoic acid (MeHA) at the N-terminal; the (2*S*)-N¹,N¹-dimethylpropane-1,2-diamine (DPD) at the C-terminal; and of the following nine amino acids: *cis*-4-methyl-L-proline (MePro), (2*S*,4*S*,6*S*)-2-amino-6-hydroxy-4-methyl-8-oxodecanoic acid (AHMOD), *threo*-β-hydroxy-L-leucine, three α-aminoisobutyric acids (Aib), two L-leucines (Leu) and β-alanine. Additional minor metabolites leucinostatin B, C, D, F, H and K have been isolated from the same submerged cultures and the structures are closely related to that of leucinostatin A (Vertuani et al. J. Antibiotics 1995, 48(3):254-260; Isogai et al. Biosci. Biotech Biochem. 1992, 56(7): 1079-1085). In the course of screening studies to discover antimalarial antibiotics from the soil microorganism and antibiotics library of the Kitasato Institute for Life Sciences, Omura et al. discovered that leucinostatin A which was purified from the culture broth of a fungal strain showed antimalarial activities, in particular *in vitro* activities against *Plasmodium falciparum* strains (Otogoro et al. J. Antibiotics, 2003, 3, 322-324). In further studies screening for antitrypanosomal compounds from said soil microorganisms and antibiotics library of the Kitasato Institute for Life Sciences, Omura et al. discovered that leucinostatin A and B isolated from a culture broth of *Paecilomyces sp.* show *in vitro* and *in vivo* antitrypanosomal activities, in particular against *Trypanosoma brucei* (Ishiyama et al. J. Antibiotics, 2009, 62, 343).

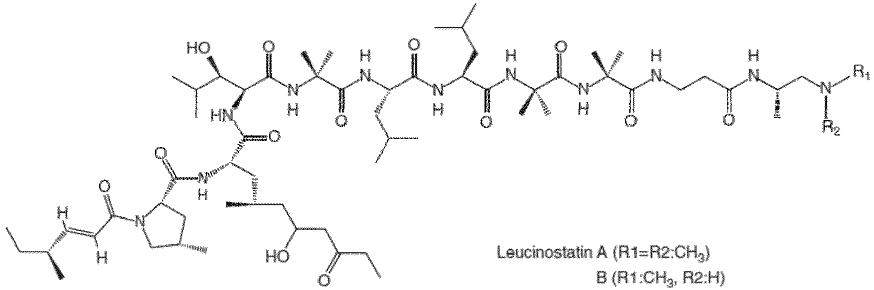

Recently, Khare et al reported a novel selective inhibitor of the kinetoplastid proteasome (GNF6702) which cleared *Trypanosoma cruzi, Leishmania* spp. and *Trypanosoma brucei* spp. parasites from mice in models of infection of Chagas disease, leishmaniasis and sleeping sickness (Khare et al. Nature 537 (2016), 229-233).

However, and in particular in light of the current treatment possibilities for protozoan infections and their variable efficiencies, serious toxicities and emerging drug resistances, there is still an urgent need for new and more effective drugs to treat said various and neglected tropical diseases that take the heaviest toll on the developing world (K. Katsuno et al. Nature Reviews, 2015, 14:751-758).

### SUMMARY OF THE INVENTION

We have surprisingly found that the inventive compounds are very potent inhibitors of protozoan parasites including *Leishmania* spp., *Trypanosoma cruzi, Trypanosoma brucei,* and *Plasmodium falciparum,* and in particular of *Leishmania* spp. and *Trypanosoma cruzi,* and, thus, represents very active compounds for the treatment of protozoan diseases, such as malaria, Human African Trypanosomiasis, Chagas disease, and leishmaniasis, and preferably for Chagas disease, and leishmaniasis. The high potency of the novel class of inventive compounds is particularly noteworthy when compared to the compounds of the prior art currently in use or in development. The latter is particularly true for the potency of the novel class of inventive compounds for the treatment of leishmaniasis and Chagas disease.

Furthermore, it has been surprisingly found that the inventive compounds are not only very potent inhibitors of protozoan parasites but also have the ability to enter the parasites host cells very efficiently and target the parasites inside said host cells without harming the same. This represents a further unique quality additionally setting the inventive compounds apart from the compounds currently in use or development.

Moreover, we have surprisingly found that preferred compounds of the present invention maintain its high biological activity despite the removal of one or several stereocenters leading to more simplified and more economic syntheses of the inventive compounds without sacrificing biological activity.

In a first aspect, the present invention provides for a compound of formula (I) 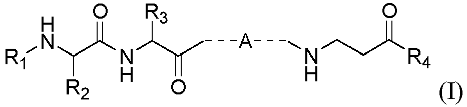 wherein said compound of formula (I) is a compound of any one of the formula (II) to (IV) 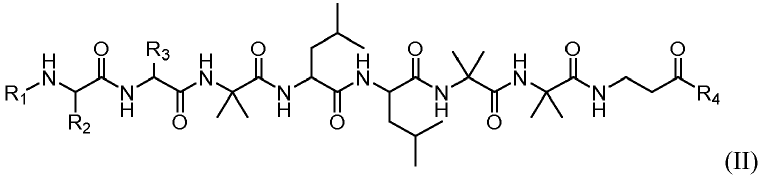 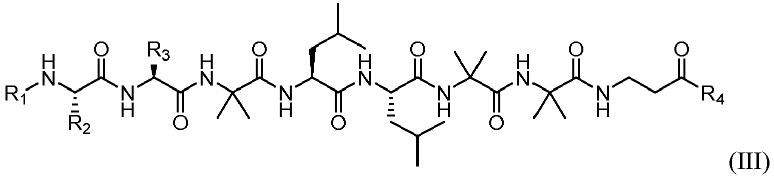 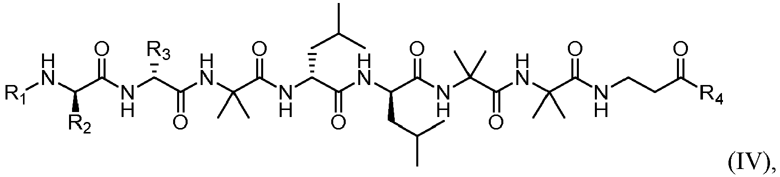 and wherein
R₁ is 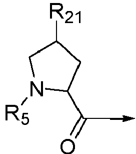 preferably 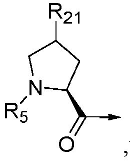 wherein
   R₅ is C(O)-R₈, wherein R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl, pyrrolidinyl, benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen preferably fluorine, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; and wherein
   the arrow indicates the attachment to the NH-moiety depicted in formula (I);
R₂ is selected from 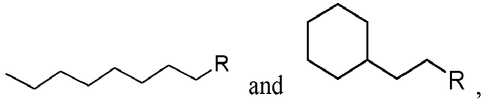 wherein
R indicates the attachment to the CH-moiety depicted in formula (I);
R₃ is selected from 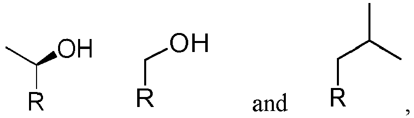 wherein R indicates the attachment to the CH-moiety depicted in formula (I);
R₄ is 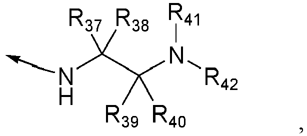 wherein
   R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
   R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅; wherein the arrow indicates the attachment to the C(O)-moiety depicted in formula (I);
and pharmaceutically acceptable salts of said compound of formula (I).

In a further aspect, the present invention provides for a compound of formula (I) for use in a method of treatment of a protozoan disease, wherein preferably said protozoan disease is selected from malaria, human African trypanosomiasis, Chagas disease or leishmaniasis, and wherein further preferably said protozoan disease is Chagas disease or leishmaniasis, 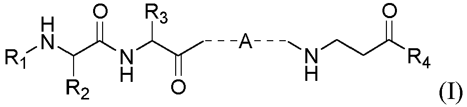 wherein said compound of formula (I) is a compound of any one of the formula (II) to (IV) 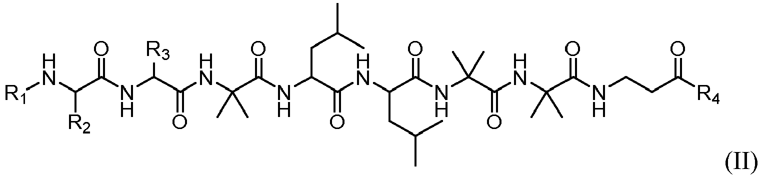 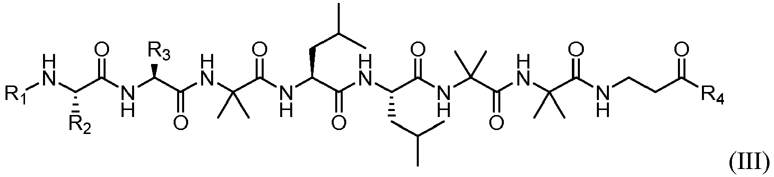 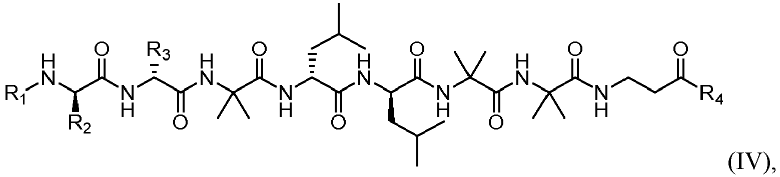 and wherein
R₁ is 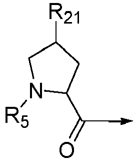 preferably 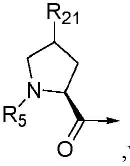 wherein
   R₅ is C(O)-R₈, wherein
      R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl, pyrrolidinyl, benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen preferably fluorine, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; and wherein
   the arrow indicates the attachment to the NH-moiety depicted in formula (I); R₂ is selected from 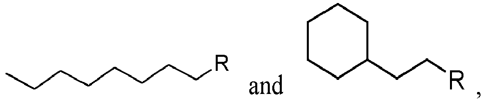
   wherein
R indicates the attachment to the CH-moiety depicted in formula (I);
R₃ is selected from 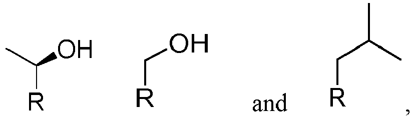 wherein R indicates the attachment to the CH-moiety depicted in formula (I);
R₄ is 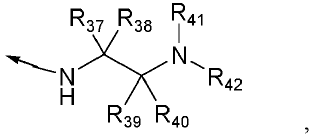 wherein
   R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
   R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅; wherein
   the arrow indicates the attachment to the C(O)-moiety depicted in formula (I);
and pharmaceutically acceptable salts of said compound of formula (I).

### DETAILED DESCRIPTION OF THE INVENTION

Unless defined otherwise, all technical and scientific terms used herein have the same meanings as commonly understood by one of ordinary skill in the art to which this invention belongs.

Each alkyl moiety either alone or as part of a larger group such as alkoxy, aminoalkyl or alkylene is a straight or branched chain and is preferably C₁-C₁₆alkyl, more preferably C₁-C₁₄alkyl. Examples include methyl, ethyl, *n*-propyl, prop-2-yl, *n*-butyl, but-2-yl, 2-methyl-prop-1-yl or 2-methyl-prop-2-yl. Examples of an alkoxy include methoxy, ethoxy, propoxy, *iso*-propoxy, *n*-butoxy, *sec*-butoxy, *tert*-butoxy, *n*-pentoxy, *neo*-pentoxy, *n*-hexoxy. Examples of aminoalkyl include aminomethyl, aminoethyl, dimethylaminomethyl, dimethylaminoethyl. As described herein, alkoxy may include further substitutents such as halogen atoms leading to haloalkoxy moieties.

Each alkylene moiety is a straight or branched chain and is, for example, -CH₂-, -CH₂-CH₂-, -CH(CH₃)-, -CH₂-CH₂-CH₂-, -CH(CH₃)-CH₂-, or -CH(CH₂CH₃)-.

Each haloalkyl moiety either alone or as part of a larger group such as haloalkoxy is an alkyl group substituted by one or more of the same or different halogen atoms. Haloalkyl moieties include for example 1 to 5 halo substituents, or 1 to 3 halo substituents. Examples include in particular fluoromethyl, difluoromethyl, trifluoromethyl, chlorodifluoromethyl and 2,2,2-trifluoro-ethyl.

The term "alkoxyalkyl" refers to a R-O-R' moiety in which the R and R' groups are alkyl groups as defined herein. Examples include methoxymethyl, methoxyethyl, ethoxyethyl and methoxypropyl.

Each alkenyl moiety either alone or as part of a larger group such as alkenyloxy or alkenylene is a straight or branched chain and is preferably C₂-C₁₆alkenyl, more preferably C₂-C₁₄alkenyl. Each moiety can be of either the (*E*)- or (*Z*)-configuration. Examples include vinyl and allyl. A compound of the present invention comprising an alkenyl moiety thus may include, if applicable, either said compound with said alkenyl moiety in its (*E*)-configuration, said compound with said alkenyl moiety in its (*Z*)-configuration and mixtures thereof in any ratio.

Each haloalkenyl moiety either alone or as part of a larger group such as haloalkenyloxy is an alkenyl group substituted by one or more of the same or different halogen atoms. Examples include,2-difluoro-vinyl and 1,2-dichloro-2-fluoro-vinyl. Haloalkenyl moieties include for example 1 to 5 halo substituents, or 1 to 3 halo substituents.

Each alkynyl moiety either alone or as part of a larger group such as alkynyloxy is a straight or branched chain and is preferably C₂-C₆alkynyl, more preferably C₂-C₄alkynyl. Examples are ethynyl and propargyl.

Halogen is fluorine, chlorine, bromine, or iodine.

The terms "carbocycle", "carbocyclyl", "carbocyclic ring" and "cycloalkyl", as interchangeably used herein, refer to a monovalent non-aromatic, saturated or partially unsaturated ring having 3 to 8 carbon atoms (C₃-C₈) as a monocyclic ring or 7 to 12 carbon atoms as a bi-, tri or tetracyclic ring as well as said cycloalkyl optionally substituted independently with one or more substituents, typically and preferably with one or two substituents as described below. "Cycloalkyl" also includes bicyclic radicals where cycloalkyl radicals are fused with a saturated, partially unsaturated ring, or aromatic carbocyclic or heterocyclic ring. Cycloalkyl groups are optionally substituted independently with one or more substituents, typically and preferably with one or two substituents, wherein said substituents are independently at each occurrence selected from C₁-C₄alkyl, halogen, CF₃, OR₁₀, NR₁₁R₁₂, C₆H₅, C₆H₅ substituted with halogen, oxo, C₁-C₃alkyl, OR₁₀, NR₁₁R₁₂, wherein R₁₀, R₁₁, R₁₂ are independently at each occurrence H, C₁-C₃alkyl. Examples of monocyclic carbocycles include, but are not limited to, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl and the like. A specific example of a tetracyclic ring is adamantanyl.

The terms "aryl", as used herein, refer to a monovalent aromatic hydrocarbon radical of 6-14 carbon atoms (C₆-C₁₄) derived by the removal of one hydrogen atom from a single carbon atom of a parent aromatic ring system as well as said aryl optionally substituted independently with one or more substituents, typically and preferably with one or two substituents as described below. Aryl includes bicyclic radicals comprising an aromatic ring fused to a saturated, partially unsaturated ring, or aromatic carbocyclic or heterocyclic ring. Aryl groups are optionally substituted independently with one or more substituents, typically and preferably with one or two substituents, wherein said substituents are independently at each occurrence selected from C₁-C₄alkyl, halogen, oxo, CF₃, OR₁₀, NR₁₁R₁₂, C₆H₅, C₆H₅ substituted with halogen, C₁-C₃alkyl, OR₁₀, NR₁₁R₁₂, wherein R₁₀, R₁₁, R₁₂ are independently at each occurrence H, C₁-C₃alkyl. Typical aryl groups include, but are not limited to, radicals derived from benzene (phenyl), substituted phenyls, naphthalene, anthracene, biphenyl, indenyl, indanyl, 1,2-dihydronapthalenyl, 1,2,3,4-tetrahydronaphthenyl and the like.

The terms "heterocycle", "heterocyclyl" and "heterocyclic ring" are used interchangeably herein and refer to a saturated or a partially unsaturated (i.e., having one or more double within the ring) carbocyclic radical of 3 to 14 ring atoms in which at least one ring atom is a heteroatom selected from nitrogen, oxygen and sulphur, the remaining ring atoms being C, where one or more ring atoms is optionally substituted independently with one or more substituents as described herein. A heterocycle may be a monocycle having 3 to 7 ring members (2 to 6 carbon atoms and 1 to 4 heteroatoms selected from N, O, and S) or a bicycle having 7 to 10 ring members (4 to 9 carbon atoms and 1 to 6 heteroatoms selected from N, O, and S). "Heterocyclyl" also includes radicals where heterocycle radicals are fused with a cycloalkyl ring, aryl or heteroaryl ring. Examples of heterocyclic rings include, but are not limited to, pyrrolidinyl, tetrahydrofuranyl, dihydrofuranyl, tetrahydrothienyl, tetrahydropyranyl, dihydropyranyl, tetrahydrothiopyranyl, piperidinyl, morpholinyl, thiomorpholinyl, thioxanyl, piperazinyl, homopiperazinyl, 2-pyrrolinyl, 3-pyrrolinyl, indolinyl, dioxanyl, dihyrooxazolyl, pyrazolinyl, pyrazolidinyl, imidazolinyl, imidazolidinyl, coumarinyl and the like. Heterocyclic rings are optionally substituted independently with one or more substituents, typically and preferably with one or two substituents, wherein said substituents are independently at each occurrence selected from C₁-C₄alkyl, halogen, oxo, CF₃, OR₁₀, NR₁₁R₁₂, C₆H₅, C₆H₅ substituted with halogen, C₁-C₃alkyl,OR₁₀, NR₁₁R₁₂, wherein R₁₀, R₁₁, R₁₂ are independently at each occurrence H, C₁-C₃alkyl.

The term "heteroaryl", as used herein, refers to a monovalent aromatic radical of 5-, 6-, or 7-membered rings and includes fused ring systems (at least one of which is heteroaryl) of 5-14 atoms, containing one or more heteroatoms independently selected from nitrogen, oxygen, and sulphur as well as said heteroaryl optionally substituted independently with one or more substituents, typically and preferably with one or two substituents as described below. Heteroaryl are optionally substituted independently with one or more substituents, typically and preferably with one or two substituents, wherein said substituents are independently at each occurrence selected from C₁-C₄alkyl, halogen, oxo, CF₃, OR₁₀, NR₁₁R₁₂, C₆H₅, C₆H₅ substituted with halogen, C₁-C₃alkyl, OR₁₀, NR₁₁R₁₂, wherein R₁₀, R₁₁, R₁₂ are independently at each occurrence H, C₁-C₃alkyl. Examples of heteroaryl groups are pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, thiadiazolyl, furazanyl, benzofurazanyl, benzopyranyl, benzothiophenyl, benzothiazolyl, benzooxazolyl, quinazolinyl, quinoxalinyl, naphthyridinyl, and furopyridinyl. Heteroaryl groups are optionally substituted independently with one or more substituents as described herein. The term "monocyclic heteroaryl" refers to a five- or six-membered, unsubstituted or substituted, monocyclic heteroaryl radical which contains 1, 2, 3 or 4 ring heteroatoms independently selected from N, O and S. Monocyclic heteroaryl radicals include, but are not limited to: 2-pyridyl, 3-pyridyl, 4-pyridyl, 3-isoxazolyl, 4-isoxazolyl, 5-isoxazolyl, 2-imidazolyl, 4-imidazolyl, 3-pyrazolyl, 4-pyrazolyl, 2-pyrrolyl, 3-pyrrolyl, 2-thiazolyl, 4-thiazolyl, 5-thiazolyl, 3-pyridazinyl, 4-pyridazinyl, 5-pyridazinyl, 2-pyrimidinyl, 5-pyrimidinyl, 6-pyrimidinyl, 2-pyrazinyl, 2-oxazolyl, 4-oxazolyl, 5-oxazolyl, 2-furanyl, 3-furanyl, 2-thienyl, 3-thienyl, 3-triazolyl, 1-triazolyl, 5-tetrazolyl, 1-tetrazolyl, and 2-tetrazolyl. Monocyclic heteroaryl are optionally substituted independently with one or more substituents as described herein. Monocyclic heteroaryl are optionally substituted independently with one or more substituents, typically and preferably with one or two substituents, wherein said substituents are independently at each occurrence selected from C₁-C₄alkyl, halogen, CF₃, OR₁₀, NR₁₁R₁₂, C₆H₅, C₆H₅ substituted with halogen, C₁-C₃alkyl, OR₁₀, NR₁₁R₁₂, wherein R₁₀, R₁₁, R₁₂ are independently at each occurrence H, C₁-C₃alkyl.

The terms "spiranyl", and "spirocycle" are used interchangeably herein and refer to a saturated or a partially unsaturated (i.e., having one or more double within the rings), typically and preferably to a saturated, bicyclic radical of 3 to 8 ring atoms per ring, wherein the rings are linked together by one common atom, typically and preferably by a carbon atom. The individual rings may be both carbocyclic rings or may contain independently of each other at least one heteroatom selected from nitrogen, oxygen and sulphur, the remaining ring atoms being C, where one or both ring atoms are optionally substituted independently with one or more substituents as described herein. Examples of heterocyclic spiranyl include diazaspiranyl of the formulas -([CH₂]ₘ)ₙC([CH₂]ₘ)ₙ-N-C₁-C₃alkyl wherein m and n are independently at each occurrence selected from 1 or 2, typically and preferably -([CH₂])₂C([CH₂])₂-N-C₁-C₃alkyl,-([CH₂])₂C([CH₂]₂)-N-C₁-C₃alkyl, -([CH₂]₂)₂C([CH₂]₂)₂-N-C₁-C₃alkyl,-([CH₂]₂[CH₂])C([CH₂]₂[CH₂])-N-C₁-C₃alkyl.

Where a group is said to be optionally substituted, preferably there are optionally 1-5 substituents, more preferably optionally 1-3 substituents, and again more preferably optionally one or two substituents.

The term "amino acid", as used herein, refers to organic compounds containing the functional groups amine (-NH2) and carboxylic acid (-COOH) and its zwitterions, typically and preferably, along with a side chain specific to each amino acid. The term "amino acid" typically and preferably includes amino acids that occur naturally, such as proteinogenic amino acids (produced by RNA-translation), non-proteinogenic amino acids (produced by other metabolic mechanisms, e.g. posttranslational modification), standard or canonical amino acids (that are directly encoded by the codons of the genetic code) and non-standard or non-canonical amino acids (not directly encoded by the genetic code). Naturally occurring amino acids include non-eukaryotic and eukaryotic amino acids. The term "amino acid", as used herein, also includes unnatural amino acids that are chemically synthesized. Moreover, the term covers alpha- (α-), beta- (β-), gamma- (γ-) and delta- (δ-) etc. amino acids as well as mixtures thereof in any ratio, and any isomeric form of an amino acid, i.e. D- and L-stereoisomers (alternatively addressed by the (*R*) and (*S*) nomenclature) as well as mixtures thereof in any ratio, preferably in a racemic ratio of 1:1. Amino acids in this invention are preferably in L-configuration. The term "D-stereoisomer", "L-stereoisomer", "D-amino acid" or "L-amino acid" refers to the chiral alpha carbon of the amino acids.

Certain compounds of formula (I) of the present invention may contain one or two or more centers of chirality and such compounds may be provided as pure enantiomers or pure diastereoisomers as well as mixtures thereof in any ratio. The compounds of the invention also include all tautomeric forms of the compounds of formula (I). The compounds of formula (I) may also be solvated, especially hydrated, which are also included in the compounds of formula (I).

The term "chiral" refers to compounds, which have the property of non-superimposability of the mirror image partner, while the term "achiral" refers to compounds, which are superimposable on their mirror image partner.

The term "stereoisomers" refers to compounds, which have identical chemical constitution, but differ with regard to the arrangement of the atoms or groups in space.

"Diastereomer" refers to a stereoisomer with two or more centers of chirality in which the compounds are not mirror images of one another. Diastereomers have different physical properties, e.g. melting points, boiling points, spectral properties, and chemical and biological reactivities. Mixtures of diastereomers may be separated under high resolution analytical procedures such as electrophoresis and chromatography.

"Enantiomers" refer to two stereoisomers of a compound which are non-superimposable mirror images of one another.

Stereochemical definitions and conventions used herein generally follow S.P. Parker, Ed., McRaw-Hiff Dictionary of Chemical Terms (1984), McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centers, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention. Many organic compounds exist in optically active forms, *i.e.,* they have the ability to rotate the plane of plane-polarized light. In describing an optically active compound, the prefixes D and L, or *R* and *S*, are used to denote the absolute configuration of the molecule about its chiral center(s). The prefixes d and 1 or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric or a scalemic mixture. A 50:50 mixture of enantiomers is referred to as a racemic mixture or a racemate. The term "tautomer" or "tautomeric form" refers to structural isomers of different energies, which are interconvertible via a low energy barrier. For example, proton tautomers include interconversions via migration of a proton, such as keto-enol and imine-enamine isomerizations.

The phrase "pharmaceutically acceptable salt" as used herein, refers to pharmaceutically acceptable organic or inorganic salts of a compound of the invention, in particular acid addition salts. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate (mesylate), ethanesulfonate, benzenesulfonate, *p*-toluenesulfonate, and pamoate salts. A pharmaceutically acceptable salt may involve the inclusion of another molecule such as an acetate ion, a succinate ion or other counter ion. The counter ion may be any organic or inorganic moiety that stabilizes the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound of the invention is a base, the desired pharmaceutically acceptable salt may be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as *p*-toluenesulfonic acid or ethanesulfonic acid, or the like.

A "solvate" refers to an association or complex of one or more solvent molecules and a compound of the invention. Examples of solvents that form solvates include, but are not limited to, water, isopropanol, ethanol, methanol, dimethyl sulfoxide (DMSO), ethyl acetate, acetic acid, and ethanolamine. The term "hydrate" refers to the complex where the solvent molecule is water.

The term "protecting group" refers to a substituent that is commonly employed to block or protect a particular functionality during the reaction of other functional groups on the compound. For example, an "amino-protecting group" is a substituent attached to an amino group that blocks or protects the amino functionality in the compound. Suitable amino-protecting groups include acetyl, trifluoroacetyl, *tert*-butoxycarbonyl (BOC), benzyloxycarbonyl and 9-fluorenylmethylenoxycarbonyl (Fmoc). For a general description of protecting groups and their use, see T. W. Greene, Protective Groups in Organic Synthesis, John Wiley & Sons, New York, 1991.

The terms "compound of this invention" and "compounds of the present invention" and "compounds of formula (I)" include stereoisomers, geometric isomers, tautomers, solvates, pharmaceutically acceptable salts, and solvates of the salts thereof.

The term "mammal" includes, but is not limited to, humans, mice, rats, guinea pigs, monkeys, dogs, cats, horses, cows, pigs, and sheep. The term "mammal", as used herein, preferably refers to humans.

The terms "treat" and "treatment" refer to both therapeutic treatment and prophylactic or preventative measures, wherein the object is to prevent or slow down (lessen) an undesired pathological change or disorder, such as the development of leishmaniasis. For purpose of this invention, beneficial or desired clinical results include, but are not limited to, alleviation of symptoms, diminishment of extent of disease, stabilized (*i*.*e*., not worsening) state of disease, delay or slowing of disease progression, amelioration or palliation of the disease state, and remission (whether partial or total), whether detectable or undetectable. "Treatment" can also mean prolonging survival as compared to expected survival if not receiving treatment. Those in need of treatment include those already with the condition or disorder as well as those prone to have the condition or disorder or those in which the condition or disorder is to be prevented.

The phrase "effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein. In the case of protozoan diseases, in particular in case of leishmaniasis, the effective amount of the drug may reduce the number of parasites, in particular of *Leishmania donovani* and/or is effective in inhibiting proliferation of a parasite in the *Leishmania* genus and includes an effective amount against a parasite from the *Leishmania* genus.

The compounds and methods described herein are advantageously used as a novel class of anti-Leishmanial agents to inhibit proliferation of *Leishmania* parasites. The compounds and methods can provide prophylaxis or treatment for a vertebrate against a parasite in the *Leishmania* genus. Preferably, the compounds and methods can provide treatment for a vertebrate, preferably a human, against a parasite in the *Leishmania* genus. The methods can provide treatment against the various stages of *Leishmania* parasite infections. Thus, administering an inventive compound or pharmaceutical composition can provide prophylaxis or treatment, preferably treatment, to a patient against the proliferation of *Leishmania* parasites. In particular, the inventive compound or pharmaceutical composition and method can provide treatment for a human against leishmania disease. Administering an inventive compound or pharmaceutical composition to a patient in a stage of infection with *Leishmania* genus can treat against cutaneous, mucocutaneous, and visceral forms of the disease. Administering an inventive compound or pharmaceutical composition to a patient in a stage of infection with *Leishmania donovani* can treat against visceral leishmaniasis (VL).

Administering an inventive compound or pharmaceutical composition to a patient in a stage of infection with *Leishmania donovani* can treat against promastigote and/or amastigote forms of *Leishmania donovani* parasites. Administering an inventive compound or pharmaceutical composition can be used to treat *Leishmania* infections where the parasites are susceptible in promastigote and/or amastigote forms of *Leishmania donovani* within the life cycle. Administering for prophylaxis may help break the life cycle of leishmaniasis disease and reduce the patient's chances of becoming infected or infecting another through a vector.

Administering an inventive compound or pharmaceutical composition to a patient can, in principle, lead to inhibiting proliferation of *Leishmania,* by directly affecting the parasite's life cycle. Once a vector ingests blood from the patient whose blood plasma contains an inventive compound or pharmaceutical composition, further development of *Leishmania* parasite in the vector may be inhibited.

Administering includes sublingual administration, oral administration, and, in principle, intravenous administration. A pharmaceutical composition can contain the active pharmaceutical ingredient and may additionally comprise a pharmaceutically acceptable vehicle or adjuvant. A pharmaceutical composition can be in the form of a solid pharmaceutical dosage form (tablet, caplet, capsule, or deliverable from an osmotic pump as examples) or syrup. Remington, The Science and Practice of Pharmacy, provides general information regarding pharmaceutical dosage forms.

A therapeutically effective amount means an amount of an inventive compound or pharmaceutical composition that can provide a therapeutic benefit to a patient against leishmaniasis. Patient includes human. A patient in need of treatment includes a human patient in need of treatment against *Leishmania* parasites.

In a first aspect, the present invention provides for a compound of formula (I) 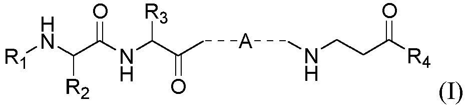 wherein said compound of formula (I) is a compound of any one of the formula (II) to (IV) 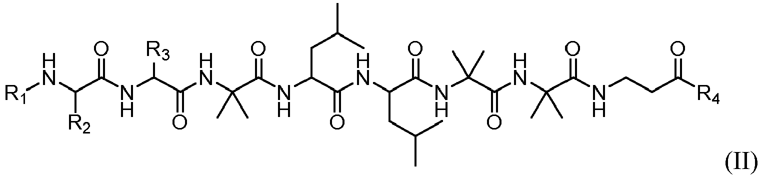 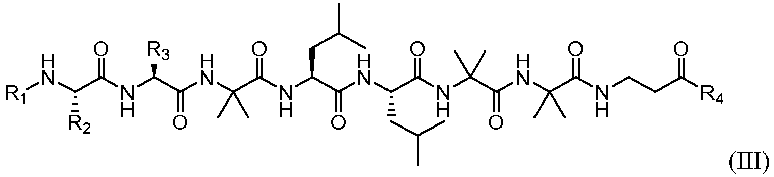 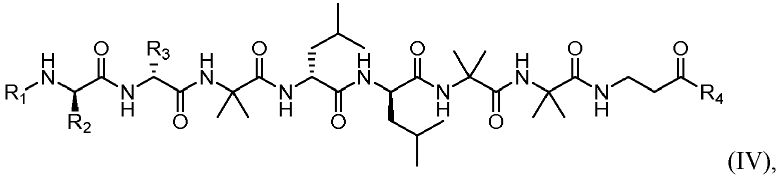 and wherein
R₁ is 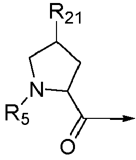 preferably 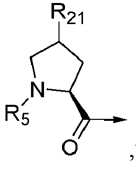 wherein R₅ is C(O)-R₈, wherein R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl, pyrrolidinyl, benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen preferably fluorine, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; and wherein the arrow indicates the attachment to the NH-moiety depicted in formula (I);
R₂ is selected from 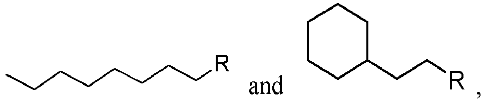 wherein
R indicates the attachment to the CH-moiety depicted in formula (I);
R₃ is selected from 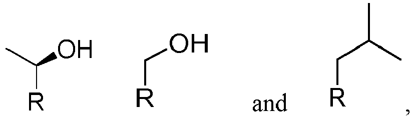 wherein R indicates the attachment to the CH-moiety depicted in formula (I);
R₄ is 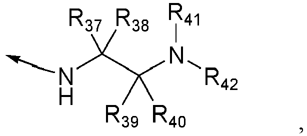 wherein
   R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
   R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅; wherein
   the arrow indicates the attachment to the C(O)-moiety depicted in formula (I);
   and pharmaceutically acceptable salts of said compound of formula (I).

Said compound of formula (I) is a compound of any one of the formulas (II) to (IV) 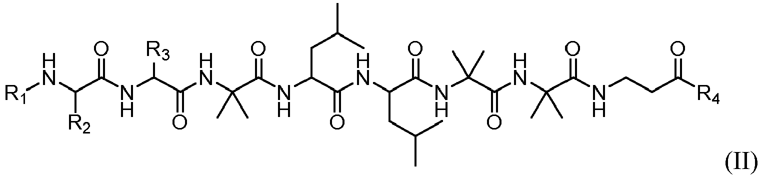 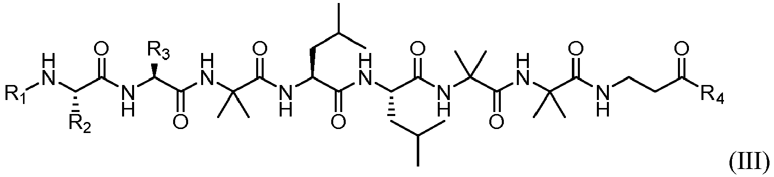 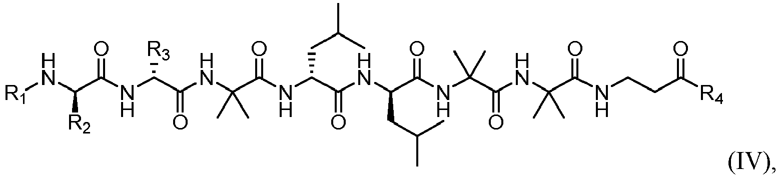 wherein preferably said compound of formula (I) is a compound of formula (III).

In another preferred embodiment, said compound of formula (I) is a compound of formula (II), wherein preferably said compound of formula (II) is a compound of formula (III) or a compound of formula (IV).

In another preferred embodiment, said compound of formula (I) is a compound of formula (III). In another preferred embodiment, said compound of formula (I) is a compound of formula (IV).

Disclosed are substituents R₁ being 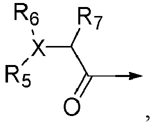 preferably 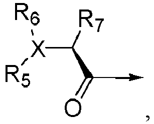 wherein
X is N or CH;
R₅ is selected from H, C₁-C₁₆alkyl, C₁-C₁₆alkenyl, C(O)-C₁-C₁₆alkyl, C(O)-C₁-C₁₆alkenyl, (wherein said C₁-C₁₆alkyl, C₁-C₁₆alkenyl, C(O)-C₁-C₁₆alkyl, C(O)-C₁-C₁₆alkenyl are) independently optionally substituted with halogen, NR₁₁R₁₂, ―[O-C₂H₄]ₙ-OCH₃ wherein n=2-20; C(O)-R₈, C(O)-C₁-C₃alkylene-R₈, N(H)C(O)-R₈, or S(O)₂-R₉, wherein
R₈ is independently at each occurrence selected from aryl, heteroaryl, cycloalkyl, heterocyclyl, each independently optionally substituted with C₁-C₄alkyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl;
R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl;
R₆, R₇ are independently at each occurrence selected from H, C₁-C₄alkyl, C₁-C₄alkenyl, C(O)-C₁-C₃alkyl, C(O)-C₁-C₄alkenyl, (wherein said C₁-C₄alkyl, C₁-C₄alkenyl, C(O)-C₁-C₃alkyl, C(O)-C₁-C₄alkenyl are) independently optionally substituted with halogen, NR₁₁R₁₂; or R₆ and R₇ together with the X-C to which they are attached form independently at each occurrence an aryl, heteroaryl, cycloalkyl, heterocyclyl, each independently optionally (*further*) substituted with C₁-C₄alkyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; and wherein
the arrow indicates the attachment to the NH-moiety depicted in formula (I).

Disclosed are substituents R₆ and R₇ that together with the X-C to which they are attached form a pyrrolidinyl.

Disclosed are substituents R₆ and R₇ that together with the X-C to which they are attached form a pyrrolidinyl, wherein said pyrrolidinyl is (*further*) substituted with methyl.

Disclosed are substituents R₂ selected from C₁-C₁₄alkyl, C₂-C₁₄alkyl optionally substituted with OH, C₂-C₁₄alkoxy, C₂-C₁₄alkenyl optionally substituted with OH; C₁-C₈alkylene-R₂₃, wherein in alkylene one or two -CH₂- moieties are optionally replaced by - CH(NR₂₄R₂₅)-, ―CH(OH)-, -C(=O)-, -NR₂₄R₂₅- or -CH(CH₃)- moieties, wherein there are no adjacent -C(=O)- moieties or adjacent -NR₂₄R₂₅- moieties, and wherein R₂₃ is independently at each occurrence selected from hydrogen; aryl, heteroaryl, cycloalkyl, heterocyclyl, each independently optionally substituted with C₁-C₄alkyl, OR₂₆, NR₂₇R₂₈; wherein R₂₆, R₂₇, R₂₈ are independently at each occurrence H, halogen, CF₃, C₁-C₃alkyl; and wherein R₂₄ and R₂₅ are independently at each occurrence H, C₁-C₃alkyl; with the proviso that R₂ is not - CH₂CH(CH₃)CH₂CH(OH)CH₂C(O)C₂H₅, ―CH₂CH(CH₃)CH₂CH=CHC(O)C₂H₅, ― CH₂CH(CH₃)CH₂CH₂CH₂C(O)C₂H₅, ―CH₂CH(CH₃)(CH₂)₃CH(OH)C₂H₅ or ― CH₂CH(CH₃)CH₂CH(OCH₃)CH₂C(O)C₂H₅.

Disclosed are substituents R₄ being 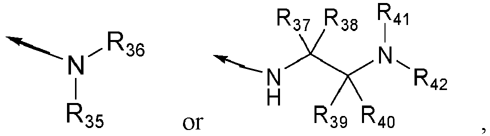 wherein
R₃₅ is independently selected from hydrogen and C₁-C₃-alkyl;
R₃₆ is independently at each occurrence selected from -cycloalkyl-NR₄₁R₄₂, wherein said cycloalkyl moiety is optionally substituted by C₁-C₄alkyl, hydroxyl, halogen, OR₄₃; -C₃-C₆alkylene-NR₄₁R₄₂, wherein said C₃-C₆alkylene moiety is optionally substituted by hydroxyl, OR₄₃, halogen; cycloalkyl optionally substituted with C₁-C₄alkyl, halogen, OR₄₃; or wherein R₃₅ and R₃₆ together with the nitrogen atom to which they are attached form independently at each occurrence a heteroaryl, a heterocyclyl or a heterocyclic spiranyl, each independently optionally substituted with C₁-C₄alkyl, halogen, OR₄₃, NR₄₄R₄₅, wherein R₄₃, R₄₄, R₄₅ are independently at each occurrence H, C₁-C₄alkyl; and wherein
R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅; wherein the arrow indicates the attachment to the C(O)-moiety depicted in formula (I).

In a further preferred embodiment, said R₄ is 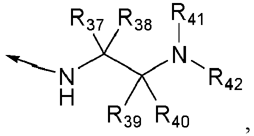 wherein
R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl; wherein the arrow indicates the attachment to the C(O)-moiety depicted in formula (I).

In a further preferred embodiment, said R₄ is 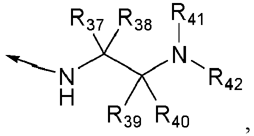 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic or a monocyclic heterocyclic ring, preferably a monocyclic carbocyclic ring, and wherein R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein preferably said monocyclic heteroaryl or said monocyclic heterocyclyl comprise one, two or three heteroatoms (including said nitrogen atom to which R₄₁ and R₄₂ are attached) selected from nitrogen, oxygen and sulphur; wherein the arrow indicates the attachment to the C(O)-moiety depicted in formula (I).

In a further preferred embodiment, said R₄ is 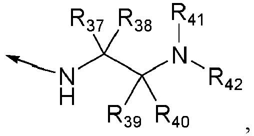 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic or a monocyclic heterocyclic ring, preferably a monocyclic carbocyclic ring, and wherein R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein said monocyclic heteroaryl or said monocyclic heterocyclyl comprise one, two or three heteroatoms (including said nitrogen atom to which R₄₁ and R₄₂ are attached) selected from nitrogen, oxygen and Sulphur; wherein the arrow indicates the attachment to the C(O)-moiety depicted in formula (I).

In a further preferred embodiment, said R₄ is 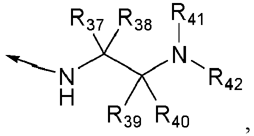 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic or a monocyclic heterocyclic ring, preferably a monocyclic carbocyclic ring, and wherein R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein said monocyclic heteroaryl or said monocyclic heterocyclyl comprise one or two heteroatoms (including said nitrogen atom to which R₄₁ and R₄₂ are attached) selected from nitrogen, oxygen and sulphur, wherein the arrow indicates the attachment to the C(O)-moiety depicted in formula (I).

In a further preferred embodiment, said R₄ is 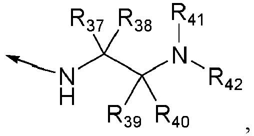 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic or a monocyclic heterocyclic ring, preferably a monocyclic carbocyclic ring, and wherein R₄₁ and R₄₂ are independently of each other C₁-C₄alkyl, preferably methyl; or together with the nitrogen atom to which they are attached form independently at each occurrence a pyridinyl imidazolyl, imidazopyridinyl, pyrimidinyl, pyrazolyl, triazolyl, pyrazinyl, tetrazolyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, indazolyl, indolizinyl, pyridazinyl, triazinyl, isoindolyl, purinyl, pyrrolidinyl, piperidinyl, morpholinyl, piperazinyl, homopiperazinyl, 2-pyrrolinyl, 3-pyrrolinyl, indolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

In a further preferred embodiment, said R₄ is 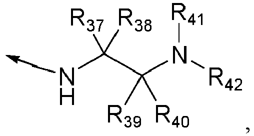 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic, wherein preferably said monocyclic carbocyclic ring is selected from cyclopropyl, cyclobutyl, cyclopentyl, cyclopentenyl, cyclohexyl, cyclohexenyl, cycloheptyl and cyclooctyl, and wherein R₄₁ and R₄₂ are independently of each other C₁-C₄alkyl, preferably methyl; or together with the nitrogen atom to which they are attached form independently at each occurrence a pyridinyl, imidazolyl, pyrimidinyl, pyrazolyl, triazolyl, pyrazinyl, tetrazolyl, isoxazolyl, thiazolyl, oxadiazolyl, oxazolyl, isothiazolyl, pyrrolyl, triazinyl, pyrrolidinyl, piperidinyl, morpholinyl, piperazinyl, homopiperazinyl, 2-pyrrolinyl, 3-pyrrolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

In a further preferred embodiment, said R₄ is 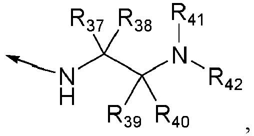 wherein said R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a monocyclic carbocyclic, wherein said monocyclic carbocyclic ring is selected from cyclopropyl, cyclobutyl, cyclopentyl, cyclopentenyl, cyclohexyl, cyclohexenyl, cycloheptyl and cyclooctyl, and wherein preferably said monocyclic carbocyclic ring is selected from cyclobutyl, cyclopentyl, cyclohexyl and cycloheptyl, and wherein R₄₁ and R₄₂ are independently of each other C₁-C₄alkyl, preferably methyl; or together with the nitrogen atom to which they are attached form independently at each occurrence a pyridinyl, imidazolyl, pyrimidinyl, pyrazolyl, pyrazinyl, isoxazolyl, oxazolyl, pyrrolyl, pyrrolidinyl, piperidinyl, morpholinyl, piperazinyl, homopiperazinyl, 2-pyrrolinyl, 3-pyrrolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

In a further preferred embodiment, said R₄ is selected from 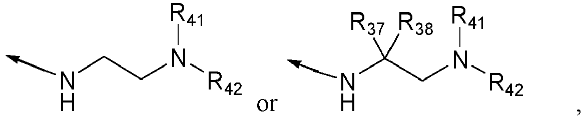 wherein R₃₇ and R₃₈, are independently at each occurrence H or C₁-C₃alkyl, preferably H or methyl, or independently at each occurrence R₃₇ and R₃₈ together with the carbon atom to which they are attached form a carbocyclic or heterocyclic ring, and wherein R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl; wherein the arrow indicates the attachment to the C(O)-moiety depicted in formula (I).

In a further preferred embodiment, said R₄ is selected from 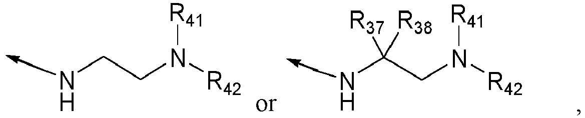 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic or a monocyclic heterocyclic ring, preferably a monocyclic carbocyclic ring, and wherein said R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein preferably said monocyclic heteroaryl or said monocyclic heterocyclyl comprise one, two or three heteroatoms (including said nitrogen atom to which R₄₁ and R₄₂ are attached) selected from nitrogen, oxygen and sulphur; wherein the arrow indicates the attachment to the C(O)-moiety depicted in formula (I).

In a further preferred embodiment, said R₄ is selected from 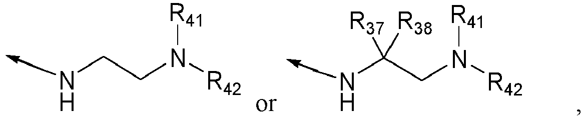 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic or a monocyclic heterocyclic ring, preferably a monocyclic carbocyclic ring, and wherein said R₄₁ and R₄₂ are independently of each other C₁-C₄alkyl preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein said monocyclic heteroaryl or said monocyclic heterocyclyl comprise one, two or three heteroatoms (including said nitrogen atom to which R₄₁ and R₄₂ are attached) selected from nitrogen, oxygen and Sulphur; wherein the arrow indicates the attachment to the C(O)-moiety depicted in formula (I).

In a further preferred embodiment, said R₄ is selected from 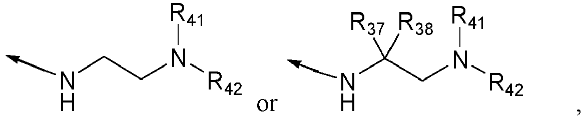 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic or a monocyclic heterocyclic ring, preferably a monocyclic carbocyclic ring, and wherein said R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, and wherein said monocyclic heteroaryl or said monocyclic heterocyclyl comprise one or two heteroatoms (including said nitrogen atom to which R₄₁ and R₄₂ are attached) selected from nitrogen, oxygen and sulphur, wherein the arrow indicates the attachment to the C(O)-moiety depicted in formula (I).

In a further preferred embodiment, said R₄ is selected from 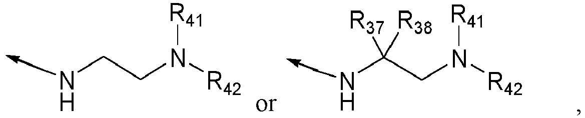 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic or a monocyclic heterocyclic ring, preferably a monocyclic carbocyclic ring, and wherein said R₄₁ and R₄₂ are independently of each other C₁-C₄alkyl preferably methyl; or together with the nitrogen atom to which they are attached form independently at each occurrence a pyridinyl imidazolyl, imidazopyridinyl, pyrimidinyl, pyrazolyl, triazolyl, pyrazinyl, tetrazolyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, indazolyl, indolizinyl, pyridazinyl, triazinyl, isoindolyl, purinyl, pyrrolidinyl, piperidinyl, morpholinyl, piperazinyl, homopiperazinyl, 2-pyrrolinyl, 3-pyrrolinyl, indolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

In a further preferred embodiment, said R₄ is selected from 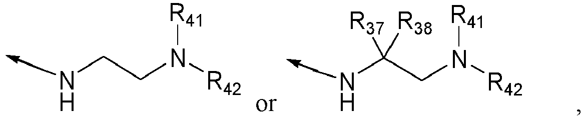 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic, wherein preferably said monocyclic carbocyclic ring is selected from cyclopropyl, cyclobutyl, cyclopentyl, cyclopentenyl, cyclohexyl, cyclohexenyl, cycloheptyl and cyclooctyl, and wherein R₄₁ and R₄₂ are independently of each other C₁-C₄alkyl, preferably methyl; or together with the nitrogen atom to which they are attached form independently at each occurrence a pyridinyl imidazolyl, pyrimidinyl, pyrazolyl, triazolyl, pyrazinyl, tetrazolyl, isoxazolyl, thiazolyl, oxadiazolyl, oxazolyl, isothiazolyl, pyrrolyl, triazinyl, pyrrolidinyl, piperidinyl, morpholinyl, piperazinyl, homopiperazinyl, 2-pyrrolinyl, 3-pyrrolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

In a further preferred embodiment, said R₄ is selected from 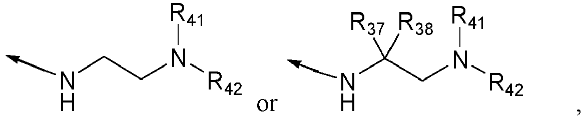 wherein R₃₇ and R₃₈, are independently at each occurrence H or C₁-C₃alkyl, preferably H or methyl, or independently at each occurrence R₃₇ and R₃₈ together with the carbon atom to which they are attached form a monocyclic carbocyclic, wherein said monocyclic carbocyclic ring is selected from cyclopropyl, cyclobutyl, cyclopentyl, cyclopentenyl, cyclohexyl, cyclohexenyl, cycloheptyl and cyclooctyl, and wherein preferably said monocyclic carbocyclic ring is selected from cyclobutyl, cyclopentyl, cyclohexyl and cycloheptyl, and wherein said R₄₁ and R₄₂ are independently of each other C₁-C₄alkyl, preferably methyl; or together with the nitrogen atom to which they are attached form independently at each occurrence a pyridinyl imidazolyl, pyrimidinyl, pyrazolyl, pyrazinyl, isoxazolyl, oxazolyl, pyrrolyl, pyrrolidinyl, piperidinyl, morpholinyl, piperazinyl, homopiperazinyl, 2-pyrrolinyl, 3-pyrrolinyl, dihyrooxazolyl, pyrazolinyl, pyrazolidinyl, imidazolinyl, imidazolidinyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl, wherein the arrow indicates the attachment to the C(O)-moiety depicted in formula (I).

Further disclosed are substituents R₁ selected from 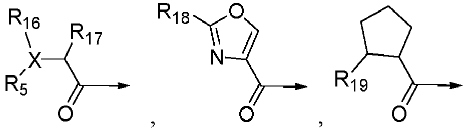 preferably 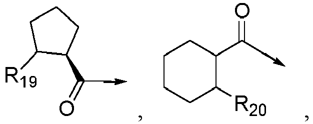 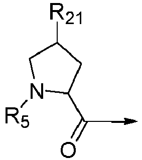 preferably 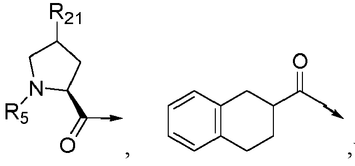 wherein X is N or CH; and wherein said R₅ is selected from H, C(O)-C₁-C₁₆alkyl, C(O)-C₁-C₁₆alkenyl, C(O)-C₁-C₄alkylene―[O-C₂H₄]ₙ-OCH₃ wherein n=2-20; C(O)-R₈, C(O)-C₁-C₃alkylene-R₈, N(H)C(O)-R₈, or S(O)₂-R₉; and wherein R₈ is independently at each occurrence selected from phenyl, naphthalene, anthracene, biphenyl, indenyl, indanyl, 1,2-dihydronapthalenyl, 1,2,3,4-tetrahydronaphthenyl, pyridinyl, imidazolyl, imidazopyridinyl, pyrimidinyl, pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, benzopyranyl, coumarinyl, cinnolinyl, indazolyl, indolizinyl, phthalazinyl, pyridazinyl, triazinyl, isoindolyl, pteridinyl, purinyl, thiadiazolyl, furazanyl, benzofurazanyl, benzothiophenyl, benzothiazolyl, benzooxazolyl, quinazolinyl, quinoxalinyl, naphthyridinyl, furopyridinyl, cyclopropyl, cyclobutyl, cyclopentyl, 1-cyclopent-1-enyl, 1-cyclopent-2-enyl, 1-cyclopent-3-enyl, cyclohexyl, 1-cyclohex-1-enyl, 1-cyclohex-2-enyl, 1-cyclohex-3-enyl, cyclohexadienyl, cycloheptyl, cyclooctyl, adamantanyl, pyrrolidinyl, tetrahydrofuranyl, dihydrofuranyl, tetrahydrothienyl, tetrahydropyranyl, dihydropyranyl, tetrahydrothiopyranyl, piperidinyl, morpholinyl, thiomorpholinyl, thioxanyl, piperazinyl, homopiperazinyl, 2-pyrrolinyl, 3-pyrrolinyl, indolinyl, dioxanyl, dihyrooxazolyl, pyrazolinyl, pyrazolidinyl, imidazolinyl, imidazolidinyl; each independently optionally substituted with C₁-C₄alkyl, halogen preferably fluorine, C₆H₅, CF₃, OR₁₀, NR₁₁R₁₂; wherein R₁₀, R₁₁, R₁₂ are independently at each occurrence H, C₁-C₃alkyl; and wherein R₉ is independently at each occurrence selected from C₁-C₄alkyl, phenyl, naphthalene, anthracene, biphenyl, indenyl, indanyl, 1,2-dihydronapthalenyl, 1,2,3,4-tetrahydronaphthenyl, pyridinyl, imidazolyl, imidazopyridinyl, pyrimidinyl, pyrazolyl, triazolyl, pyrazinyl, tetrazolyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, benzopyranyl, cinnolinyl, indazolyl, indolizinyl, phthalazinyl, pyridazinyl, triazinyl, isoindolyl, pteridinyl, purinyl, thiadiazolyl, furazanyl, benzofurazanyl, benzothiophenyl, benzothiazolyl, benzooxazolyl, quinazolinyl, quinoxalinyl, naphthyridinyl, furopyridinyl, each independently optionally substituted with C₁-C₄alkyl, halogen preferably fluorine, CF₃, OR₁₃, NR₁₄R₁₅; wherein R₁₃, R₁₄, R₁₅ are independently at each occurrence H, C₁-C₃alkyl; and wherein R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl.; and wherein R₁₆, R₁₇ are independently at each occurrence selected from H, C₁-C₄alkyl, wherein said C₁-C₄alkyl is independently optionally substituted with halogen, NR₁₁R₁₂; and wherein R₁₈ is selected from H, C₁-C₄alkyl preferably methyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; R₁₉ is selected from H, C₁-C₄alkyl; and wherein R₂₀ is selected from H, C₁-C₄alkyl, C(O)-R₂₂, C(O)-C₁-C₃alkyl-R₂₂, or N(H)C(O)-R₂₂, wherein R₂₂ is independently at each occurrence selected from aryl optionally substituted with C₁-C₄alkyl, halogen, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; wherein preferably said R₂₁ is H, and wherein the arrow indicates the attachment to the NH-moiety depicted in formula (I).

Further disclosed are substituents R₁ selected from 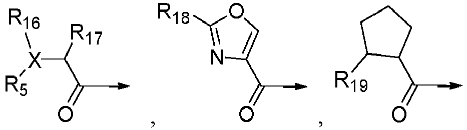 preferably 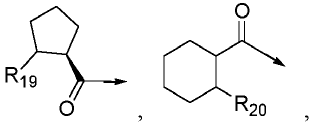 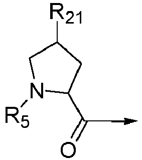 preferably 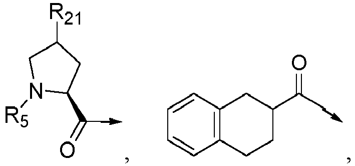 wherein X is N or CH; and wherein said R₅ is selected from H, C(O)-C₁-C₁₆alkyl, C(O)-C₁-C₁₆alkenyl, C(O)-C₁-C₄alkylene―[O-C₂H₄]ₙ-OCH₃ wherein n=2-20, wherein preferably said C₁-C₁₆alkyl and said C₁-C₁₆alkenyl of said C(O)-C₁-C₁₆alkyl and said C(O)-C₁-C₁₆alkenyl are straight C₁-C₁₆alkyl and C₁-C₁₆alkenyl chains; C(O)-R₈, C(O)-C₁-C₃alkylene-R₈, N(H)C(O)-R₈, or S(O)₂-R₉; and wherein R₈ is independently at each occurrence selected from phenyl, naphthalene, biphenyl, indenyl, indanyl, 1,2-dihydronapthalenyl, 1,2,3,4-tetrahydronaphthenyl, pyridinyl, imidazolyl, imidazopyridinyl, pyrimidinyl, pyrazolyl, pyrazinyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, benzopyranyl, coumarinyl, indazolyl, indolizinyl, piperidinyl, morpholinyl, pyrrolidinyl, pyridazinyl, triazinyl, isoindolyl, triazolyl, furazanyl, benzofurazanyl, benzothiophenyl, benzothiazolyl, benzooxazolyl, quinazolinyl, quinoxalinyl, naphthyridinyl, furopyridinyl, cyclopentyl, cyclopentenyl, cyclohexyl, cyclohexenyl, adamantanyl; each independently optionally substituted with C₁-C₄alkyl, halogen, C₆H₅, CF₃, OR₁₀, NR₁₁R₁₂; wherein R₁₀, R₁₁, R₁₂ are independently at each occurrence H, C₁-C₃alkyl; and wherein R₉ is independently at each occurrence selected from C₁-C₄alkyl, phenyl, naphthalene, anthracene, biphenyl, indenyl, indanyl, 1,2-dihydronapthalenyl, 1,2,3,4-tetrahydronaphthenyl, pyridinyl, imidazolyl pyrimidinyl, pyrazolyl, pyrazinyl, furyl, thienyl, isoxazolyl, oxadiazolyl, oxazolyl, pyrrolyl, quinolinyl, indolyl, benzimidazolyl, benzofuranyl, benzopyranyl, indazolyl, indolizinyl, pyridazinyl, isoindolyl, furazanyl, benzofurazanyl, benzothiophenyl, benzothiazolyl, benzooxazolyl, each independently optionally substituted with C₁-C₄alkyl, halogen preferably fluorine, CF₃, OR₁₃, NR₁₄R₁₅; wherein R₁₃, R₁₄, R₁₅ are independently at each occurrence H, C₁-C₃alkyl; and wherein R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl.; and wherein R₁₆, R₁₇ are independently at each occurrence selected from H, C₁-C₄alkyl, wherein said C₁-C₄alkyl is independently optionally substituted with halogen, NR₁₁R₁₂; and wherein R₁₈ is selected from H, C₁-C₄alkyl preferably methyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; R₁₉ is selected from H, C₁-C₄alkyl; and wherein R₂₀ is selected from H, C₁-C₄alkyl, C(O)-R₂₂, C(O)-C₁-C₃alkyl-R₂₂, or N(H)C(O)-R₂₂, wherein R₂₂ is independently at each occurrence selected from aryl optionally substituted with C₁-C₄alkyl, halogen, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; wherein preferably said R₂₁ is H, and wherein the arrow indicates the attachment to the NH-moiety depicted in formula (I).

Further disclosed are substituents R₁ selected from 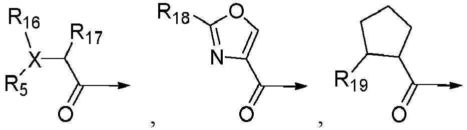 preferably 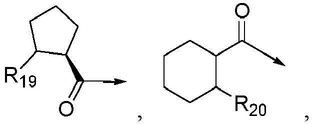 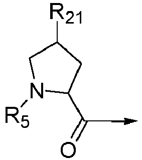 preferably 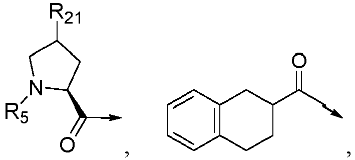 wherein X is N or CH; and wherein said R₅ is C(O)-R₈, wherein R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl, pyrrolidinyl, benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen preferably fluorine, CF₃, OR₁₀, NR₁₁R₁₂; wherein R₁₀, R₁₁, R₁₂ are independently at each occurrence H, C₁-C₃alkyl; and wherein R₉ is independently at each occurrence selected from C₁-C₄alkyl, phenyl, naphthalene, 1,2-dihydronapthalenyl, 1,2,3,4-tetrahydronaphthenyl, each independently optionally substituted with C₁-C₄alkyl, halogen preferably fluorine, CF₃, OR₁₃, NR₁₄R₁₅; wherein R₁₃, R₁₄, R₁₅ are independently at each occurrence H, C₁-C₃alkyl; and wherein R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl; and wherein R₁₆, R₁₇ are independently at each occurrence selected from H, C₁-C₄alkyl, wherein said C₁-C₄alkyl is independently optionally substituted with halogen, NR₁₁R₁₂; and wherein R₁₈ is selected from H, C₁-C₄alkyl preferably methyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; R₁₉ is selected from H, C₁-C₄alkyl; and wherein R₂₀ is selected from H, C₁-C₄alkyl, C(O)-R₂₂, C(O)-C₁-C₃alkyl-R₂₂, or N(H)C(O)-R₂₂, wherein R₂₂ is independently at each occurrence selected from aryl optionally substituted with C₁-C₄alkyl, halogen, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; wherein preferably said R₂₁ is H, and wherein the arrow indicates the attachment to the NH-moiety depicted in formula (I).

Further disclosed are substituents R₁ selected from 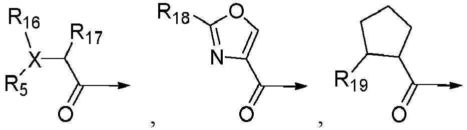 preferably 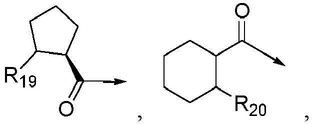 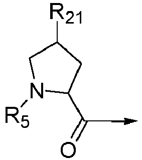 preferably 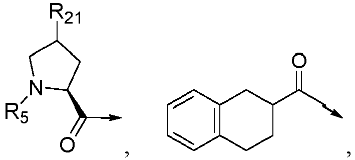 wherein X is N or CH; and wherein said R₅ is C(O)-R₈, wherein R₈ is phenyl independently optionally substituted with C₁-C₄alkyl, halogen, preferably flurorine, CF₃, OR₁₀, NR₁₁R₁₂; wherein R₁₀, R₁₁, R₁₂ are independently at each occurrence H, C₁-C₃alkyl; and wherein R₁₆, R₁₇ are independently at each occurrence selected from H, C₁-C₄alkyl, wherein said C₁-C₄alkyl is independently optionally substituted with halogen, NR₁₁R₁₂; and wherein R₁₈ is selected from H, C₁-C₄alkyl preferably methyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; R₁₉ is selected from H, C₁-C₄alkyl; and wherein R₂₀ is selected from H, C₁-C₄alkyl, C(O)-R₂₂, C(O)-C₁-C₃alkyl-R₂₂, or N(H)C(O)-R₂₂, wherein R₂₂ is independently at each occurrence selected from aryl optionally substituted with C₁-C₄alkyl, halogen, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; wherein preferably said R₂₁ is H, and wherein the arrow indicates the attachment to the NH-moiety depicted in formula (I).

Further disclosed are substituents R₁ selected from 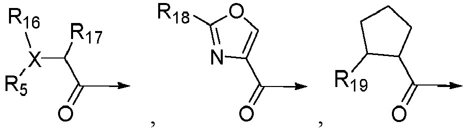 preferably 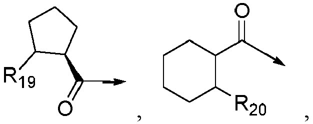 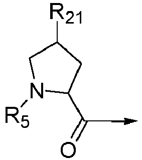 preferably 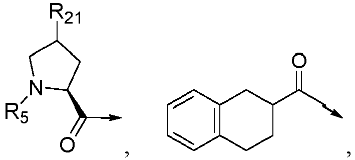 wherein X is N or CH; and wherein said R₅ is C(O)-R₈, wherein R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen, preferably flurorine, CF₃, OR₁₀, NR₁₁R₁₂; wherein R₁₀, R₁₁, R₁₂ are independently at each occurrence H, C₁-C₃alkyl; and wherein R₁₆, R₁₇ are independently at each occurrence selected from H, C₁-C₄alkyl, wherein said C₁-C₄alkyl is independently optionally substituted with halogen, NR₁₁R₁₂; and wherein R₁₈ is selected from H, C₁-C₄alkyl preferably methyl, halogen, CF₃, OR₁₀, NR₁₁R₁₂, C₆H₅ and C₆H₅ substituted with halogen, C₁-C₃alkyl, OR₁₀, NR₁₁R₁₂; wherein R₁₀, R₁₁, R₁₂ are independently at each occurrence H, C₁-C₃alkyl; R₁₉ is selected from H, C₁-C₄alkyl; and wherein R₂₀ is selected from H, C₁-C₄alkyl, C(O)-R₂₂, C(O)-C₁-C₃alkyl-R₂₂, or N(H)C(O)-R₂₂, wherein R₂₂ is independently at each occurrence selected from aryl optionally substituted with C₁-C₄alkyl, halogen, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; wherein preferably said R₂₁ is H, and wherein the arrow indicates the attachment to the NH-moiety depicted in formula (I).

We have surprisingly found that preferred compounds of the present invention maintain its high biological activity despite the removal of one or several stereocenters (present in the naturally occurring analogue of leucinostatin A) leading to more simplified and more economic syntheses of the inventive compounds without sacrificing biological activity.

Further disclosed are substituents R₁ selected from 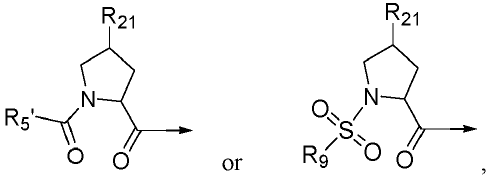 wherein R₅' is selected from C₁-C₁₆alkyl, C₁-C₁₆alkenyl independently optionally substituted with halogen, NR₁₁R₁₂, ―[O-C₂H₄]ₙ-OCH₃ wherein n=2-20; R₈, or -C₁-C₃alkylene-R₈, wherein R₈ is independently at each occurrence selected from aryl, heteroaryl, cycloalkyl, heterocyclyl, each independently optionally substituted with C₁-C₄alkyl, halogen, C₆H₅, CF₃, OR₁₀, NR₁₁R₁₂; wherein R₁₀, R₁₁, R₁₂ are independently at each occurrence H, C₁-C₃alkyl; and R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl; R₂₁ is selected from H, C₁-C₄alkyl, and wherein preferably R₂₁ is H; and the arrow indicates the attachment to the NH-moiety depicted in formula (I).

Further disclosed are substituents R₁ selected from 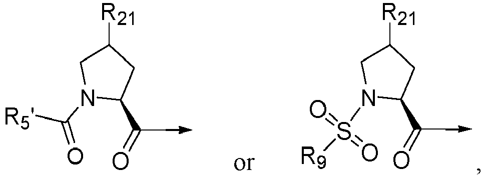 wherein R₅' is selected from C₁-C₁₆alkyl, C₁-C₁₆alkenyl independently optionally substituted with halogen, NR₁₁R₁₂, ―[O-C₂H₄]ₙ-OCH₃ wherein n=2-20; R₈, or -C₁-C₃alkylene-R₈, wherein R₈ is independently at each occurrence selected from aryl, heteroaryl, cycloalkyl, heterocyclyl, each independently optionally substituted with C₁-C₄alkyl, halogen, C₆H₅, CF₃, OR₁₀, NR₁₁R₁₂; wherein R₁₀, R₁₁, R₁₂ are independently at each occurrence H, C₁-C₃alkyl; and R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl; R₂₁ is selected from H, C₁-C₄alkyl, and wherein preferably R₂₁ is H; and the arrow indicates the attachment to the NH-moiety depicted in formula (I).

Further disclosed are substituents R₁ selected from 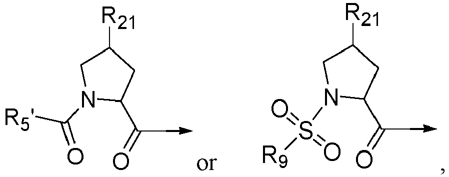 preferably 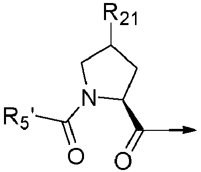 or 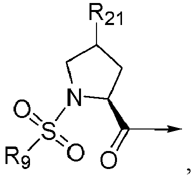 wherein R₅' is selected from C₁-C₁₆alkyl, C₁-C₁₆alkenyl independently optionally substituted with halogen, NR₁₁R₁₂, ―[O-C₂H₄]ₙ-OCH₃ wherein n=2-20; R₈, or -C₁-C₃alkylene-R₈, wherein R₈ is independently at each occurrence selected from aryl, heteroaryl, cycloalkyl, heterocyclyl, each independently optionally substituted with C₁-C₄alkyl, halogen, C₆H₅, CF₃, OR₁₀, NR₁₁R₁₂; wherein R₁₀, R₁₁, R₁₂ are independently at each occurrence H, C₁-C₃alkyl; and R₉ is independently at each occurrence selected from C₁-C₄alkyl, aryl, heteroaryl, each independently optionally substituted with C₁-C₄alkyl, halogen, CF₃, OR₁₃, NR₁₄R₁₅; wherein R₁₃, R₁₄, R₁₅ are independently at each occurrence H, C₁-C₃alkyl; the arrow indicates the attachment to the NH-moiety depicted in formula (I).

In a very preferred embodiment of the present invention, said R₁ is selected from 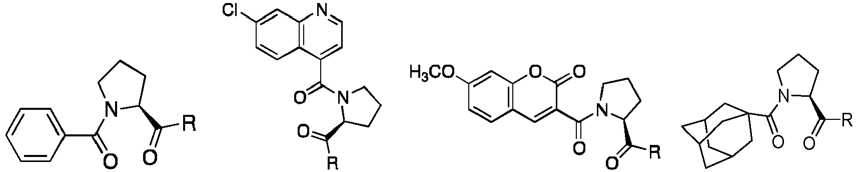 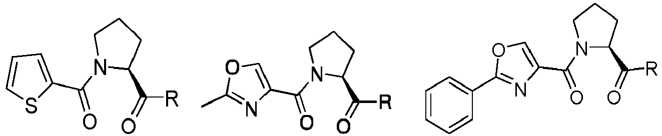 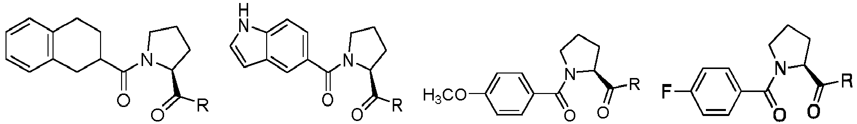 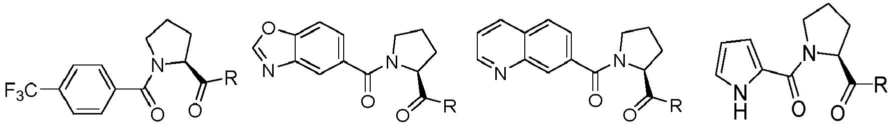 , wherein R indicates the attachment to the NH-moiety depicted in formula (I).

Further disclosed are substituents R₂ selected from C₂-C₁₄alkyl, C₂-C₁₄alkyl optionally substituted with OH, C₂-C₁₄alkoxy, C₂-C₁₄alkenyl optionally substituted with OH, C₁-C₈alkylene-R₂₃, wherein in alkylene one or two -CH₂- moieties are optionally replaced by - CH(NR₂₄R₂₅)-, -CH(OH)-, -C(=O)-, -NR₂₄R₂₅- or -CH(CH₃)- moieties, wherein there are no adjacent -C(=O)- moieties or adjacent -NR₂₄R₂₅- moieties, and wherein R₂₃ is independently at each occurrence selected from hydrogen; phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen, CF₃, OR₂₆, NR₂₇R₂₈; wherein R₂₆, R₂₇, R₂₈ are independently at each occurrence H, C₁-C₃alkyl; and wherein R₂₄ and R₂₅ are independently at each occurrence H, C₁-C₃alkyl; with the proviso that R₂ is not ― CH₂CH(CH₃)CH₂CH(OH)CH₂C(O)C₂H₅, ―CH₂CH(CH₃)CH₂CH=CHC(O)C₂H₅, ― CH₂CH(CH₃)CH₂CH₂CH₂C(O)C₂H₅, ―CH₂CH(CH₃)(CH₂)₃CH(OH)C₂H₅ or ― CH₂CH(CH₃)CH₂CH(OCH₃)CH₂C(O)C₂H₅.

Further disclosed are substituents R₂ selected from 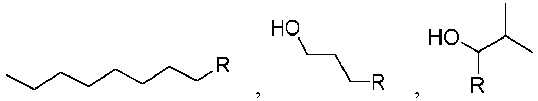 preferably 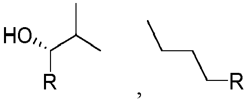 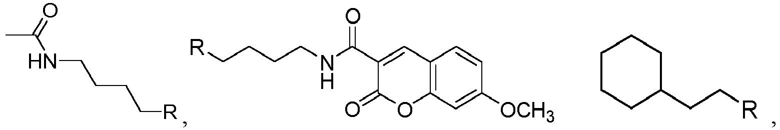 wherein R indicates the attachment to the CH-moiety depicted in formula (I).

Further disclosed are substituents R₃ selected from C₂-C₁₂alkyl optionally substituted with OH, C₂-C₁₂alkoxy, C₂-C₁₂alkenyl optionally substituted with OH, C₁-C₈alkylene-R₂₉, wherein in alkylene one or two -CH₂- moieties are optionally replaced by ―CH(NR₃₀R₃₁)-, ― CH(OH)-, or -CH(CH₃)- moieties, and wherein R₂₉ is independently at each occurrence selected from hydrogen; 1,2-dihydronapthalenyl, 1,2,3,4-tetrahydronaphthenyl, oxazolyl, pyrrolyl, quinolinyl, isoquinolinyl, indolyl, piperidinyl, morpholinyl benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl, each independently optionally substituted with C₁-C₄alkyl, OR₃₂, NR₃₃R₃₄; wherein R₃₂, R₃₃, R₃₄ are independently at each occurrence H, halogen, CF₃, C₁-C₃alkyl; and wherein R₃₀ and R₃₁ are independently at each occurrence H, C₁-C₃alkyl

Further disclosed are substituents R₃ selected from 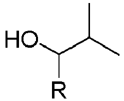 preferably 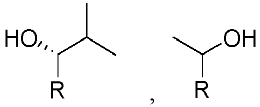 preferably 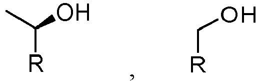 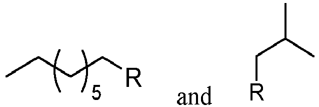 wherein R indicates the attachment to the CH-moiety depicted in formula (I). In a further very preferred embodiment, said R₄ is 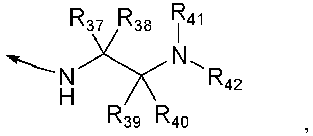 wherein R₃₇, R₃₈, R₃₉ and R₄₀ are independently at each occurrence hydrogen or C₁-C₃alkyl, preferably hydrogen or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein R₄₁ and R₄₂ are independently of each other hydrogen or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl; wherein the arrow indicates the attachment to the C(O)-moiety depicted in formula (I).

In a further very preferred embodiment, said R₄ is selected from 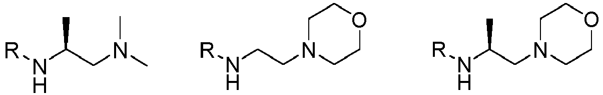 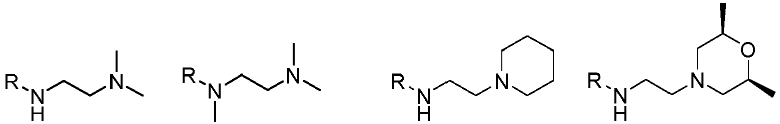 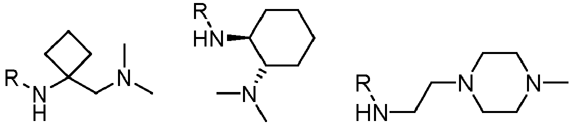 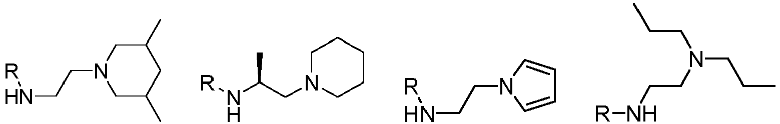 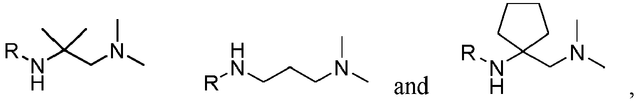 wherein R indicates the attachment to the C(O)-moiety depicted in formula (I).

In a further very preferred embodiment, said compound is selected from
**5649:** (2S)-2-{[(2S)-1-benzoylpyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide;
**5768:** (2S)-2-{[(2S)-1-(7-chloroquinoline-4-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide;
**5769:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(7-methoxy-2-oxo-2H-chromene-3-carbonyl)pyrrolidin-2-yl]formamido}decanamide;
**5770:** (2S)-2-{[(2S)-1-(adamantane-1-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide;
**5904:** (2S)-N-[(1{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(thiophene-2-carbonyl)pyrrolidin-2-yl]formamido}decanamide;
**5905:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(2-methyl-1,3-oxazole-4-carbonyl)pyrrolidin-2-yl]formamido}decanamide;
**5910:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl] carbamoyl}-3-methylbutyl]carbamoyl}-3-methylbutyl]carbamoyl}-1-methylethyl)carbamoyl]-3-methylbutyl]-2-{[(2S)-1-(1,2,3,4-tetrahydronaphthalene-2-carbonyl)pyrrolidin-2-yl]formamido}decanamide;
**5911:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(1H-indole-5-carbonyl)pyrrolidin-2-yl]formamido}decanamide;
**5912:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(4-methoxybenzoyl)pyrrolidin-2-yl]formamido}decanamide;
**5934:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(4-fluorobenzoyl)pyrrolidin-2-yl]formamido}decanamide;
**5936:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-[4-(trifluoromethyl)benzoyl]pyrrolidin-2-yl]formamido}decanamide;
**5937:** (2S)-2-{[(2S)-1-(1,3-benzoxazole-5-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide;
**5938:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(quinoline-7-carbonyl)pyrrolidin-2-yl]formamido}decanamide;
**5939:** (2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(1H-pyrrole-2-carbonyl)pyrrolidin-2-yl]formamido}decanamide;
**6025:** (2S)-2-[(2S)-4-cyclohexyl-2-{[(2S)-1-(2-methyl-1,3-oxazole-4-carbonyl)pyrrolidin-2-yl]formamido}butanamido]-N-(1-{[(1S)-1-{[(1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)-4-methylpentanamide;
**6026:** (2S)-2-[(2S)-2-{2-[(2S)-2-[(2S)-4-cyclohexyl-2-{[(2S)-1-(2-methyl-1,3-oxazole-4-carbonyl)pyrrolidin-2-yl]formamido}butanamido]-4-methylpentanamido]-2-methylpropanamido}-4-methylpentanamido]-4-methyl-N-[1-methyl-1-({1-methyl-1-[(2-{[(2S)-1-(morpholin-4-yl)propan-2-yl]carbamoyl}ethyl)carbamoyl]ethyl}carbamoyl)ethyl]pentanamide;
**6027:** (2S)-2-[(2S)-4-cyclohexyl-2-{[(2S)-1-(4-fluorobenzoyl)pyrrolidin-2-yl]formamido}butanamido]-N-(1-{[(1S)-1-{[1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)-4-methylpentanamide;
**6028:** (2S)-2-[(2S)-2-{2-[(2S)-2-[(2S)-4-cyclohexyl-2-{[(2S)-1-(4-fluorobenzoyl)pyrrolidin-2-yl]formamido}butanamido]-4-methylpentanamido]-2-methylpropanamido}-4-methylpentanamido]-4-methyl-N-[1-methyl-1-({1-methyl-1-[(2-{[(2S)-1-(morpholin-4-yl)propan-2-yl]carbamoyl}ethyl)carbamoyl]ethyl}carbamoyl)ethyl]pentanamide;
**6253:** (2S)-N-[(1S)-1-[(1-{[(1S)-1-{[(1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)carbamoyl]-3-methylbutyl]-2-{[(2S)-1-(2-methyl-1,3-oxazole-4-carbonyl)pyrrolidin-2-yl]formamido}decanamide;
**6254:** (2S)-N-[(1S)-1-[(1-{[(1S)-1-{[(1S)-1-({1-[(1-{[2-({1-[(dimethylamino)methyl]cyclopentyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)carbamoyl]-3-methylbutyl]-2-{[(2S)-1-(2-methyl-1,3-oxazole-4-carbonyl)pyrrolidin-2-yl]formamido}decanamide;

In a further very preferred embodiment, said compound is selected from a formula as depicted in claim 11 and selected from **5649, 5768, 5769, 5770, 5904, 5905, 5910, 5911, 5912, 5934, 5936, 5937, 5938, 5939, 6025, 6026, 6027, 6028, 6253** or **6254**, .

In a further aspect, the present invention provides for a compound of formula (I) for use in a method of treatment of treatment of infectious diseases.

In a further aspect, the present invention provides for a compound of formula (I) for use in a method of treatment of a protozoan disease, wherein preferably said protozoan disease is selected from malaria, human African trypanosomiasis, Chagas disease or leishmaniasis, and wherein further preferably said protozoan disease is Chagas disease or leishmaniasis.

In a further aspect, the present invention provides for a compound of formula (I) for use in a method of treatment of a protozoan disease, wherein preferably said protozoan disease is selected from acute malaria, cerebral malaria, human African trypanosomiasis, Chagas disease, cutaneous leishmaniasis (CL), mucocutaneous leishmaniasis (MCL) or visceral leishmaniasis (VL), and wherein further preferably said protozoan disease is selected from Chagas disease, cutaneous leishmaniasis (CL), mucocutaneous leishmaniasis (MCL) or visceral leishmaniasis (VL).

In a further aspect, the present invention provides for a compound of formula (I) for use in a method of treatment of a condition, disease or disorder caused by a parasite selected from a parasite of the *Plasmodium* genus, a parasite of the *Trypanosoma* genus or a parasite of the *Leishmania* genus, wherein preferably said parasite is selected from *Trypanosoma cruzi, Leishmania donovani, Plasmodium falciparum, Plasmodium vivax, Plasmodium ovale, Plasmodium malaria,* and wherein further preferably said parasite is selected from *Trypanosoma cruzi* or *Leishmania donovani.*

In a further aspect, the present invention provides for a compound of formula (I) for use in a method of treatment of leishmaniasis, and wherein preferably said leishmaniasis is cutaneous leishmaniasis (CL), mucocutaneous leishmaniasis (MCL) or visceral leishmaniasis (VL), and again further preferably wherein said leishmaniasis is visceral leishmaniasis (VL).

In a further aspect, the present invention provides for a compound of formula (I) for use in a method of treatment of Chagas disease.

Further disclosed is a method of inhibiting proliferation of Leishmania parasites in a patient for prophylaxis or to a patient in need of treatment which comprises administering to said patient an effective amount of a compound of formula (I). Preferably said compound of formula (I) has an *in vitro* selectivity index greater than Miltefosine. In another preferred embodiment, said compound has an IC50 value against *Leishmania donovani* promastigotes and amastigotes lower than Miltefosine.

Further disclosed is a method for the treatment of a condition, disease or disorder caused by a parasite selected from a parasite of the *Plasmodium* genus, a parasite of the *Trypanosoma* genus or a parasite of the *Leishmania* genus, wherein preferably said parasite is selected from *Trypanosoma cruzi, Leishmania donovani, Plasmodium falciparum, Plasmodium vivax, Plasmodium ovale, Plasmodium malaria,* comprising administration of a therapeutically effective amount of a compound of formula (I), to a subject, preferably to a human subject, in need of such treatment. In another preferred embodiment, said parasite is selected from *Trypanosoma cruzi* or *Leishmania donovani.*

Further disclosed is a method of treating a patient for prophylaxis or to a patient in need of treatment for leishmaniasis comprising the step of administering to said patient a therapeutically effective amount of a compound of formula (I).

Further disclosed is a method of treating a patient for prophylaxis or to a patient in need of treatment for Chagas disease comprising the step of administering to said patient a therapeutically effective amount of a compound of formula (I).

In a further aspect, the present invention provides for a pharmaceutical composition comprising a compound of formula (I) and a pharmaceutically acceptable carrier or adjuvant, wherein said pharmaceutical composition is preferably formulated for use in inhibiting proliferation of a *Leishmania* parasite in a patient for prophylaxis or to a patient in need of treatment for leishmaniasis.

In a further aspect, the present invention provides for a pharmaceutical composition comprising a compound of formula (I) and a pharmaceutically acceptable carrier or adjuvant, wherein said pharmaceutical composition is preferably formulated for use in inhibiting proliferation of a *Trypanosoma cruzi* parasite in a patient for prophylaxis or to a patient in need of treatment for Chagas disease.

Those skilled in the art will recognize that modifications and variations may be made without departing from the true spirit and scope of the invention. The invention, therefore, is not to be limited to the embodiments described and illustrated in the following non-limiting examples but is to be determined from the appended claims.

### EXAMPLES

All reagents and solvents used in the synthesis were purchased from Sigma Aldrich, Bachem, Iris Biotech, Fluorochem, Enamine, UkrOrgSyntez, Polypeptide and used as received. (4S, E)-4-Methyl-2-hexenoic acid was synthesized according to a procedure reported in literature (Tetrahedron Letters, 1991, 32, 3985-3988). Solvents were stored over molecular sieves 4 Å. Peptides were synthesized manually by Solid Phase Peptide Synthesis (SPPS) using the standard Fmoc/tert-butyl protocol. Peptide acids were synthesized on Wang resin loaded with the Fmoc-beta-Ala (0.75 mmol/g, particle size 200- 400 mesh, Bachem). Synthesis was performed using double coupling (each for 30 min or 60 min depending on the amino acid), Fmoc-protected amino acids or free acid (3 equiv) and COMU (3 equiv) as coupling reagent, in the presence of DIPEA (6 equiv) in dimethylformamide (DMF). Fmoc-removal was performed twice with 20% piperidine/DMF solution (v/v) for 3 min. When necessary the coupling cycle was followed by acetic anhydride capping step. Washing steps were carried out with DMF and isopropanol. The final cleavage of the peptide from the resin and deprotection of amino acids side chain were achieved at room temperature in a mixture of TFA/H₂O/TIPS (95:2.5:2.5) for 90 min at room temperature. The crude peptides were dissolved in water/acetonitrile 1/1 (v/v) and were purified by semi preparative HPLC Gilson PLC 2020 Personal Purification System using as mobile phase H₂O/acetonitrile (95:5, v/v) + 0.2% acetic acid and acetonitrile/ H₂O + 0.2% acetic acid, gradient ACN 10% to 100%, to give peptide acids ≥90% pure. The purified peptide acid (1 equiv) was coupled in solution with suitable primary or secondary amines (2 equiv) in presence of DIPEA (3 equiv) and COMU (2 equiv) to give the final peptide. Solvent was partially removed, and the crude peptide was directly purified by Gilson PLC 2020 Personal Purification System, as described above, to give final products ≥95% pure.

High-resolution mass spectrometry was performed on an Agilent Technologies 6530 Q-TOF. Mass and purity of final compounds was determined by UPLC-MS using a Waters Acquity system equipped with a Waters BEH C18 column and diode array detector (254 nm). The mobile phase consisted of H2O-Acetonitrile (solvent A, 97:3 v/v, LC-MS Ultra Chromasolv®, UHPLC grade, Sigma-Aldrich, Germany) and 0.1 % Formic acid (LC-MS grade, Sigma-Aldrich, Germany) and Acetonitrile-H2O (solvent B, 97:3 v/v, LC-MS Ultra Chromasolv®, UHPLC grade, Sigma-Aldrich, Germany) and 0.1 % Formic acid (LC-MS grade, Sigma-Aldrich, Germany) with a 4 min run time, 0.6 mL/min flow rate, 10 µL injection volume and a gradient elution according to the following program: linear increment starting with 100 % A to 100 % B in 3 min and returning to the initial conditions within the next 1 min. MS detection of analytes was performed on a Waters Xevo® triple quadrupole mass spectrometer equipped with electrospray ionization (ESI) interface (Waters, Xevo® TQD QCA065) in positive and negative ion mode with mass range from 100-1500 m/z.

### EXAMPLE 1

### General procedure for the synthesis of the inventive compounds

The inventive compounds were synthesized manually by Solid Phase Peptide Syntheis (SPPS) using the standard Fmoc/tert-butyl protocol starting from Fmoc-beta-Ala linked to Wang resin as depicted in Scheme 1. Washing steps were carried out with DMF and isopropanol. Fmoc-removal was performed with 20% piperidine/DMF solution (v/v) for 3 min, repeated twice. By employing sequentially the building blocks listed in Table 1 (Amino acids used in steps 1 to 8, Scheme 1) and free acid or sulfonyl chloride derivatives (step 9, Scheme 1) listed in Table 2, the different peptides were assembled on solid phase support in typically and preferably 9 steps. Cleavage of the peptide from resin and deprotection of amino acids side chain was achieved in a mixture of TFA/H2O/TIPS (95:2.5:2.5) for 90 min at room temperature. The crude peptides were were purified by semi preparative HPLC Gilson PLC 2020 Personal Purification System. The purified peptide acid was coupled in solution with suitable primary or secondary amines reported in Table 3 (step 10, Scheme 1) in presence of DIPEA and COMU to give the final peptide, which was then directly purified on Gilson PLC 2020 Personal Purification System.

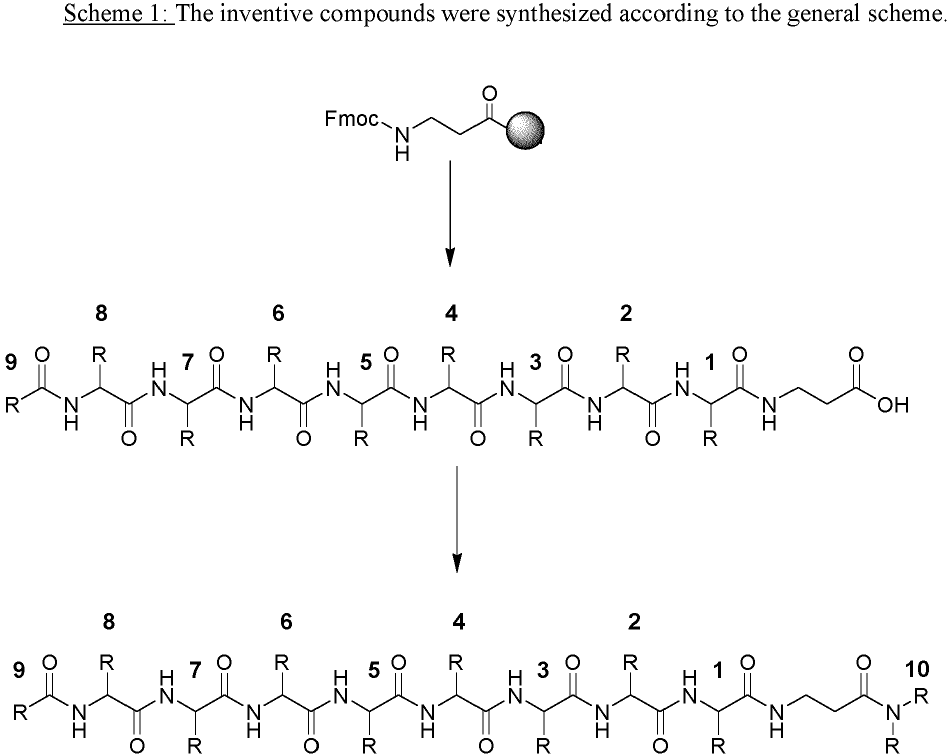

**Table 1: Amino acid building blocks used for the synthesis of the inventive compounds**

| **AMINO ACIDS** | | |
|---|---|---|
| | **Structure** | **IUPAC Name** |
| Fmoc-Aib-OH | 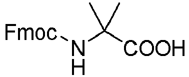 | 2-((((9H-fluoren-9-yl)methoxy)carbonyl)amino )-2-methylpropanoic acid |
| Fmoc-Leu-OH | 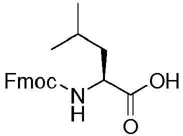 | (((9H-fluoren-9-yl)methoxy)carbonyl)-L-leucine |
| Fmoc-Hydroxy-Leu-OH | 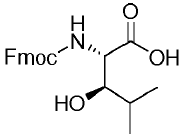 | (2S,3R)-2-((((9H-fluoren-9-yl)methoxy)carbonyl)amino )-3-hydroxy-4-methylpentanoic acid |
| Fmoc-(S)-2-aminodecanoic acid | 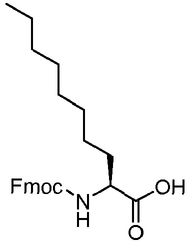 | (S)-2-((((9H-fluoren-9-yl)methoxy)carbonyl)amino )decanoic acid |
| Fmoc- Pro-OH | 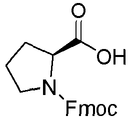 | (((9H-fluoren-9-yl)methoxy)carbonyl)-L-proline |
| Fmoc-Me-Pro-OH | 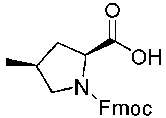 | (2S,4S)-1-(((9H-fluoren-9-yl)methoxy)carbonyl)-4-methylpyrrolidine-2-carboxylic acid |
| Fmoc-O-trityl-5-hydroxy- L-norvaline | 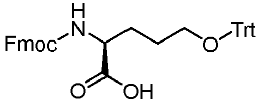 | (S)-2-((((9H-fluoren-9-yl)methoxy)carbonyl)amino )-5-(trityloxy)pentanoic acid |
| Fmoc-Thr-OH | 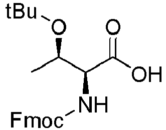 | N-(((9H-fluoren-9-yl)methoxy)carbonyl)-O-(tert-butyl)- L-threonine |
| Fmoc-Ser-OH | 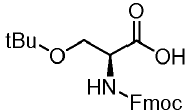 | N-(((9H-fluoren-9-yl)methoxy)carbonyl)-O-(tert-butyl)- L-serine |
| Fmoc-Nle-OH | 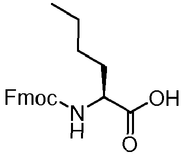 | (S)-2-((((9H-fluoren-9-yl)methoxy)carbonyl)amino )hexanoic acid |
| Fmoc- Ala-OH | 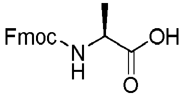 | (((9H-fluoren-9-yl)methoxy)carbonyl)-L-alanine |
| Fmoc-acetyl-lysine | 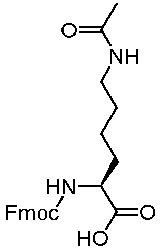 | N2-(((9H-fluoren-9-yl)methoxy)carbonyl)-N⁶-acetyl-L-lysine |
| Fmoc-N⁶-7-Methoxycoumarin-3-carboxyl-L-lysine | 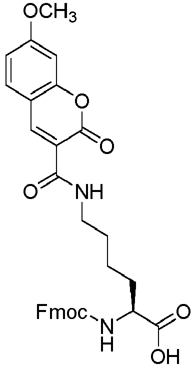 | N2-(((9H-fluoren-9-yl)methoxy)carbonyl)-N⁶-(7-methoxy-2-oxo-2H-chromene-3-carbonyl)-L-lysine |
| Fmoc-L-homocyclohexylalanine | 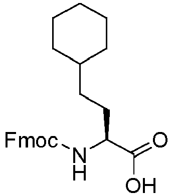 | (S)-2-((((9H-fluoren-9-yl)methoxy)carbonyl)amino )-4-cyclohexylbutanoic acid |
| Fmoc-D-Leu-OH | 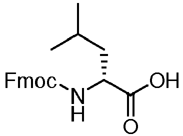 | (((9H-fluoren-9-yl)methoxy)carbonyl)-D-leucine |
| Fmoc-(R)-2-aminodecanoic acid | 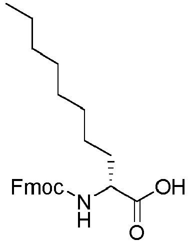 | (R)-2-((((9H-fluoren-9-yl)methoxy)carbonyl)amino )decanoic acid |
| Fmoc-D-Pro-OH | 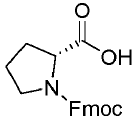 | (((9H-fluoren-9-yl)methoxy)carbonyl)-D-proline |

**Table 2: Free acids or sulfonyl chloride derivatives used for the synthesis of the inventive compounds (step 9, Scheme 1).**

| **ACIDS** | | |
|---|---|---|
| | **Structure** | **IUPAC Name** |
| (S,E)-4-methylhex-2-enoic acid | 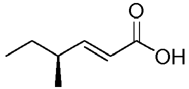 | (S,E)-4-methylhex-2-enoic acid |
| Hex-2-enoic acid | 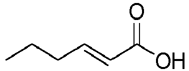 | (E)-hex-2-enoic acid |
| Hexanoic acid | 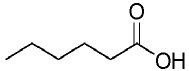 | Hexanoic acid |
| Acetic acid | 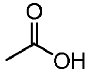 | Acetic acid |
| Oxazole-4-carboxylic acid | 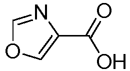 | Oxazole-4-carboxylic acid |
| 2-methyloxazole-4-carboxylic acid | 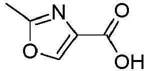 | 2-methyloxazole-4-carboxylic acid |
| Cyclopentanecarboxylic acid | 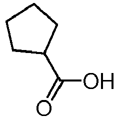 | Cyclopentanecarboxylic acid |
| Benzoic acid | 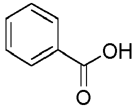 | Benzoic acid |
| 3-Morpholinopropanoic acid | 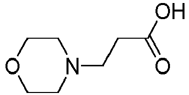 | 3-Morpholinopropanoic acid |
| Dodecanoic acid | 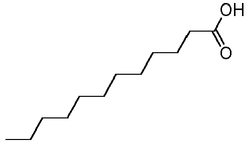 | Dodecanoic acid |
| Methanesulfonyl chloride | 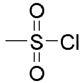 | Methanesulfonyl chloride |
| 5-(dimethylamino)naphthale ne-1-sulfonyl chloride | 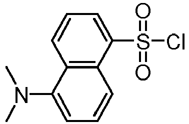 | 5-(dimethylamino)naphthalen e-1-sulfonyl chloride |
| 7-Chloroquinoline-4-carboxylic acid | 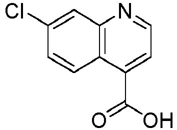 | 7-Chloroquinoline-4-carboxylic acid |
| 7-Methoxycoumarin-3-carboxylic acid | 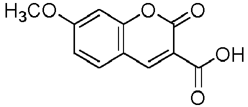 | 7-Methoxy-2-oxo-2H-chromene-3-carboxylic acid |
| 1-Adamantanecarboxylic acid | 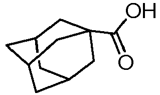 | Adamantane- 1 -carboxylic acid |
| 2-(Adamantan- 1 -yl)acetic acid | 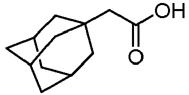 | 2-(Adamantan- 1 -yl)acetic acid |
| | | |
| Thiophene-2-carboxylic acid | 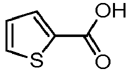 | Thiophene-2-carboxylic acid |
| 2-Phenyloxazole-4-carboxylic acid | 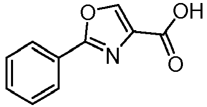 | 2-Phenyloxazole-4-carboxylic acid |
| Tetrahydronaphthalene-2-carboxylic acid | 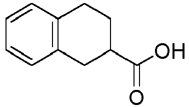 | 1,2,3,4-Tetrahydronaphthalene-2-carboxylic acid |
| 1H-indole-5-carboxylic acid | 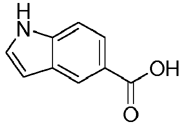 | 1H-indole-5-carboxylic acid |
| 4-Methoxybenzoic acid | 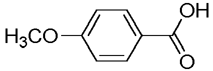 | 4-Methoxybenzoic acid |
| 4-Fluorobenzoic acid | 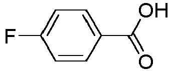 | 4-Fluorobenzoic acid |
| 4-Fluorobenzenesulfonyl chloride | 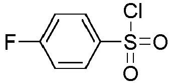 | 4-Fluorobenzenesulfonyl chloride |
| 4-(trifluoromethyl)benzoic acid | 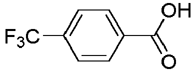 | 4-(trifluoromethyl)benzoic acid |
| Benzo[d]oxazole-5-carboxylic acid | 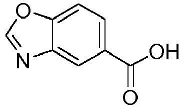 | Benzo[d]oxazole-5-carboxylic acid |
| Quinoline-7-carboxylic acid | 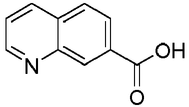 | Quinoline-7-carboxylic acid |
| 1H-pyrrole-2-carboxylic acid | 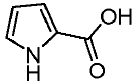 | 1H-pyrrole-2-carboxylic acid |
| (1R,2S)-2-benzoylcyclohexane-1-carboxylic acid | 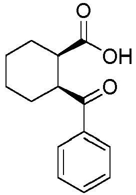 | (1R,2S)-2-benzoylcyclohexane-1-carboxylic acid |
| (1S,2R)-2-benzamidocyclohexane-1-carboxylic acid | 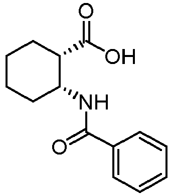 | (1S,2R)-2-benzamidocyclohexane-1-carboxylic acid |
| mPEG acid (n=11) | 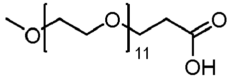 | O-(2-Carboxyethyl)-O'-methyl-undecaethylene glycol |
| Tetradecanoic acid | 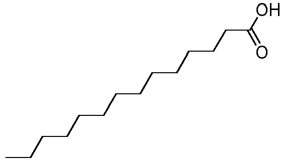 | Tetradecanoic acid |

**Table 3: Primary or secondary amines used for the synthesis of the inventive compounds (step 10, Scheme 1).**

| **AMINES** | | |
|---|---|---|
| **Short Name** | **Structure** | **IUPAC Name** |
| (S)-N¹,N¹-dimethylpropane-1,2-diamine | 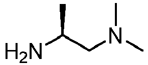 | (S)-N¹,N¹⁻dimethylpropane-1,2-diamine |
| 2-Morpholinoethan-1-amine | 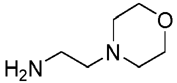 | 2-Morpholinoethan-1-amine |
| 1 -Methylpiperazine | 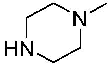 | 1 -Methylpiperazine |
| Morpholine | 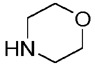 | Morpholine |
| (S)-1-morpholinopropan-2-amine | 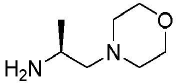 | (S)-1-morpholinopropan-2-amine |
| N¹,N¹-dimethylethane-1,2-diamine | 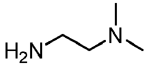 | N1,N1-dimethylethane-1,2-diamine |
| N¹,N¹,N²-trimethylethane-1,2-diamine | 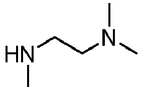 | N¹,N¹,N²-trimethylethane-1,2-diamine |
| Cyclopropanamine | 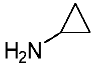 | Cyclopropanamine |
| 2-(Piperidin-1-yl)ethan-1-amine | 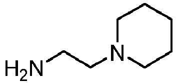 | 2-(Piperidin-1-yl)ethan-1-amine |
| 2-(2,6-dimethylmorpholino)ethan -1-amine | 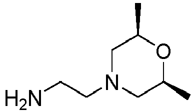 | 2-((2S,6R)-2,6-dimethylmorpholino)ethan-1-amine |
| N¹,N¹-dimethylcyclohexane-1,4-diamine | 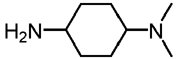 | N¹,N¹-dimethylcyclohexane-1,4-diamine |
| 1-((dimethylamino)methyl)c yclobutan-1-amine | 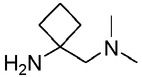 | 1-((dimethylamino)methyl)cy clobutan-1-amine |
| N¹,N¹-dimethylcyclohexane -1,2-diamine | 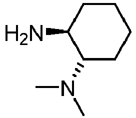 | (1S,2S)-N¹N¹-dimethylcyclohexane -1,2-diamine |
| 2-(4-methylpiperazin-1-yl)ethan-1-amine | 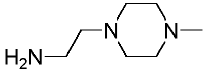 | 2-(4-methylpiperazin-1 - yl)ethan-1-amine |
| Dimethylpiperidin-1-yl ethan-1-amine | 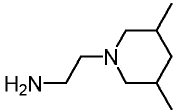 | 2-(3,5-Dimethylpiperidin-1-yl) ethan-1-amine |
| (S)-1-(piperidin-1-yl)propan-2-amine | 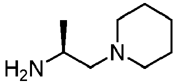 | (S)-1-(piperidin-1-yl)propan-2-amine |
| 2-(1H-pyrrol-1-yl)ethan-1-amine | 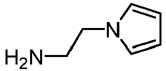 | 2-(1H-pyrrol-1-yl)ethan-1-amine |
| N¹,N¹-dipropylethane-1,2-diamine | 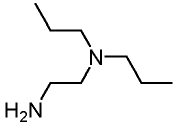 | N¹,N¹-dipropylethane-1,2-diamine |
| N¹,N¹,2-trimethylpropane-1,2-diamine | 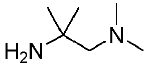 | N¹,N¹,2-trimethylpropane-1,2-diamine |
| N¹,N¹-dimethylpropane-1,3-diamine | 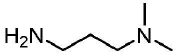 | N¹,N¹-dimethylpropane-1,3-diamine |
| 1-((dimethylamino)methyl)c yclopentan-1-amine | 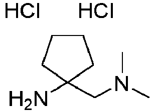 | 1-((dimethylamino)methyl)cy clopentan-1-amine dihydrochloride |
| 1-Methyl-1,6-diazaspiro[3.3]heptane | 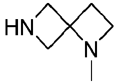 | 1-Methyl-1,6-diazaspiro[3.3]heptane |
| 2-Methyl-2,6-diazaspiro[3.3]heptane | 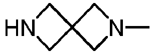 | 2-Methyl-2,6-diazaspiro[3.3]heptane |
| | | |

### EXAMPLE 2

### Synthesis of the inventive compounds

In an analogous manner the inventive compounds have been synthesized as described in the following with reference to general procedure depicted in Scheme 1. Each of the synthesized compounds are identified by its compound number (4-digit number), its structural formula and its IUPAC name as generated by the ChemDraw software. Furthermore, the synthesis of each compound is described by way of the building blocks, acids and amines used as disclosed in Tables 1-3. Moreover, the experimentally measured molecular mass is provided. Finally, for each inventive compound the caracterization of the substituients R₁, R₂, R₃ and R₄ in accordance with formula (I) is given.

**5649:**

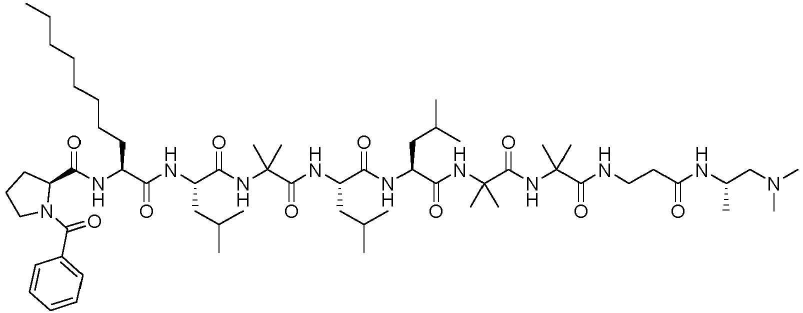

(2S)-2-{[(2S)-1-benzoylpyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide.

| **Cpd** | **1** | **2** | **3** | **4** | **5** |
|---|---|---|---|---|---|
| **5649** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | Benzoic acid | (S)-N¹,N¹-dimethylpropan e-1,2-diamine |

| **Cpd** | **Molecular mass** | **Experimentally measured mass** |
|---|---|---|
| **5649** | 1137.79 | 1138.73 |

| **Cpd** | **R1** | **R2** | **R3** | **R4** |
|---|---|---|---|---|
| **5649** | 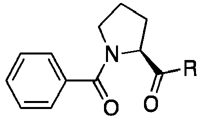 | 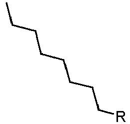 | 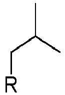 | 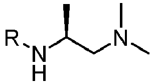 |

**5768:**

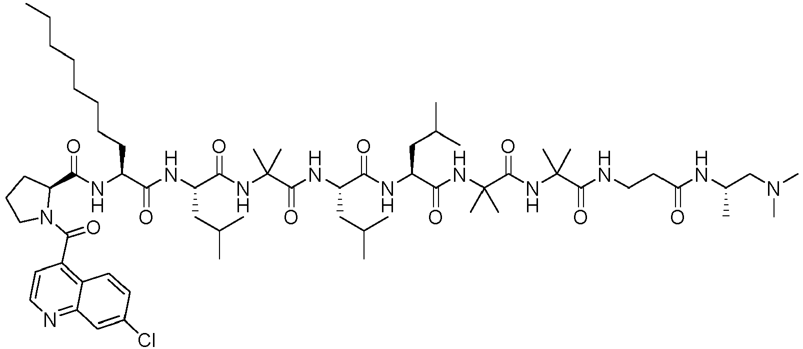

(2S)-2-{[(2S)-1-(7-chloroquinoline-4-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide.

| **Cpd** | **1** | **2** | **3** | **4** | **5** |
|---|---|---|---|---|---|
| **5768** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | 7-Chloroquinoli ne-4-carboxylic acid | (S)-N¹,N¹-dimethylpropan e-1,2-diamine |

| **Cpd** | **Molecular mass** | **Experimentally measured mass** |
|---|---|---|
| **5768** | 1222.76 | 1223.43 |

| **Cpd** | **R1** | **R2** | **R3** | **R4** |
|---|---|---|---|---|
| **5768** | 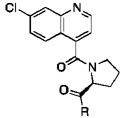 | 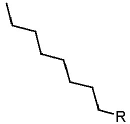 | 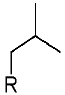 | 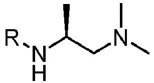 |

**5769:**

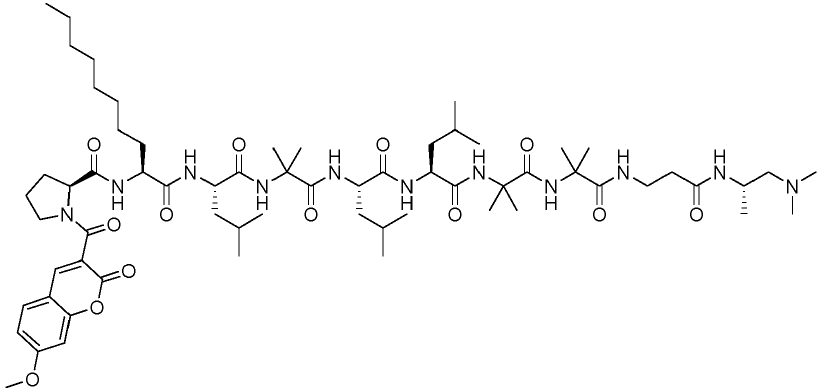

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3 -methylbutyl]carbamoyl}-1-methylethyl)carbamoyl]-3 - methylbutyl]-2-{[(2S)-1-(7-methoxy-2-oxo-2H-chromene-3-carbonyl)pyrrolidin-2-yl]formamido}decanamide.

| Cpd | 1 | 2 | 3 | 4 | 5 |
|---|---|---|---|---|---|
| 5769 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | 7-Methoxy coumarin-3-carboxylic acid | (S)-N¹,N¹-dimethylpropan e-1,2-diamine |

| **Cpd** | **Molecular mass** | **Experimentally measured mass** |
|---|---|---|
| 5769 | 1235.79 | 1236.52 |

| **Cpd** | **R1** | **R2** | **R3** | **R4** |
|---|---|---|---|---|
| 5769 | 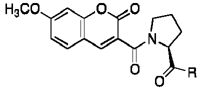 | 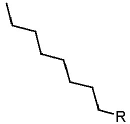 | 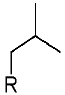 | 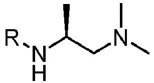 |

**5770:**

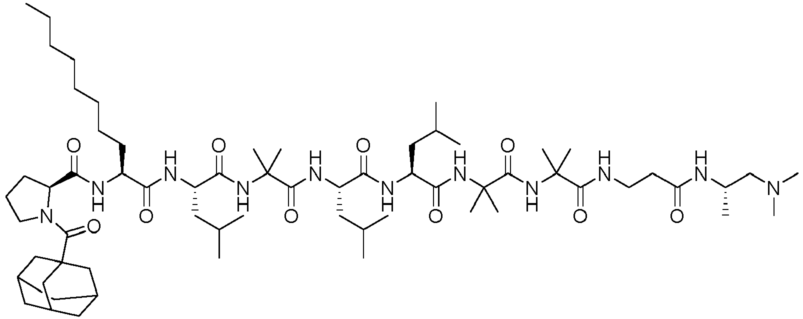

(2S)-2-{[(2S)-1-(adamantane-1-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide.

| Cpd | 1 | 2 | 3 | 4 | 5 |
|---|---|---|---|---|---|
| 5770 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | Adamantane-1-carboxylic acid | (S)-N¹,N¹-dimethylpropan e-1,2-diamine |

| **Cpd** | **Molecular mass** | **Experimentally measured mass** |
|---|---|---|
| 5770 | 1195.87 | 1196.76 |

| **Cpd** | **R1** | **R2** | **R3** | **R4** |
|---|---|---|---|---|
| 5770 | 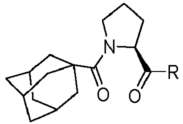 | 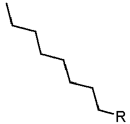 | 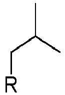 | 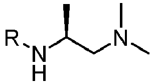 |

**5904:**

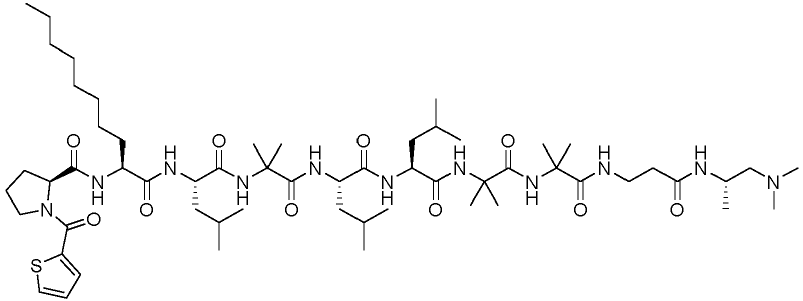

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3 - methylbutyl]-2-{[(2S)-1-(thiophene-2-carbonyl)pyrrolidin-2-yl]formamido}decanamide.

| **Cpd** | **1** | **2** | **3** | **4** | **5** |
|---|---|---|---|---|---|
| **5904** | Fmoc-Aib-OH | Fmoc-Aib-OH | Fmoc-Leu-OH | Fmoc-Leu-OH | Fmoc-Aib-OH |
| | **6** | **7** | **8** | **9** | **10** |
| | Fmoc-Leu-OH | Fmoc-(S)-2-aminodecanoic acid | Fmoc- Pro-OH | Thiophene-2-carboxylic acid | (S)-N¹,N¹-dimethylpropan e-1,2-diamine |

| **Cpd** | **Molecular mass** | **Experimentally measured mass** |
|---|---|---|
| **5904** | 1143.75 | 1144.61 |

| **Cpd** | **R1** | **R2** | **R3** | **R4** |
|---|---|---|---|---|
| **5904** | 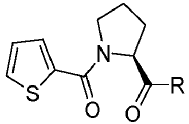 | 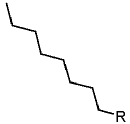 | 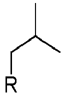 | 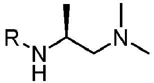 |

**5905:**

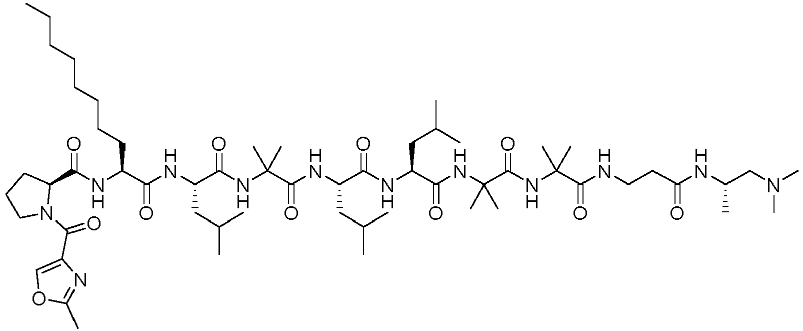

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3 - methylbutyl]-2-{[(2S)-1-(2-methyl-1,3-oxazole-4-carbonyl)pyrrolidin-2-yl]formamido}decanamide.

| **Cpd** | **1** | **2** | **3** | **4** | **5** |
|---|---|---|---|---|---|
| **5905** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | Oxazole-4-carboxylic acid | (S)-N¹,N¹-dimethylpropan e-1,2-diamine |

| **Cpd** | **Molecular mass** | **Experimentally measured mass** |
|---|---|---|
| **5905** | 1142.78 | 1143.56 |

| **Cpd** | **R1** | **R2** | **R3** | **R4** |
|---|---|---|---|---|
| **5905** | 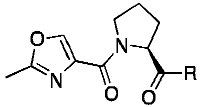 | 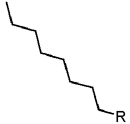 | 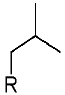 | 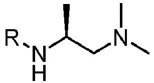 |

**5910:**

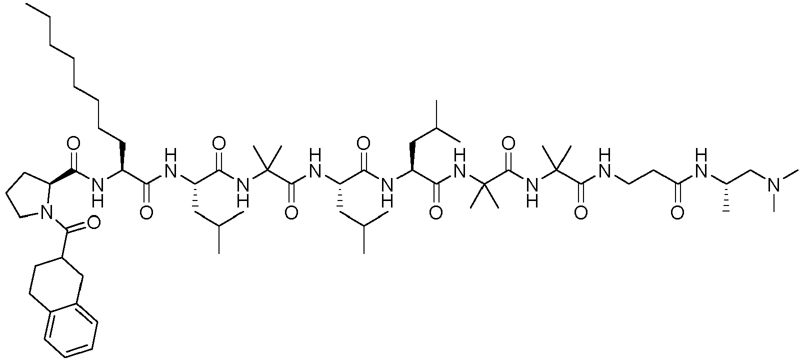

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3 - methylbutyl]-2-{[(2S)-1-(1,2,3,4-tetrahydronaphthalene-2-carbonyl)pyrrolidin-2-yl]formamido}decanamide.

| **Cpd** | **1** | **2** | **3** | **4** | **5** |
|---|---|---|---|---|---|
| **5910** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | Tetrahydronap hthalene-2-carboxylic acid | (S)-N¹,N¹-dimethylpropan e-1,2-diamine |

| **Cpd** | **Molecular mass** | **Experimentally measured mass** |
|---|---|---|
| **5910** | 1191.84 | 1190.70 |

| **Cpd** | **R1** | **R2** | **R3** | **R4** |
|---|---|---|---|---|
| **5910** | 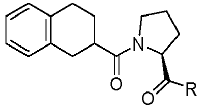 | 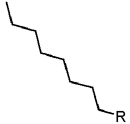 | 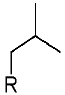 | 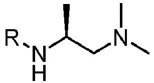 |

**5911:**

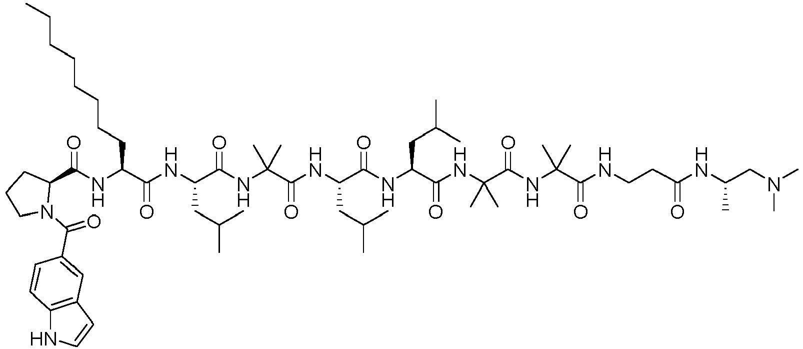

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(1H-indole-5-carbonyl)pyrrolidin-2-yl]formamido}decanamide.

| **Cpd** | **1** | **2** | **3** | **4** | **5** |
|---|---|---|---|---|---|
| **5911** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | 1H-indole-5-carboxylic acid | (S)-N¹,N¹-dimethylpropan e-1,2-diamine |

| **Cpd** | **Molecular mass** | **Experimentally measured mass** |
|---|---|---|
| **5911** | 1176.80 | 1177.58 |

| **Cpd** | **R1** | **R2** | **R3** | **R4** |
|---|---|---|---|---|
| **5911** | 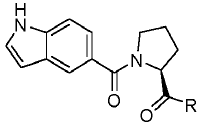 | 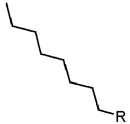 | 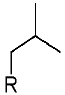 | 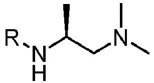 |

**5912:**

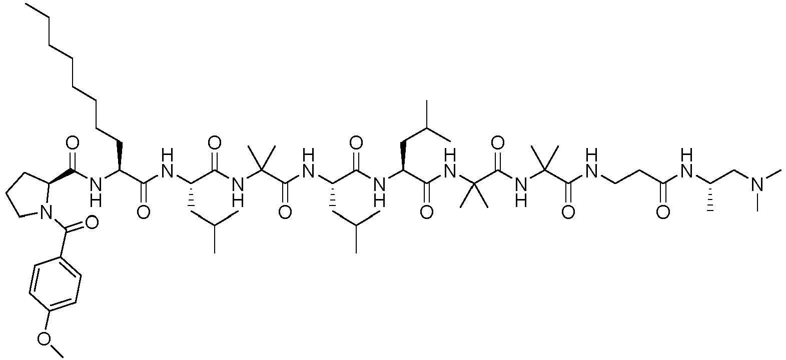

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(4-methoxybenzoyl)pyrrolidin-2-yl]formamido}decanamide.

| Cpd | 1 | 2 | 3 | 4 | 5 |
|---|---|---|---|---|---|
| 5912 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | 4-Methoxybenzo ic acid | (S)-N¹,N¹-dimethylpropan e-1,2-diamine |

| **Cpd** | **Molecular mass** | **Experimentally measured mass** |
|---|---|---|
| 5912 | 1167.80 | 1168.62 |

| **Cpd** | **R1** | **R2** | **R3** | **R4** |
|---|---|---|---|---|
| 5912 | 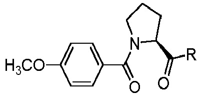 | 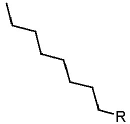 | 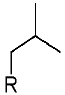 | 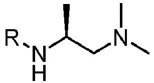 |

**5934:**

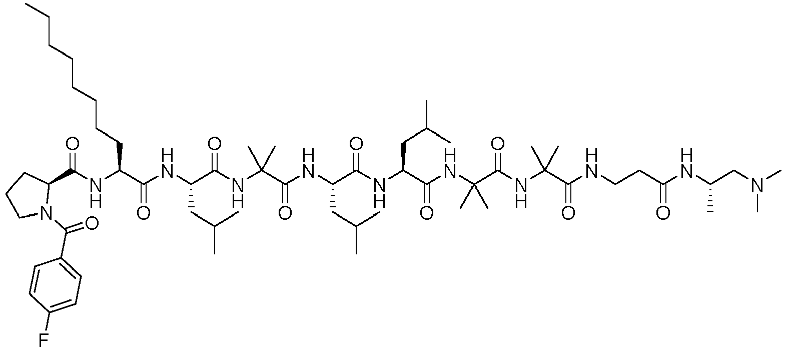

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(4-fluorobenzoyl)pyrrolidin-2-yl]formamido}decanamide.

| Cpd | 1 | 2 | 3 | 4 | 5 |
|---|---|---|---|---|---|
| 5934 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | 4-Fluorobenzoic acid | (S)-N¹,N¹-dimethylpropan e-1,2-diamine |

| **Cpd** | **Molecular mass** | **Experimentally measured mass** |
|---|---|---|
| 5934 | 1155.78 | 1156,51 |

| **Cpd** | **R1** | **R2** | **R3** | **R4** |
|---|---|---|---|---|
| 5934 | 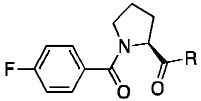 | 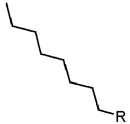 | 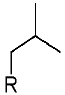 | 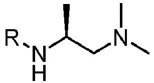 |

**5936:**

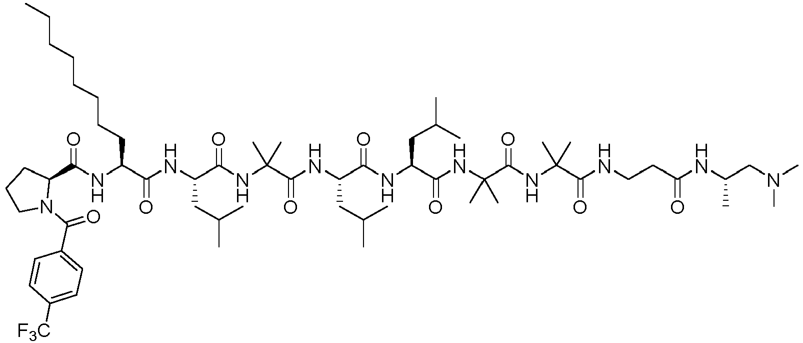

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-[4-(trifluoromethyl)benzoyl]pyrrolidin-2-yl]formamido}decanamide.

| Cpd | 1 | 2 | 3 | 4 | 5 |
|---|---|---|---|---|---|
| 5936 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | 4-(trifluorometh yl)benzoic acid | (S)-N¹,N¹-dimethylpropan e-1,2-diamine |

| **Cpd** | **Molecular mass** | **Experimentally measured mass** |
|---|---|---|
| 5936 | 1205.78 | 1206,49 |

| **Cpd** | **R1** | **R2** | **R3** | **R4** |
|---|---|---|---|---|
| 5936 | 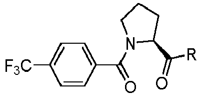 | 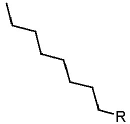 | 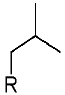 | 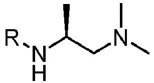 |

**5937:**

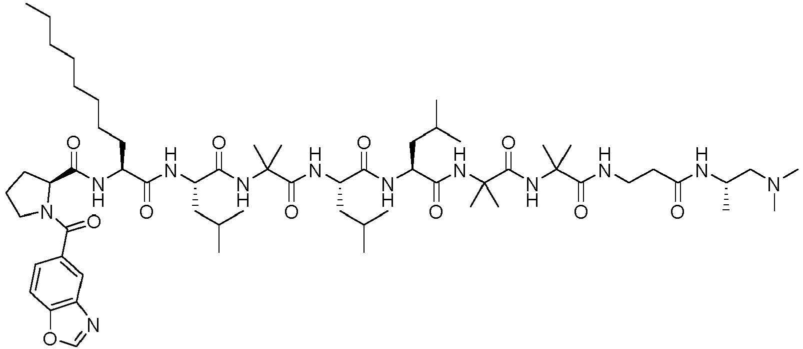

(2S)-2-{[(2S)-1-(1,3-benzoxazole-5-carbonyl)pyrrolidin-2-yl]formamido}-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]decanamide.

| Cpd | 1 | 2 | 3 | 4 | 5 |
|---|---|---|---|---|---|
| 5937 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-(S)-2-aminodecanoic acid | Fmoc- Pro-OH | Benzo[d]oxaz ole-5-carboxylic acid | (S)-N¹,N¹-dimethylpropan e-1,2-diamine |

| **Cpd** | **Molecular mass** | **Experimentally measured mass** |
|---|---|---|
| 5937 | 1178.78 | 1179,68 |

| **Cpd** | **R1** | **R2** | **R3** | **R4** |
|---|---|---|---|---|
| 5937 | 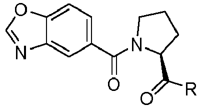 | 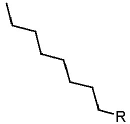 | 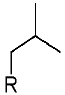 | 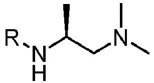 |

**5938:**

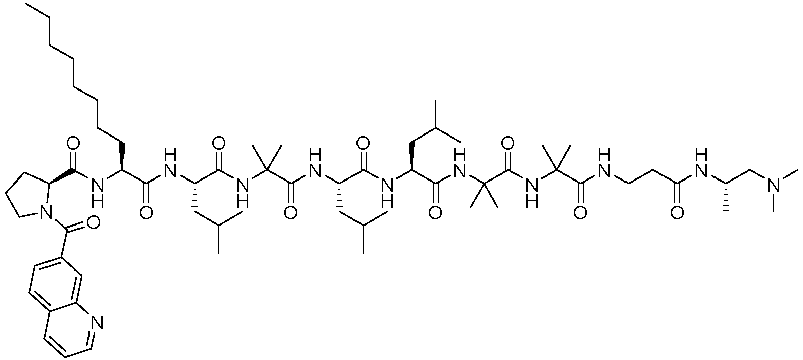

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3- methylbutyl]-2-{[(2S)-1-(quinoline-7-carbonyl)pyrrolidin-2-yl]formamido}decanamide.

| **Cpd** | **1** | **2** | **3** | **4** | **5** |
|---|---|---|---|---|---|
| **5938** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | Quinoline- 7-carboxylic acid | (S)-N¹,N¹-dimethylpropan e-1,2-diamine |

| **Cpd** | **Molecular mass** | **Experimentally measured mass** |
|---|---|---|
| **5938** | 1188.80 | 1189,55 |

| **Cpd** | **R1** | **R2** | **R3** | **R4** |
|---|---|---|---|---|
| **5938** | 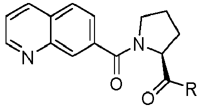 | 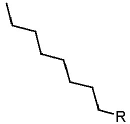 | 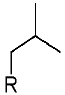 | 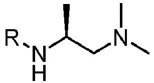 |

**5939:**

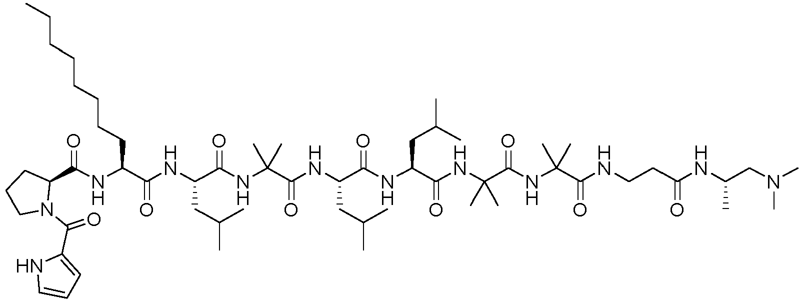

(2S)-N-[(1S)-1-[(1-{[(1S)-1-{[(1S)-1-{[1-({1-[(2-{[(2S)-1-(dimethylamino)propan-2-yl]carbamoyl}ethyl)carbamoyl]-1-methylethyl}carbamoyl)-1-methylethyl]carbamoyl}-3-methylbutyl]carbamoyl}-3-methylbutyl]carbamoyl}-1-methylethyl)carbamoyl]-3-methylbutyl]-2-{[(2S)-1-(1H-pyrrole-2-carbonyl)pyrrolidin-2-yl]formamido}decanamide.

| **Cpd** | **1** | **2** | **3** | **4** | **5** |
|---|---|---|---|---|---|
| **5939** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | 1H-pyrrole-2-carboxylic acid | (S)-N¹,N¹-dimethylpropan e-1,2-diamine |

| **Cpd** | **Molecular mass** | **Experimentally measured mass** |
|---|---|---|
| **5939** | 1126.78 | 1127,6 |

| **Cpd** | **R1** | **R2** | **R3** | **R4** |
|---|---|---|---|---|
| **5939** | 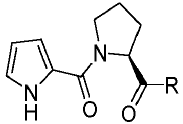 | 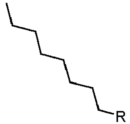 | 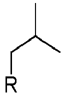 | 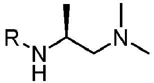 |

**6025:**

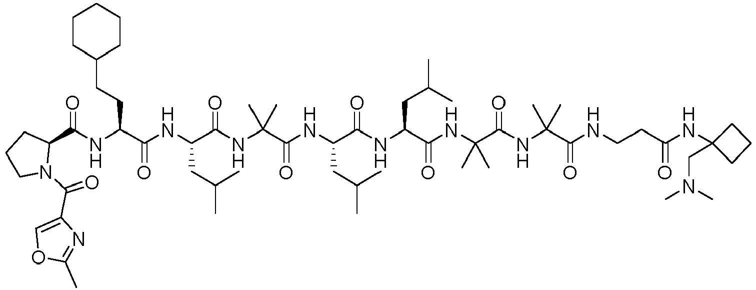

(2S)-2-[(2S)-4-cyclohexyl-2-{[(2S)-1-(2-methyl-1,3-oxazole-4-carbonyl)pyrrolidin-2-yl]formamido}butanamido]-N-(1-{[(1S)-1-{[(1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)-4-methylpentanamide.

| Cpd | 1 | 2 | 3 | 4 | 5 |
|---|---|---|---|---|---|
| 6025 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-L-homocyclohex ylalanine | Fmoc- Pro-OH | 2-methyloxazole -4-carboxylic acid | 1-((dimethylamino )methyl)cyclobu tan-1-amine |

| **Cpd** | **Molecular mass** | **Experimentally measured mass** |
|---|---|---|
| 6025 | 1166.78 | 1167.57 |

| **Cpd** | **R1** | **R2** | **R3** | **R4** |
|---|---|---|---|---|
| 6025 | 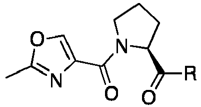 | 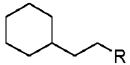 | 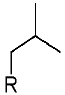 | 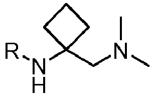 |

**6026:**

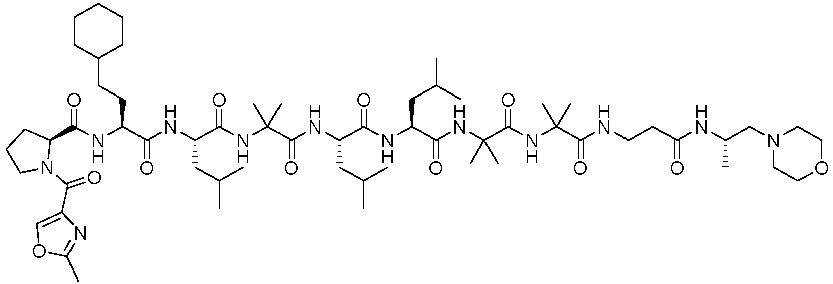

(2S)-2-[(2S)-2-{2-[(2S)-2-[(2S)-4-cyclohexyl-2-{[(2S)-1-(2-methyl-1,3-oxazole-4-carbonyl)pyrrolidin-2-yl]formamido}butanamido]-4-methylpentanamido]-2-methylpropanamido}-4-methylpentanamido]-4-methyl-N-[1-methyl-1-({1-methyl-1-[(2-{[(2S)-1-(morpholin-4-yl)propan-2-yl]carbamoyl}ethyl)carbamoyl]ethyl}carbamoyl)ethyl]pentanamide.

| Cpd | 1 | 2 | 3 | 4 | 5 |
|---|---|---|---|---|---|
| 6026 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-L-homocyclohex ylalanine | Fmoc- Pro-OH | 2-methyloxazole -4-carboxylic acid | (S)-1-morpholinoprop an-2-amine |

| **Cpd** | **Molecular mass** | **Experimentally measured mass** |
|---|---|---|
| 6026 | 1182.77 | 1183.60 |

| **Cpd** | **R1** | **R2** | **R3** | **R4** |
|---|---|---|---|---|
| 6026 | 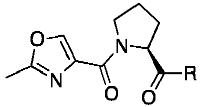 | 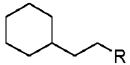 | 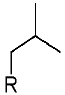 | 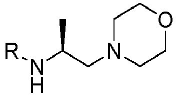 |

**6027:**

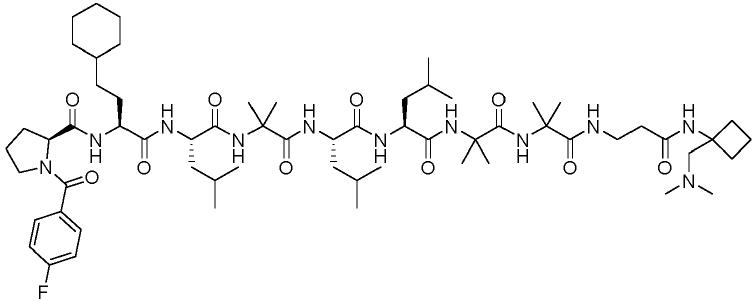

(2S)-2-[(2S)-4-cyclohexyl-2-{[(2S)-1-(4-fluorobenzoyl)pyrrolidin-2-yl]formamido}butanamido]-N-(1-{[(1S)-1-{[(1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)-4-methylpentanamide.

| Cpd | 1 | 2 | 3 | 4 | 5 |
|---|---|---|---|---|---|
| 6027 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-L-homocyclohex ylalanine | Fmoc- Pro-OH | 4-Fluorobenzoic acid | 1-((dimethylamino )methyl)cyclobu tan-1-amine |

| **Cpd** | **Molecular mass** | **Experimentally measured mass** |
|---|---|---|
| 6027 | 1179.78 | 1181.22 |

| **Cpd** | **R1** | **R2** | **R3** | **R4** |
|---|---|---|---|---|
| 6027 | 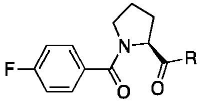 | 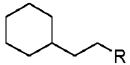 | 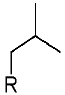 | 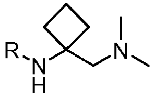 |

**6028:**

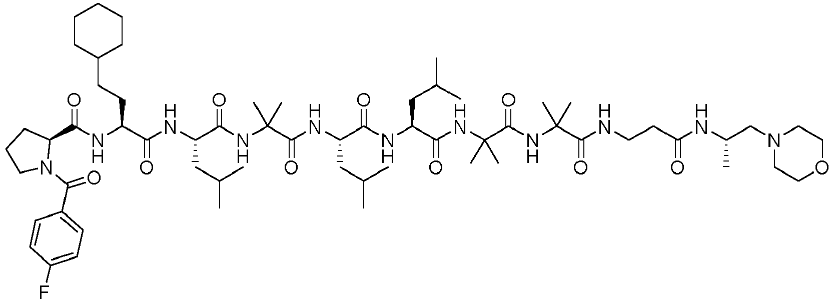

(2S)-2-[(2S)-2-{2-[(2S)-2-[(2S)-4-cyclohexyl-2-{[(2S)-1-(4-fluorobenzoyl)pyrrolidin-2-yl]formamido}butanamido]-4-methylpentanamido]-2-methylpropanamido}-4-methylpentanamido]-4-methyl-N-[1-methyl-1-({1-methyl-1-[(2-{S)-1-(morpholin-4-yl)propan-2-yl]carbamoyl}ethyl)carbamoyl]ethyl}carbamoyl)ethyl]pentanamide.

| Cpd | 1 | 2 | 3 | 4 | 5 |
|---|---|---|---|---|---|
| 6028 | Fmoc-Aib-OH | Fmoc-Aib-OH | Fmoc-Leu-OH | Fmoc-Leu-OH | Fmoc-Aib-OH |

| | 6 | 7 | 8 | 9 | 10 |
|---|---|---|---|---|---|
| | Fmoc-Leu-OH | Fmoc-L-homocyclohex ylalanine | Fmoc- Pro-OH | 4-Fluorobenzoic acid | (S)-1-morpholinoprop an-2-amine |

| **Cpd** | **Molecular mass** | **Experimentally measured mass** |
|---|---|---|
| 6028 | 1195.77 | 1196.34 |

| **Cpd** | **R1** | **R2** | **R3** | **R4** |
|---|---|---|---|---|
| 6028 | 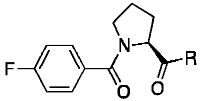 | 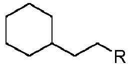 | 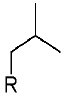 | 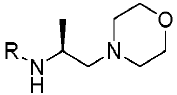 |

**6253:**

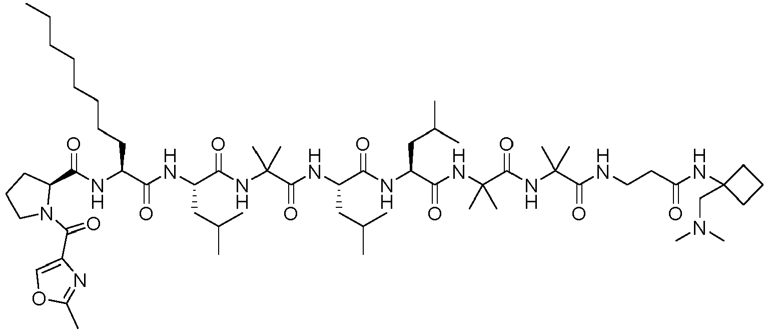

(2S)-N-[(1S)-1-[(1-{[(1S)-1-{[(1S)-1-({1-[(1-{[2-({1-[(dimethylamino)methyl]cyclobutyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)carbamoyl]-3-methylbutyl]-2-{[(2S)-1-(2-methyl-1,3-oxazole-4-carbonyl)pyrrolidin-2-yl]formamido}decanamide.

| **Cpd** | **1** | **2** | **3** | **4** | **5** |
|---|---|---|---|---|---|
| **6253** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | 2-methyloxazole -4-carboxylic acid | 1-((dimethylamino )methyl)cyclobu tan-1-amine |

| **Cpd** | **Molecular mass** | **Experimentally measured mass** |
|---|---|---|
| **6253** | 1168.79 | 1169.39 |

| **Cpd** | **R1** | **R2** | **R3** | **R4** |
|---|---|---|---|---|
| **6253** | 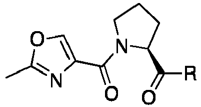 | 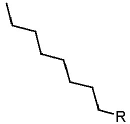 | 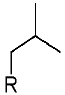 | 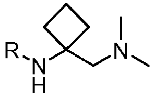 |

**6254:**

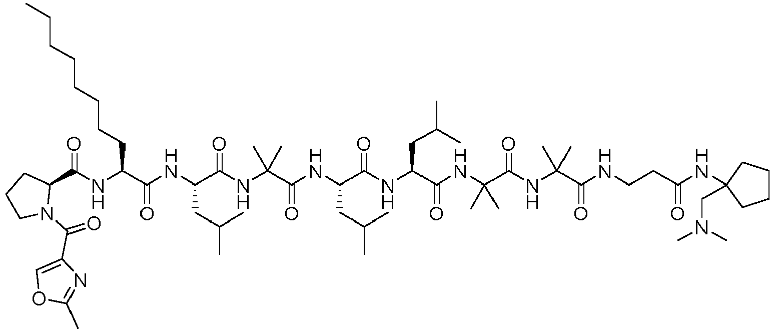

(2S)-N-[(1S)-1-[(1-{[(1S)-1-{[(1S)-1-({1-[(1-{[2-({1-[(dimethylamino)methyl]cyclopentyl}carbamoyl)ethyl]carbamoyl}-1-methylethyl)carbamoyl]-1-methylethyl}carbamoyl)-3-methylbutyl]carbamoyl}-3-methylbutyl]carbamoyl}-1-methylethyl)carbamoyl]-3-methylbutyl]-2-{[(2S)-1-(2-methyl-1,3-oxazole-4-carbonyl)pyrrolidin-2-yl]formamido}decanamide.

| **Cpd** | **1** | **2** | **3** | **4** | **5** |
|---|---|---|---|---|---|
| **6254** | Fmoc-Aib-OH | Fmoc-Aib-OH | Fmoc-Leu-OH | Fmoc-Leu-OH | Fmoc-Aib-OH |

| | **6** | **7** | **8** | **9** | **10** |
|---|---|---|---|---|---|
| | Fmoc-Leu-OH | Fmoc-(S)-2-aminodecanoic acid | Fmoc- Pro-OH | 2-methyloxazole -4-carboxylic acid | 1-((dimethylamino )methyl)cyclope ntan-1-amine |

| **Cpd** | **Molecular mass** | **Experimentally measured mass** |
|---|---|---|
| **6254** | 1182.81 | 1184.02 |

| **Cpd** | **R1** | **R2** | **R3** | **R4** |
|---|---|---|---|---|
| **6254** | 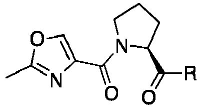 | 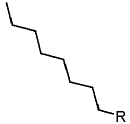 | 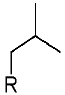 | 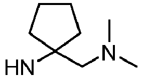 |

### EXAMPLE 3

### In vitro antiprotozoal assays and cytotoxic assays

Testing of compounds against *Plasmodium falciparum* (K1 strain), *Trypanosoma brucei rhodesiense* (STIB 900 strain), *Trypanosoma cruzi* trypomastigote forms (Tulahuen strain), *and Leishmania donovani* (strain MHOM/ET/67/L82) was performed in 96 well plates at concentrations in serial dilutions. Tests were done in triplicate. IC₅₀ values against the parasites as well as cytotoxic effects against rat myoblast (L6-cells) were determined by serial dilution and repeated twice.

The cytotoxicity assay was performed by a similar protocol as the Alamar Blue assay whereby L6-cells were seeded in 100 µl RPMI 1640 supplemented in 96-well micro titer plates (4000 cells/well). Podophyllotoxin (Sigma-Aldrich, Switzerland) was used as the reference drug (IC₅₀= 0.05 ± 0.01 µM). Its purity according to the supplier was more than 95%. After 68 h of incubation under humidified 5% CO₂ atmosphere, 10 µl of the Alamar blue marker was added to all wells. The plates were incubated for an additional 2 h. A Spectramax Gemini XS micro plate fluorescence reader (Molecular Devices Cooperation, Sunnyvale, CA) was used to measure the plates (Molecular Devices) using an excitation wavelength of 536 nm and an emission wavelength of 588 nm. The IC₅₀s were calculated by Softmax Pro software (Molecular Devices Cooperation, Sunnyvale, CA).

**Table 4: In vitro inhibition of protozoan parasites in nM, parasites are: Trypanosoma brucei rhodesiense (T.b.rhod.), intracellular Trypanosoma cruzi (T.cruzi.), axenic Leishmania donovani (L.don. ax), intracellular Leishmania donovani (L.don. InMac), and Plasmodium falciparum (P.falc NF5). Cytotoxicity was measured against rat myoblast cells (Cytotox L6).**

| **Cpd** | **T.b.rhod.** | **T.cruzi.** | **L.don. ax** | **L.don. InMac** | **P.falc NF5** | **Cytotox L6** |
|---|---|---|---|---|---|---|
| **Positive control** | 7,0 ^{a} | 2081 ^{b} | 118 ^{c} | 1196 ^{c} | 6,25 ^{d} | 19,3 ^{e} |
| **5649** | 0,7 | 8,8 | 3,5 | 27,2 | 2,1 | 274,0 |
| **5768** | 8,7 | 373,0 | 33,5 | 257,3 | 24,5 | 627,4 |
| **5769** | 2,8 | 167,4 | 8,1 | 215,9 | 5,3 | 422,9 |
| **5770** | 0,6 | 101,1 | 1,0 | 338,4 | 3,3 | 185,1 |
| **5904** | 0,2 | 36312,0 | 42524,0 | 42471,0 | 32143,0 | 215,8 |
| **5905** | 0,2 | 42514,0 | 31959,0 | 42382,0 | 26390,0 | 851,8 |
| **5910** | 24838,0 | 41,1 | 42645,0 | 42557,0 | 42645,0 | 230,2 |
| **5911** | 25569,0 | 34,0 | 14,4 | 34,8 | 42471,0 | 456,9 |
| **5912** | 0,3 | 17807,0 | 41395,0 | 42594,0 | 20852,0 | 253,7 |
| **5934** | 0,2 | 42496,0 | 27851,0 | <34,6 | 21582,0 | 306,1 |
| **5936** | 0,7 | 52,6 | 11749,0 | <33,2 | 31260,0 | 410,7 |
| **5937** | 43466,0 | 45,4 | 21,2 | 58,5 | 27638,0 | 1161,5 |
| **5938** | 0,5 | 42425,0 | 42442,0 | 37,8 | 13210,0 | 713,7 |
| **5939** | 0,1 | 42531,0 | 35977,0 | 209,3 | 36220,0 | 611,1 |
| **6025** | 0,5 | 214,6 | 31291,0 | 41,1 | 20941,0 | 1910,0 |
| **6026** | 41395,0 | 422,0 | 44652,0 | 50,7 | 38,0 | 1584,3 |
| **6027** | 0,4 | 155,0 | 34090,0 | 54,2 | 42498,0 | 1389,2 |
| **6028** | 41944,0 | 230,2 | 12479,0 | 417,9 | 26,3 | 1592,1 |
| **6253** | <0,9 | 68,4 | 46844,0 | | | 944,8 |
| **6254** | <0,8 | 34,6 | 13940,0 | | | 527,6 |

The positive controls were ^{a} Melarsoprol, ^{b} Benznidazol, ^{c} Miltefosine, ^{d} Chloroquine, ^{e} Podophyllotoxin.

### EXAMPLE 4

### Activity against Leishmania donovani parasites

Axenically grown *Leishmania donovani* amastigotes (strain MHOM/ET/67/L82) were grown and tests were done as previously described using the resazurin assay (Räz B et al. Acta Tropica 1997, 68:139-147). The plates were developed for 2-4 hours and read on a Spectramax Gemini XS micro plate fluorometer (Molecular Devices Cooperation) using an excitation wavelength of 536 nm and emission wavelength of 588 nm. Fluorescence development was measured and expressed as percentage of the negative control. Miltfosine (VWR, Paisley, Scotland) was used as a reference drug (IC₅₀= 0.241 µg/mL). Its purity according to the supplier was more than 95%. Data were transferred to the graphic programme Softmax Pro (Molecular Devices), with which IC₅₀ values were calculated.

Table 4 above shows (i) *in vitro* inhibition of axenic *Leishmania donovani* (L.don. ax) and intracellular *Leishmania donovani* (L.don. InMac) parasites in nM.

### EXAMPLE 5

### Activity against Trypanosoma cruzi parasites

*Trypanosoma cruzi* trypomastigote forms (Tulahuen strain C2C4 containing β-galactosidase (Lac Z) gene) were cultured as described by Buckner et al. (Buckner FS et al. Antimicrob Agents Ch1996; 40: 2592-2597) in rat myoblast cells (L6-cells). Benznidazole (Sigma-Aldrich, Switzerland) was used as a standard drug (IC₅₀= 0.48 µg/mL). Its purity according to the supplier was more than 95%. After incubation, the substrate chlorophenyl red β-D-galactopyranoside agent CPRG/Nonident was added to all wells and a color change was developed within 2 to 6 h. The plates were read photometrically at 540 nm (Molecular Devices). Data were evaluated and IC₅₀ values calculated using Softmax Pro software (Molecular Devices).

Table 4 above shows (i) *in vitro* inhibition of intracellular *Trypanosoma cruzi* (T.cruzi).

### EXAMPLE 6

### Activity against Trypanosoma brucei rhodesiense parasites

*Trypanosoma brucei rhodesiense* (STIB 900) were grown in axenic medium as described by Baltz et al. (1985) The samples were tested using the Alamar Blue assay protocol (Baltz T et al. EMBO J 1985; 4:1273-1277) to determine the 50% inhibitory concentration (IC₅₀). A Spectramax Gemini XS micro plate fluorescence reader (Molecular Devices Cooperation, Sunnyvale, CA) with excitation wavelength 536 nm and an emission wavelength 588 nm was used to measure the plates. Melarsoprol (Arsobal®, Sanofi-Aventis, Switzerland) was used as a reference drug (IC₅₀= 0.03 ± 0.01 µM). Its purity was over 95 %, as was certified by the supplier. The IC₅₀ values were calculated by using Softmax Pro software (Molecular Devices Cooperation, Sunnyvale, CA).

Table 4 above shows (i) *in vitro* inhibition of *Trypanosoma brucei rhodesiense* (T.b.rhod.).

### EXAMPLE 7

### Activity against Plasmodium falciparum parasites

Determination of activity against *Plasmodium falciparum* K1 strain was done using a modified version of the ³H-hypoxanthine incorporation assay by Trager and Jensen (Trager W. and Jensen, J.B. Science 1976; 193:673-675). After incubation the plates were harvested using a Betaplate cell harvester (Wallac, Switzerland) onto glass-fiber filters and washed. The dried filters were inserted into plastic foils with 10 ml scintillation fluid. The radioactivity was counted with a Betaplate liquid scintillation counter (Wallac, Switzerland) as counts per minute per well at each drug concentration and compared to the untreated controls. Chloroquine (Sigma-Aldrich, Switzerland) was used as a positive control (IC₅₀= 0.05 ± 0.01 µM). Its purity according to the supplier was more than 95%. IC₅₀ values were calculated by linear interpolation. All assays were run in duplicate and repeated two times.

Table 4 above shows (i) *in vitro* inhibition of *Plasmodium falciparum* (P.falc NF5).

### EXAMPLE 8

### In vivo testing in acute Trypanosoma brucei brucei model in mice

### Acute Mouse Sleeping Sickness Model

This model mimics the first stage of the human African trypanosomiasis. Adult female NMRI mice were purchased from Janvier (St. Berthevin, France). They weighed between 20 and 25 g at the beginning of the study and were kept under standard conditions at 22 °C and 60-70% humidity in macrolon type III cages with food pellets and water *ad libitum.* The samples were first dissolved in 100% DMSO, followed by addition of distilled H₂O to a final DMSO concentration of 10%. For determination of the *in vivo* antitrypanosomal activity, mice were infected intraperitoneally with 1 × 10⁴ STIB900 bloodstream forms. Experimental groups of four mice were treated intraperitoneally or orally once or twice a day on four consecutive days from day 3 to day 6 post infection. A control group of four mice was infected, but remained untreated. The determination of the parasitaemia was done on days 7, 10, 14, 17, 21, 24, 28, and 31 post infection. To do this 6 µL of tail blood were diluted in 24 µL sodium citrate (3.2%), whereby the first µL was discarded to obtain circulating blood. Five µL of this mixture were transferred to a glass slide and covered with an 18 × 18 mm cover slide. The sample was examined under a light microscope (200-fold magnification) and parasites were counted in 3 of the 16 squares of the grid.

Table 5: *In vivo* effects of compounds 6027 and 6025 (column 1), the treatment duration in days (column 2), the amount in each dose (column 3), number of doses per day (column 4), route of application being intraperitonieally (i.p.) or oral (po)(column 5). Each of the four test animal per cage (column 7) was monitored for its parasitaemia according to cell counts in a grid as described in the legend (columns 8-15). Whether or not animals survived is shown (grey= dead) for the corresponding days in the test duration (columns 8 -15), and days of survival are shown (column 8). If parasitaemia relapsed after being previously surpressed under the level of detection (indicated in columns 8-15 as 0), then the day of relapse is shown (column 9). Finally, the mean duration of survival is shown (column 10).

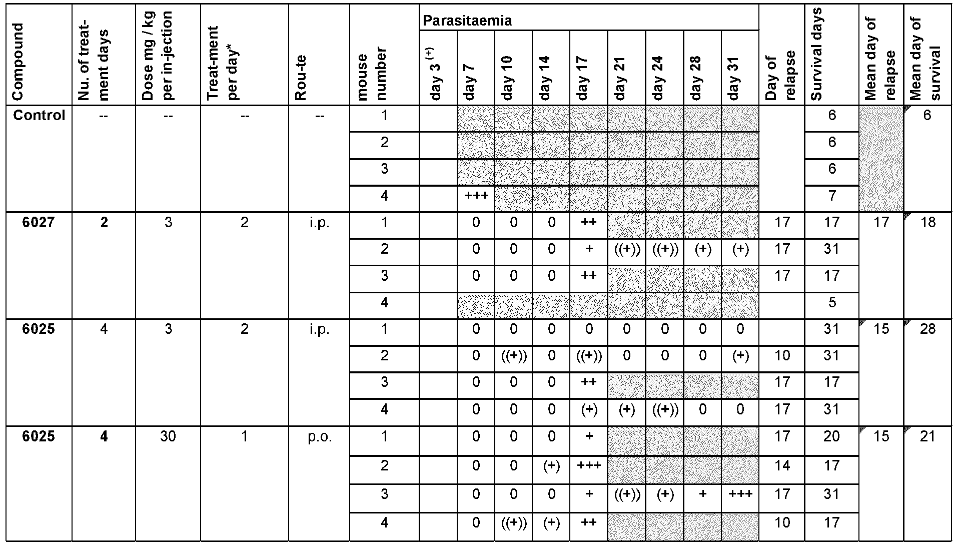

0 = no parasite in 20 fields of view; ((+)) = 1 Trypanosome in 20 fields of view; (+) = 1 Trypanosome per field of view; + = 2 - 5 Trypanosomes per field of view; ++ = 6 - 20 Trypanosomes per field of view, the mouse is killed; +++ = > 20 Trypanosomes per field of view, the mouse is killed.

Results: Compounds 6025 and 6027 were tested *in vivo* against at 3 mg/kg twice a day for 4 (6025) and 2 (6027) days. Compound 6025 was further tested orally at 30 mg/kg once a day. All three tests showed significant reduction of parasitaemia compared to untreated controls. On day seven three out of four mice in the control group had died, and the remaining one had to be sacrificed that day due to very high parasitaemia. In compound treated groups all animals survived treatment, except for one in the group with compound 6027 which died on day 5 for unspecified reasons.

Compound treated groups showed strong antiprotozoal effects, as the animals displayed no observable parasitaemia on day 7. However, none of the groups showed a permanent cure of all animals under these test conditions, which became clear when experiments were followed up until day 31. In the case of compound 6027 all surviving animals showed parasitaemia by day 17, which lead to two animals being sacrificed, and one surviving until day 31 with low parasitaemia. In the case of the group treated intraperitoneally with compound 6025, three out of four animals survived until day 31 at which point 2 of them had no observable parasitaemia. The group treated orally with compound 6025 all had relapsed by day 17 and one survived until day 31.

In summary compounds 6025 and 6027, alongside many other compounds of the present invention had shown strong inhibitory effects against a several protozoan parasites *in vitro.* Therefore, their antiprotozoal effects were also tested *in vivo* using intraperitonially and oral application. The antiprotozoal assays used was a mouse model using *Trypanosoma brucei,* which is a model of acute sleeping sickness. Compounds showed strong inhibition of protozoan parasites after intraperitoneal application (6025 and 6027) and oral application (6025). This is strong evidence of their usefulness in development as drug leads for protozoan diseases.

### EXAMPLE 9

### In vivo testing in acute Leishmania donovani model in mice

Female BALB/c mice are infected, via the tail vein, with 2×10⁷ freshly harvested *L. donovani* amastigotes. After 7 days, patency of infection is checked and dosing commenced. Groups of five mice receive inventive compounds (1.5, 3, 6, 25 mg/kg) i.p.x5days or o.p.x5days; Pentostam (45, 15 and 5 mgSbV/kg) s.c×5 days; Fungizone (1, 0.2, 0.04 mg/kg) i.v.×1 day; AmBisome (5, 1, 0.2 mg/kg) i.v.×1 day. Liver impression smears are made 14 days post infection and activity determined by counting, microscopically, the number of amastigotes per liver cell in treated and untreated mice. ED50 values are calculated by sigmoidal regression analysis (Yardley V. and Croft SL. International Journal of Antimicrobial Agents 13 (2000) 243―248).

## Claims

1. A compound of formula (I) 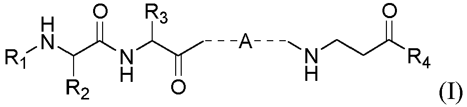 wherein said compound is a compound of any one of the formula (II) to (IV) 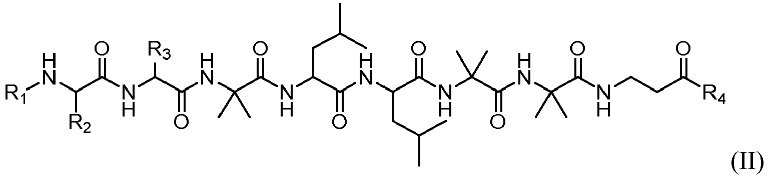 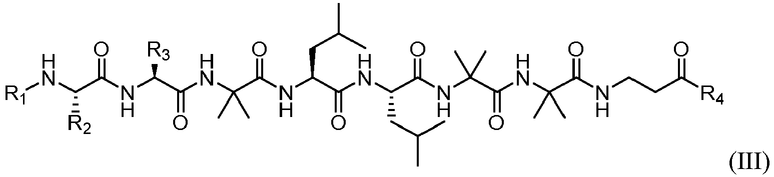 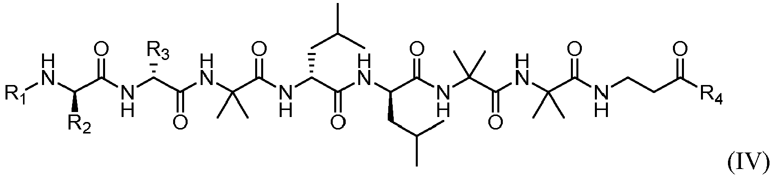 and wherein
R₁ is 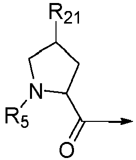 preferably 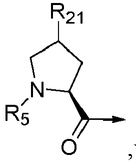 wherein
R₅ is C(O)-R₈, wherein
R₈ is independently at each occurrence selected from phenyl, naphthalene, 1,2-dihydronapthalenyl, 1,2,3,4-tetrahydronaphthenyl, pyridinyl, pyrazolyl, pyrazinyl, furyl, thienyl, isoxazolyl, oxazolyl, pyrrolyl, quinolinyl, isoquinolinyl, indolyl, piperidinyl, morpholinyl, pyrrolidinyl, benzimidazolyl, benzofuranyl, benzopyranyl, coumarinyl, pyridazinyl, isoindolyl, benzothiophenyl, benzooxazolyl, cyclopentyl, cyclopentenyl, cyclohexyl, cyclohexenyl, adamantanyl; each independently optionally substituted with C₁-C₄alkyl, halogen preferably fluorine, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl; and wherein
the arrow indicates the attachment to the NH-moiety depicted in formula (I);
R₂ is selected from 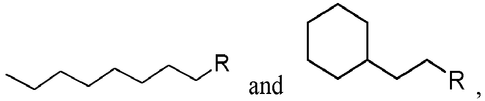 wherein R indicates the attachment to the CH-moiety depicted in formula (I);
R₃ is selected from 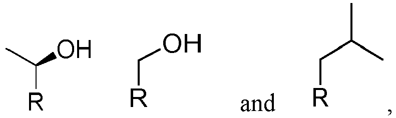 wherein R indicates the attachment to the CH-moiety depicted in formula (I)
R₄ is 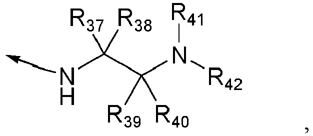 wherein
R₃₇, R₃₈, R₃₉ and R₄₀ are independently at each occurrence H or C₁-C₃alkyl, preferably H or methyl, or independently at each occurrence two of said R₃₇, R₃₈, R₃₉ and R₄₀ together with the carbon atom to which they are attached form a carbocyclic or heterocyclic ring, preferably a carbocyclic ring, and wherein
R₄₁ and R₄₂ are independently of each other H or C₁-C₄alkyl optionally substituted with halogen, hydroxyl or C₃-C₆cycloalkyl; or together with the nitrogen atom to which they are attached form independently at each occurrence a heteroaryl or a heterocyclyl, each independently optionally substituted with halogen, C₁-C₄alkyl, OR₄₃, NR₄₄R₄₅; wherein
the arrow indicates the attachment to the C(O)-moiety depicted in formula (I);
and pharmaceutically acceptable salts of said compound of formula (I).

2. The compound of claim 1, wherein said compound of formula (I) is a compound of formula (III).

3. The compound of claim 1 or claim 2, wherein said R₈ is phenyl independently optionally substituted with C₁-C₄alkyl, halogen, preferably fluorine, CF₃, OR₁₀, NR₁₁R₁₂; wherein R₁₀, R₁₁, R₁₂ are independently at each occurrence H, C₁-C₃alkyl; and wherein R₂₁ is selected from H, C₁-C₄alkyl.

4. The compound of any one of the claims 1 to 3, wherein said R₂₁ is H.

5. The compound of claim 1, wherein said R₁ is selected from 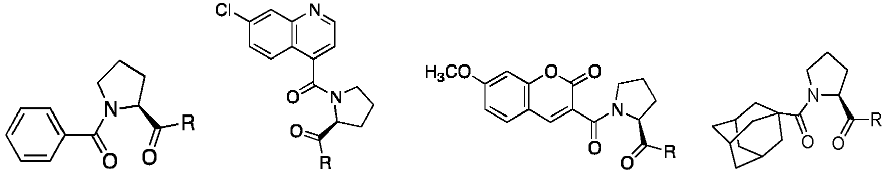 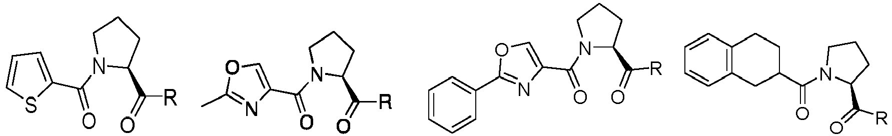 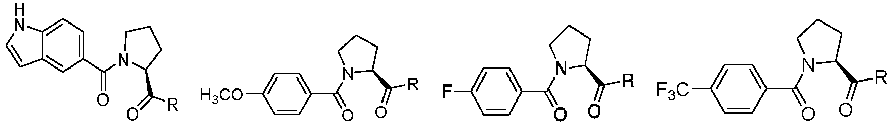 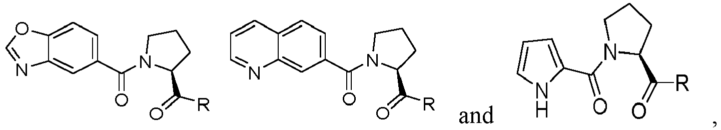 wherein R indicates the attachment to the NH-moiety depicted in formula (I).

6. The compound of any one of the claims 1 to 5, wherein said R₄ is 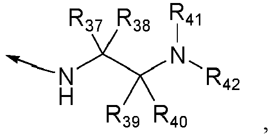 wherein
R₃₇, R₃₈, R₃₉ and R₄₀ are independently at each occurrence hydrogen or C₁-C₃alkyl, preferably hydrogen or methyl, or independently at each occurrence two of said R₃₇, R₃₈, R₃₉ and R₄₀ together with the carbon atom to which they are attached form a monocyclic carbocyclic or monocyclic heterocyclic ring, preferably a monocyclic carbocyclic ring, and wherein
R₄₁ and R₄₂ are independently of each other C₁-C₄alkyl, preferably methyl; or together with the nitrogen atom to which they are attached form independently at each occurrence a monocyclic heteroaryl or a monocyclic heterocyclyl, each independently optionally substituted with halogen, C₁-C₄alkyl, OR₄₃, NR₄₄R₄₅, wherein R₄₃, R₄₄, R₄₅ are independently at each occurrence H, C₁-C₄alkyl; wherein the arrow indicates the attachment to the C(O)-moiety depicted in formula (I).

7. The compound of any one of the claims 1 to 6, wherein said R₄ is selected from 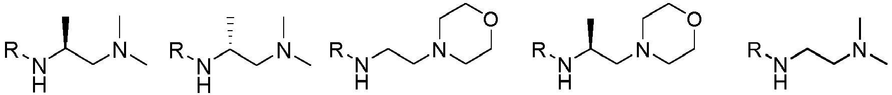 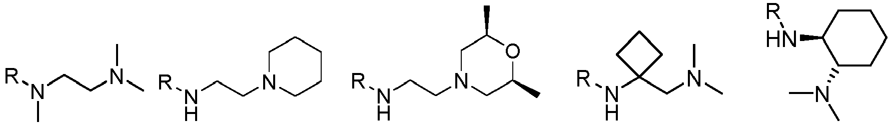 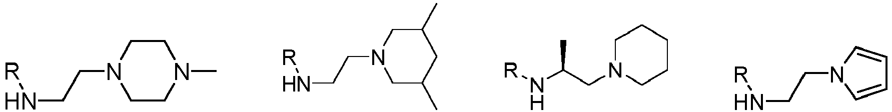 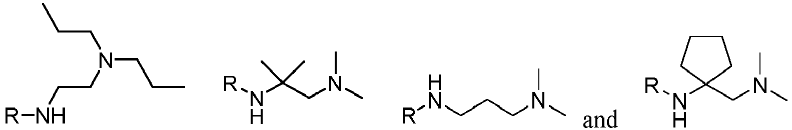 wherein R indicates the attachment to the C(O)-moiety depicted in formula (I).

8. The compound according to claim 1, wherein said compound is selected from
**5649:** 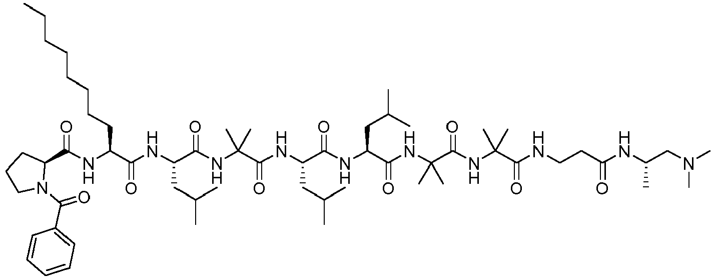
**5768:** 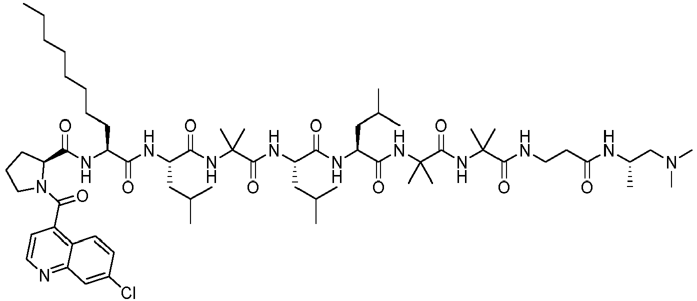
**5769:** 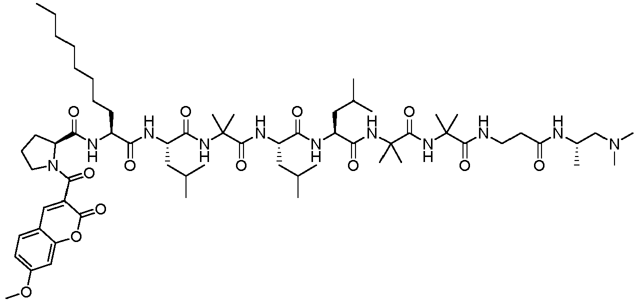
**5770:** 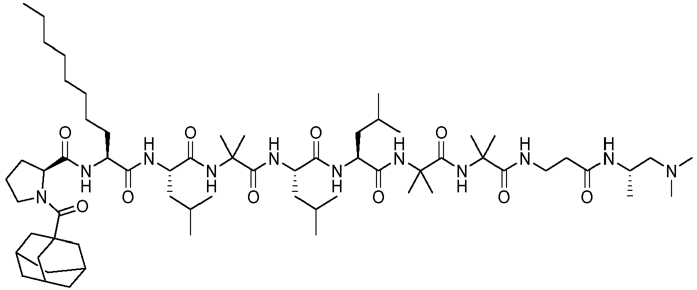
**5904:** 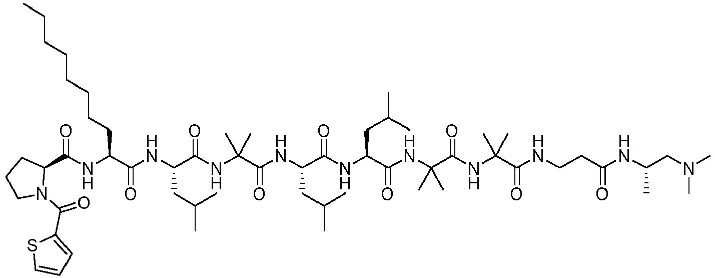
**5905:** 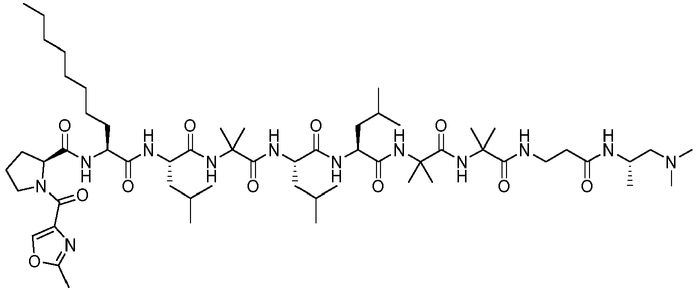
**5910:** 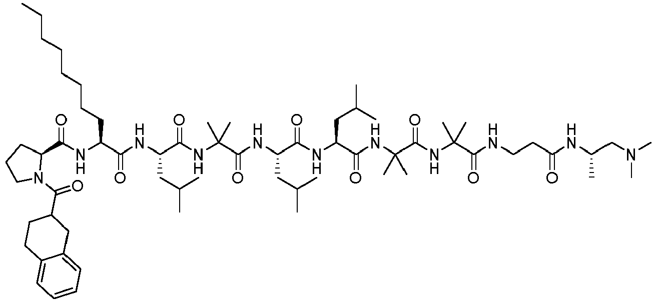
**5911:** 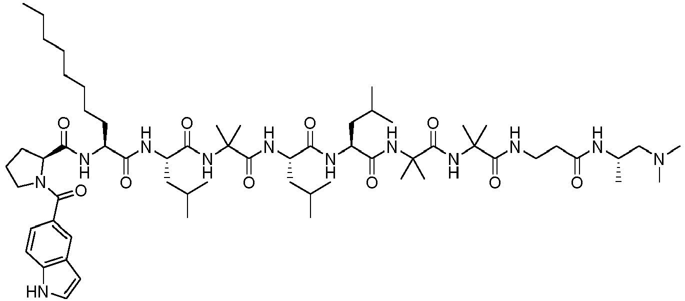
**5912:** 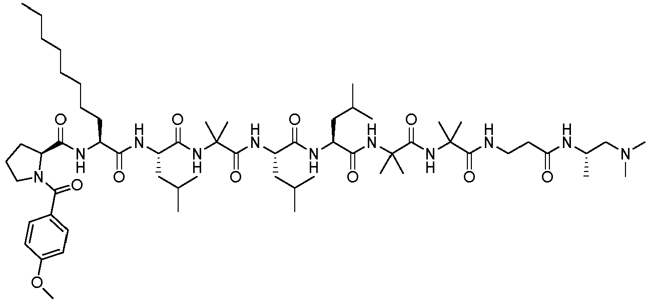
**5934:** 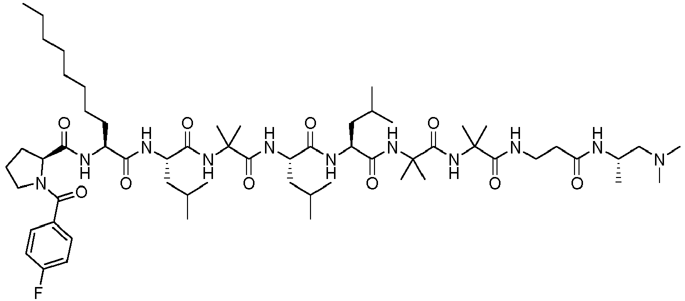
**5936:** 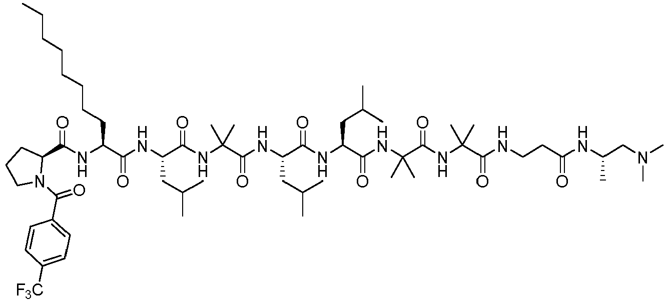
**5937:** 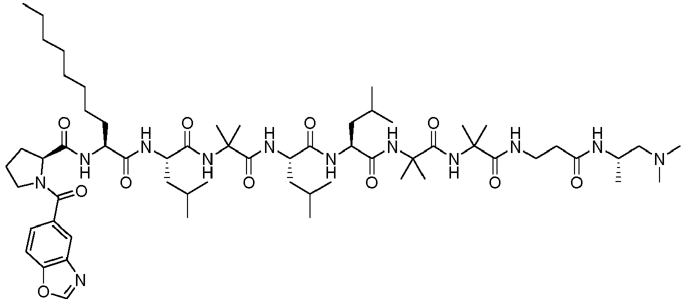
**5938:** 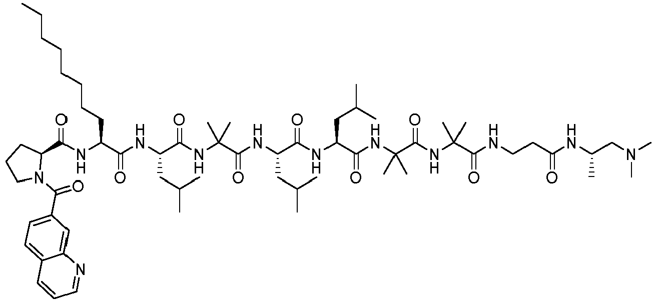
**5939:** 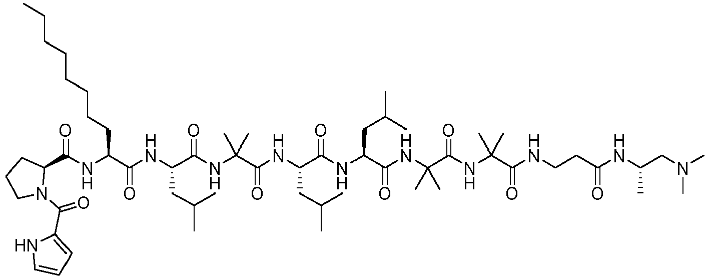
**6025:** 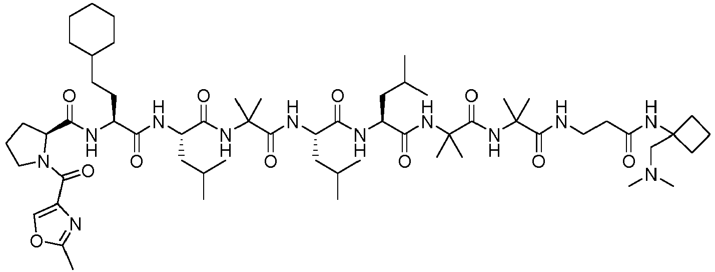
**6026:** 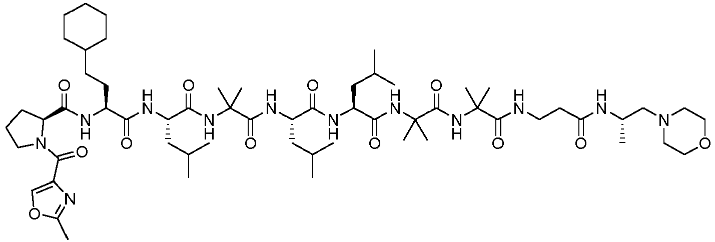
**6027:** 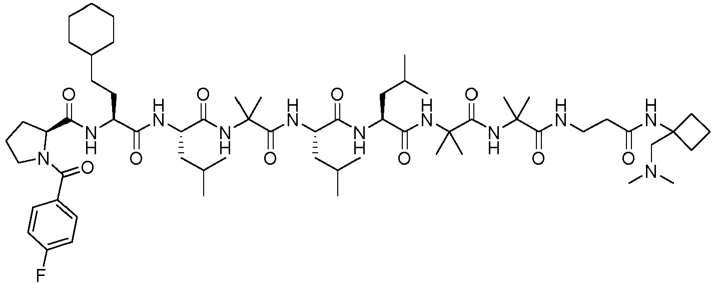
**6028:** 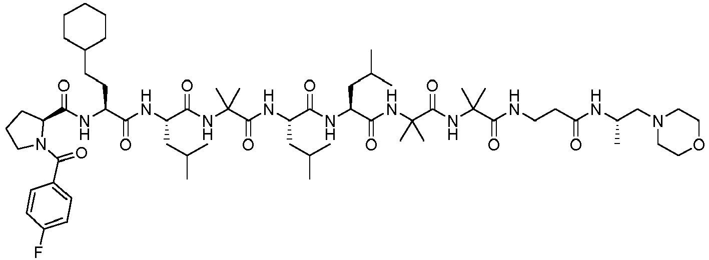
**6253:** 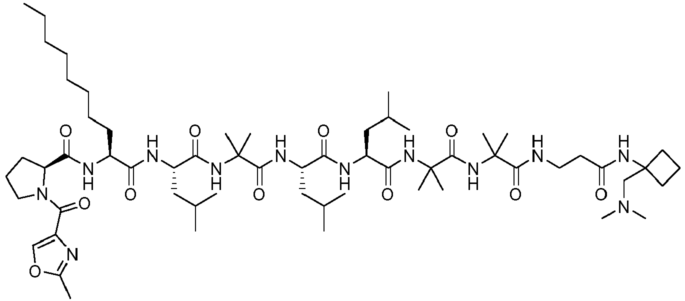
**6254:**

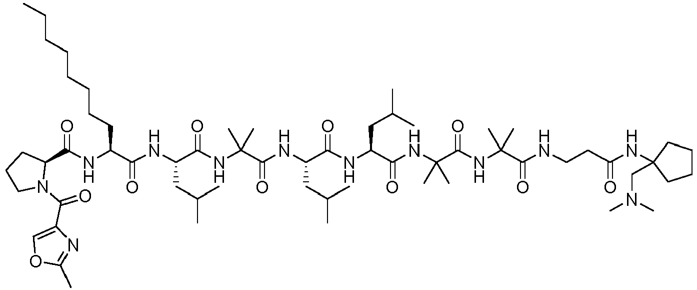

9. The compound of any one of the claims 1 to 8 for use in a method of treatment of a protozoan disease, wherein preferably said protozoan disease is selected from malaria, human African trypanosomiasis, Chagas disease and leishmaniasis, and wherein further preferably said disease is selected from acute malaria, cerebral malaria, human African trypanosomiasis, Chagas disease, cutaneous leishmaniasis (CL), mucocutaneous leishmaniasis (MCL) and visceral leishmaniasis (VL).

10. The compound for use of claim 9, wherein said protozoan disease is selected from Chagas disease or leishmaniasis, and wherein preferably said protozoan disease is selected from Chagas disease, cutaneous leishmaniasis (CL), mucocutaneous leishmaniasis (MCL) and visceral leishmaniasis (VL), and wherein further preferably said protozoan disease is selected from Chagas disease and visceral leishmaniasis (VL)

11. The compound of any one of the claims 1 to 8 for use in the treatment of a condition, disease or disorder caused by a parasite selected from a parasite of the *Plasmodium* genus, a parasite of the *Trypanosoma* genus or a parasite of the *Leishmania* genus, wherein preferably said parasite is selected from *Trypanosoma cruzi, Leishmania donovani, Plasmodium falciparum, Plasmodium vivax, Plasmodium ovale, Plasmodium malaria,* and wherein further preferably said parasite is selected from *Trypanosoma cruzi* and *Leishmania donovani.*

12. A pharmaceutical composition comprising a compound of any one of the claims 1 to 8 and a pharmaceutically acceptable carrier or adjuvant, wherein said pharmaceutical composition is preferably formulated for use in inhibiting proliferation of a *Leishmania* parasite in a patient for prophylaxis or to a patient in need of treatment for leishmaniasis.

## Patentansprüche

1. Verbindung von Formel (I) 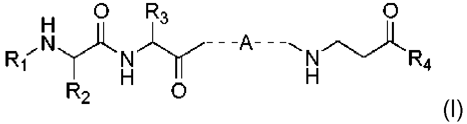 wobei die Verbindung eine Verbindung nach einer beliebigen der Formeln (II) bis (IV) ist 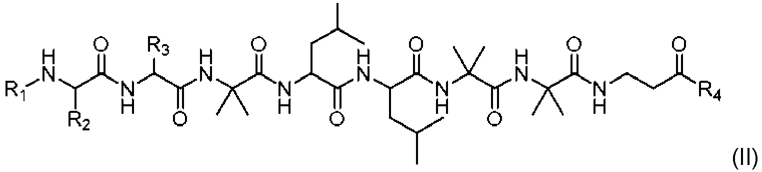 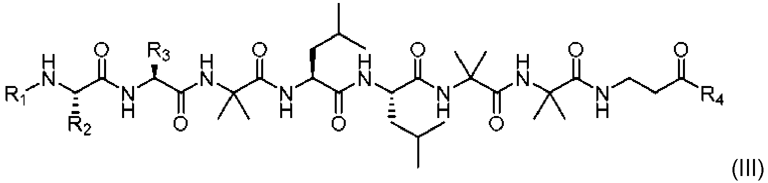 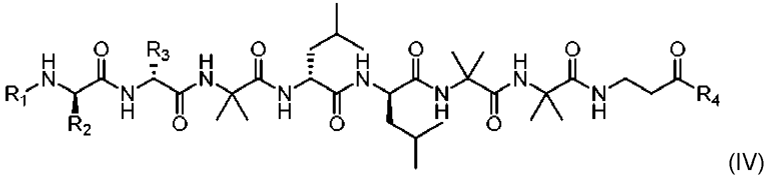 und wobei
R₁ Folgendes ist 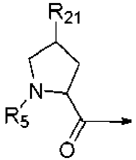 vorzugsweise 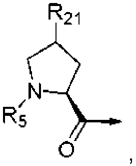 wobei R₅ C(O)-R₈ ist, wobei
R₈ bei jedem Auftreten unabhängig aus Phenyl, Naphthalin, 1,2-Dihydronapthalenyl, 1,2,3,4-Tetrahydronaphthenyl, Pyridinyl, Pyrazolyl, Pyrazinyl, Furyl, Thienyl, Isoxazolyl, Oxazolyl, Pyrrolyl, Chinolinyl, Isochinolinyl, Indolyl, Piperidinyl, Morpholinyl, Pyrrolidinyl, Benzimidazolyl, Benzofuranyl, Benzopyranyl, Cumarinyl, Pyridazinyl, Isoindolyl, Benzothiophenyl, Benzoxazolyl, Cyclopentyl, Cyclopentenyl, Cyclohexyl, Cyclohexenyl, Adamantyl ausgewählt ist; jeweils unabhängig optional substituiert mit C₁-C₄-Alkyl, Halogen, vorzugsweise Fluor, CF₃, OR₁₀, NR₁₁R₁₂; wobei R₁₀, R₁₁, R₁₂ bei jedem Auftreten unabhängig H, C₁-C₃-Alkyl sind; und wobei R₂₁ aus H, C₁-C₄-Alkyl ausgewählt ist; und wobei
der Pfeil die Bindung an das NH-Molekülteil, das in Formel (I) dargestellt ist, angibt;
R₂ aus Folgendem ausgewählt ist 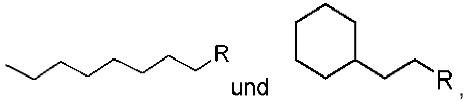 wobei
R die Bindung an das CH-Molekülteil, das in Formel (I) dargestellt ist, angibt;
R₃ aus Folgendem ausgewählt ist 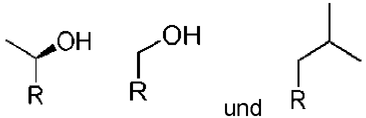
wobei R die Bindung an das CH-Molekülteil, das in Formel (I) dargestellt ist, angibt; R₄ Folgendes ist 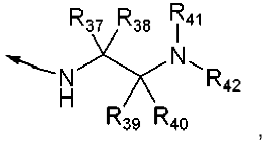 wobei
R₃₇, R₃₈, R₃₉ und R₄₀ unabhängig voneinander bei jedem Auftreten H- oder C₁-C₃-Alkyl sind, vorzugsweise H oder Methyl, oder unabhängig voneinander bei jedem Auftreten zwei der R₃₇, R₃₈, R₃₉ und R₄₀ zusammen mit dem Kohlenstoffatom, an das sie gebunden sind, einen carbocyclischen oder heterocyclischen Ring ausbilden, vorzugsweise einen carbocyclischen Ring, und wobei
R₄₁ und R₄₂ unabhängig voneinander H- oder C₁-C₄-Alkyl sind, optional substituiert mit Halogen, Hydroxyl oder C₃-C₆-Cycloalkyl; oder zusammen mit dem Stickstoffatom, an das sie gebunden sind, bei jedem Auftreten unabhängig voneinander ein Heteroaryl oder ein Heterocyclyl ausbilden, die jeweils unabhängig voneinander optional mit Halogen, C₁-C₄-Alkyl, OR₄₃, NR₄₄R₄₅ substituiert sind; wobei
der Pfeil die Bindung an das C(O)-Molekülteil, das in Formel (I) dargestellt ist, angibt; und pharmazeutisch unbedenkliche Salze der Verbindung von Formel (I).

2. Verbindung nach Anspruch 1, wobei die Verbindung eine Verbindung von Formel (III) ist.

3. Verbindung nach Anspruch 1 oder 2, wobei das R₈ Phenyl ist, unabhängig optional substituiert mit C₁-C₄-Alkyl,Halogen, vorzugsweise Fluor, CF₃, OR₁₀, NR₁₁R₁₂; wobei R₁₀, R₁₁, R₁₂ bei jedem Auftreten unabhängig H-, C₁-C₃-Alkyl sind; und wobei R₂₁ aus H, C₁-C₄-Alkyl ausgewählt ist.

4. Verbindung nach einem der Ansprüche 1 bis 3, wobei das R₂₁ H ist.

5. Verbindung nach Anspruch 1, wobei R₁ aus Folgendem ausgewählt ist 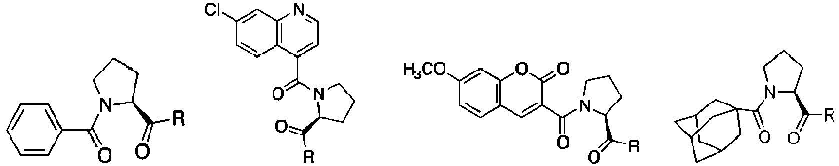 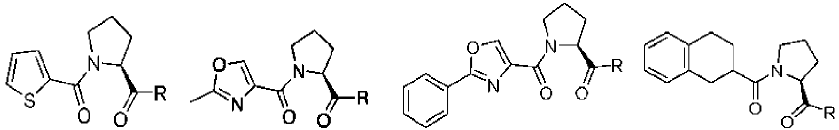 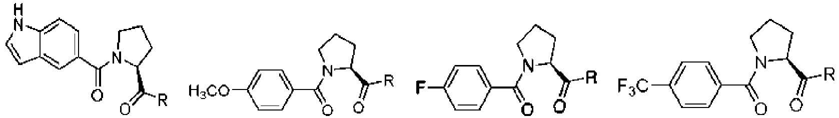 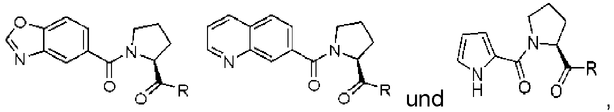 wobei R die Bindung an das NH-Molekülteil, das in Formel (I) dargestellt ist, angibt.

6. Verbindung nach einem der Ansprüche 1 bis 5, wobei das R₄ Folgendes ist 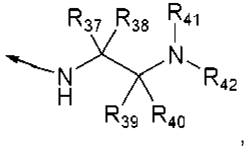 wobei R₃₇, R₃₈, R₃₉ und R₄₀ unabhängig voneinander bei jedem Auftreten Wasserstoff oder C₁-C₃-Alkyl sind, vorzugsweise Wasserstoff oder Methyl, oder unabhängig bei jedem Auftreten zwei der R₃₇, R₃₈, R₃₉ und R₄₀ zusammen mit dem Kohlenstoffatom, an das sie gebunden sind, einen monocyclischen carbocyclischen oder monocyclischen heterocyclischen Ring ausbilden, vorzugsweise einen monocyclischen carbocyclischen Ring, und wobei
R₄₁ und R₄₂ unabhängig voneinander C₁-C₄-Alkyl sind, vorzugsweise Methyl; oder zusammen mit dem Stickstoffatom, an das sie gebunden sind, unabhängig bei jedem Auftreten ein monocyclisches Heteroaryl oder ein monocyclisches Heterocyclyl ausbilden, die jeweils unabhängig optional mit Halogen, C₁-C₄-Alkyl,OR₄₃, NR₄₄R₄₅ substituiert sind, wobei R₄₃, R₄₄, R₄₅ bei jedem Auftreten unabhängig H-, C₁-C₄-Alkyl sind; wobei der Pfeil die Bindung an das C(O)-Molekülteil, das in Formel (I) dargestellt ist, angibt.

7. Verbindung nach einem der Ansprüche 1 bis 6, wobei das R₄ aus Folgendem ausgewählt ist 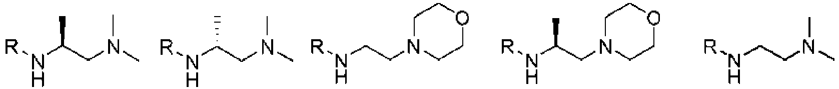 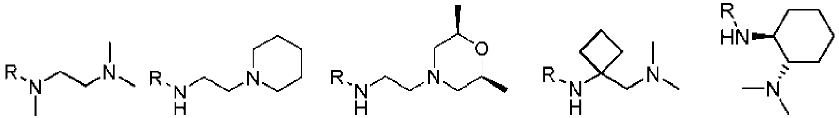 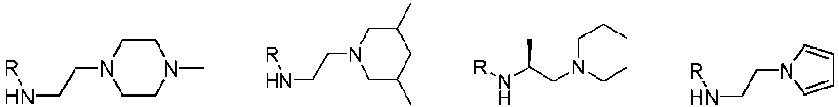 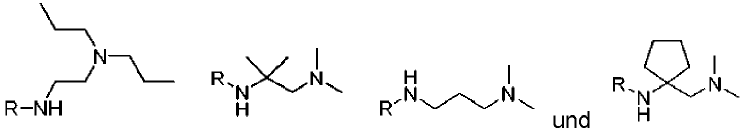 wobei R die Bindung an das C(O)-Molekülteil, das in Formel (I) dargestellt ist, angibt.

8. Verbindung nach Anspruch 1, wobei die Verbindung aus Folgendem ausgewählt ist 5649: 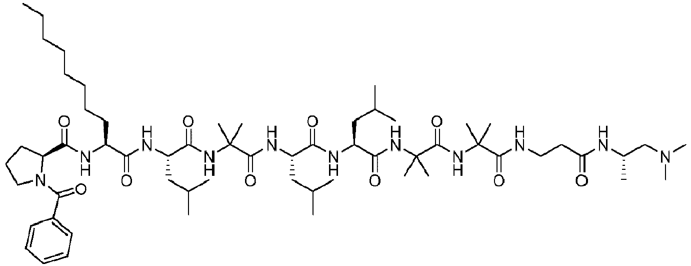
**5768:** 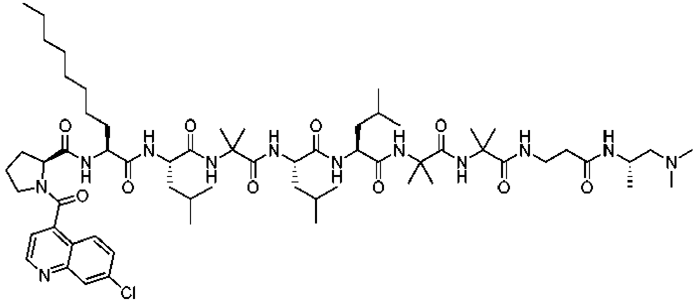
**5769:** 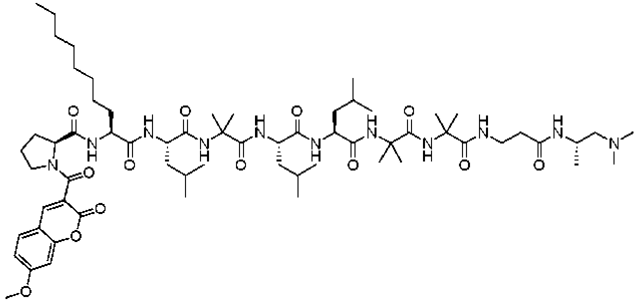
**5770:** 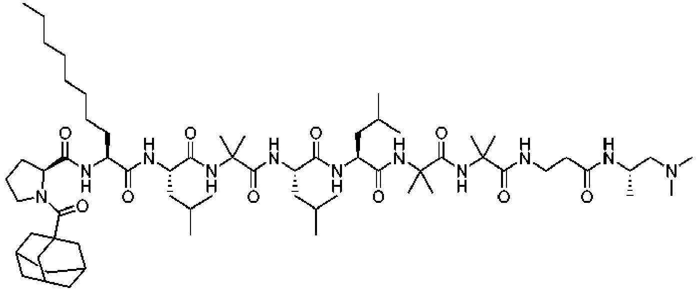
**5904:** 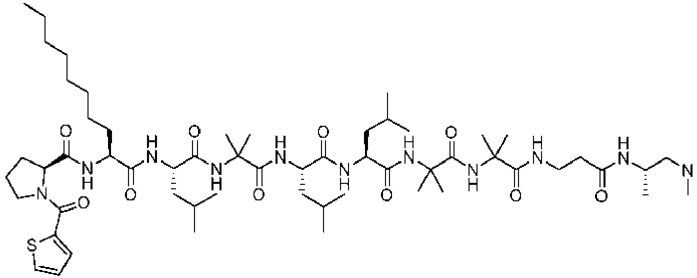
**5905:** 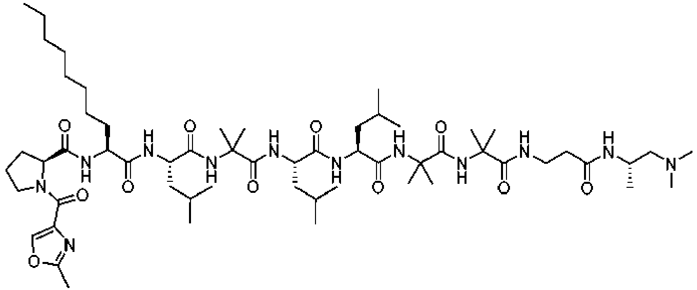
**5910:** 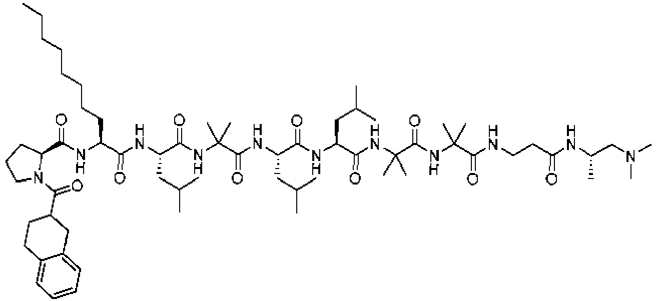
**5911:** 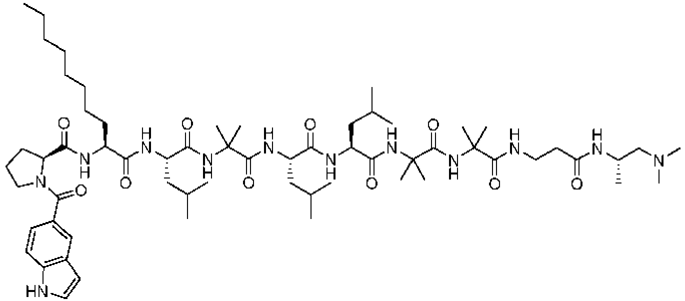
**5912:** 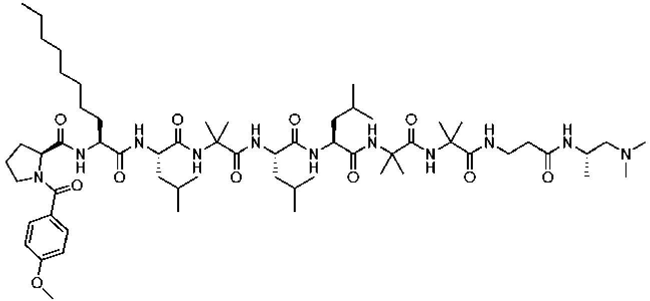 **5934:** 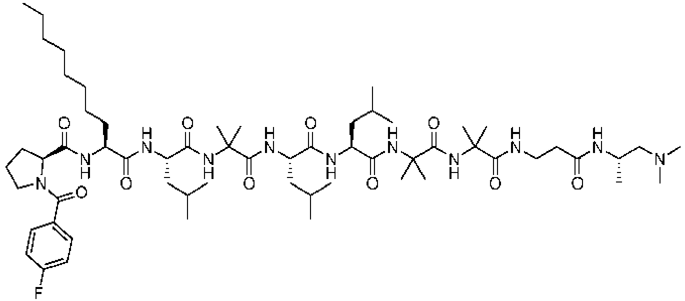
**5936:** 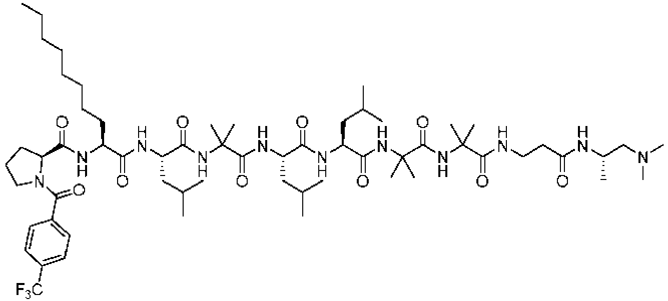
**5937:** 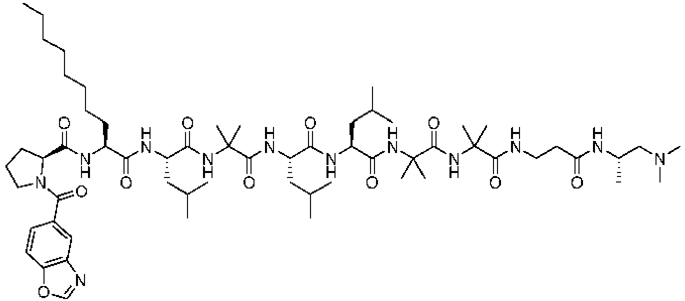
**5938:** 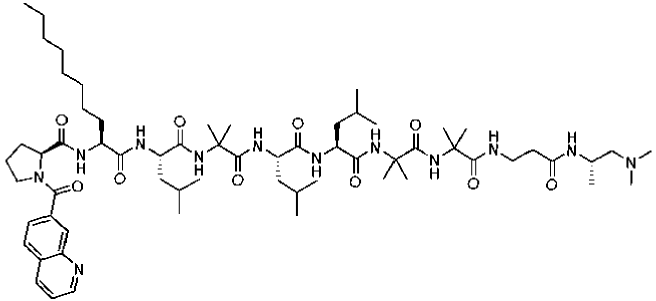
**5939:** 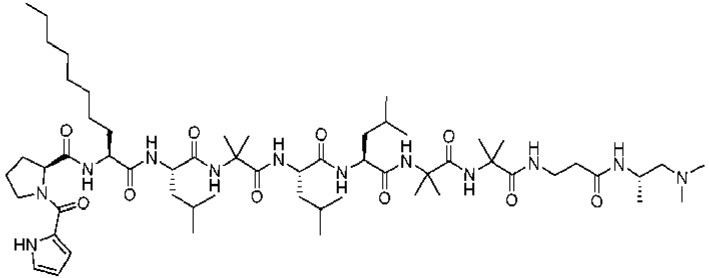
**6025:** 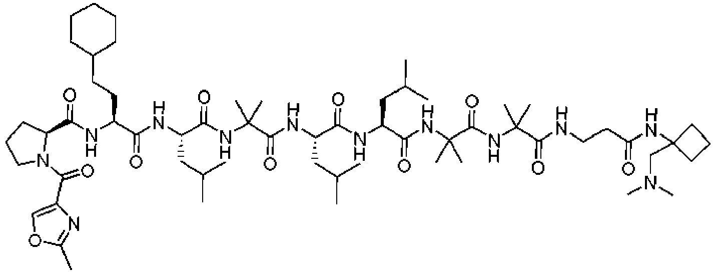
**6026:** 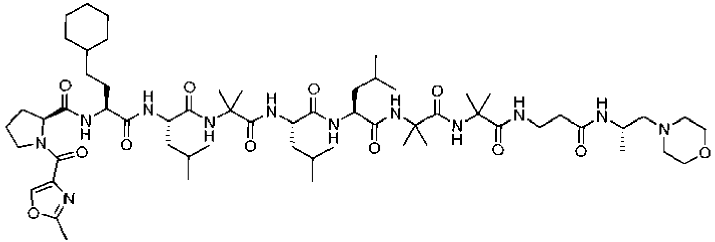
**6027:** 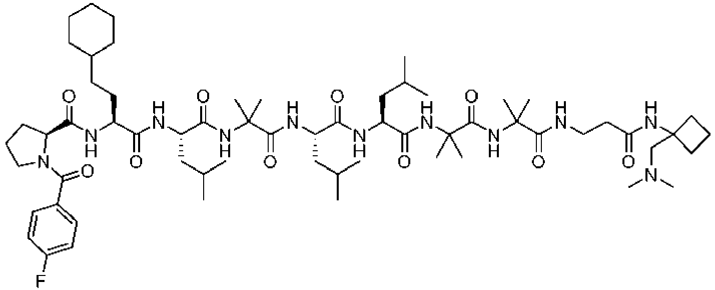
**6028:** 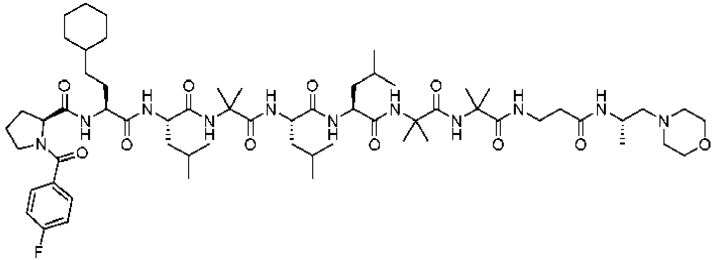
**6253:** 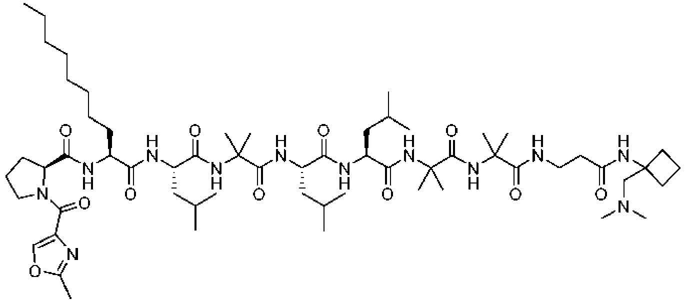
**6254:**

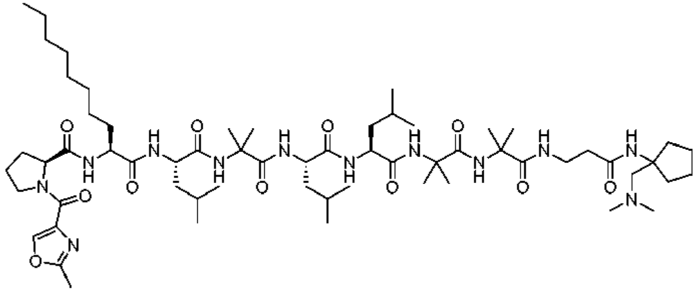

9. Verbindung nach einem der Ansprüche 1 bis 8 zur Verwendung in einem Verfahren zum Behandeln einer Protozoenkrankheit, wobei die Protozoenkrankheit vorzugsweise aus Malaria, humaner afrikanischer Trypanosomiasis, Chagas-Krankheit und Leishmaniose ausgewählt ist, und wobei ferner vorzugsweise die Krankheit aus akuter Malaria, zerebraler Malaria, humaner afrikanischer Trypanosomiasis, Chagas-Krankheit, kutaner Leishmaniose *(Cutaneous leishmaniasis* ― CL), mukokutaner Leishmaniose *(Mucocutaneous leishmaniasis* ― MCL) und viszeraler Leishmaniose *(Visceral leishmaniasis* ― VL) ausgewählt ist.

10. Verbindung zur Verwendung nach Anspruch 9, wobei die Protozoenkrankheit aus der Chagas-Krankheit oder Leishmaniose ausgewählt ist und wobei die Protozoenkrankheit vorzugsweise aus der Chagas-Krankheit, der kutanen Leishmaniose (CL), der mukokutanen Leishmaniose (MCL) und der viszeralen Leishmaniose (VL) ausgewählt ist, und wobei ferner vorzugsweise die Protozoenkrankheit aus der Chagas-Krankheit und der viszeralen Leishmaniose (VL) ausgewählt ist

11. Verbindung nach einem der Ansprüche 1 bis 8 zur Verwendung bei der Behandlung eines Zustands, einer Krankheit oder einer Störung, die durch einen Parasiten verursacht wird, der aus einem Parasiten der *Plasmodium*-Gattung, einem Parasiten der *Trypanosoma-*Gattung oder einem Parasiten der *Leishmania*-Gattung ausgewählt ist; wobei der Parasit vorzugsweise aus *Trypanosoma cruzi, Leishmania donovani, Plasmodium falciparum, Plasmodium vivax, Plasmodium ovale, Plasmodium malaria* ausgewählt ist und wobei der Parasit ferner bevorzugt aus *Trypanosoma cruzi* und *Leishmania donovani* ausgewählt ist.

12. Pharmazeutische Zusammensetzung, die eine Verbindung nach einem der Ansprüche 1 bis 8 und einen pharmazeutisch unbedenklichen Träger oder Hilfsstoff umfasst, wobei die pharmazeutische Zusammensetzung vorzugsweise zur Verwendung bei einer Hemmung der Proliferation eines *Leishmania-Parasiten* bei einem Patienten für eine Prophylaxe oder bei einem Patienten, der die Behandlung von Leishmaniose benötigt, formuliert ist.

## Revendications

1. Composé de formule (I) 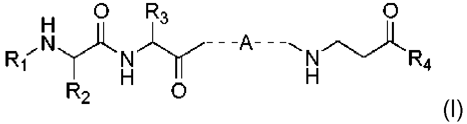 où ledit composé est un composé selon l'une quelconque des formules (II) à (IV) 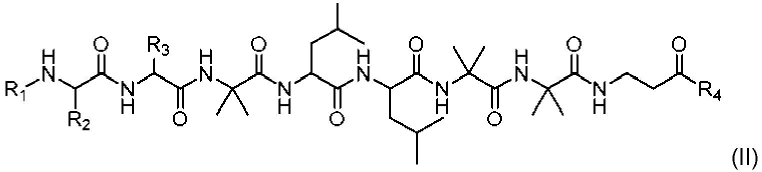 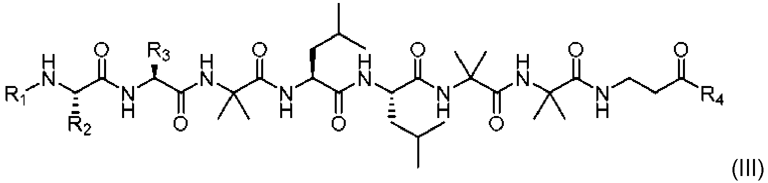 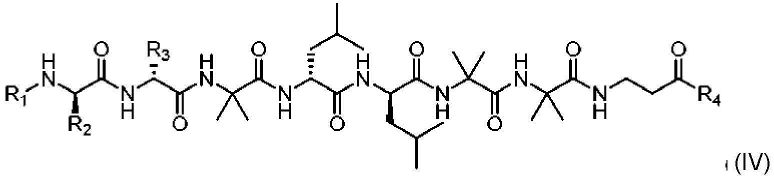 et où
R₁ représente 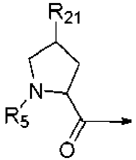 de préférence 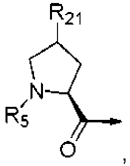 où
R₅ représente C(O)-R₈, où
R₈ représente indépendamment à chaque occurrence choisi parmi le phényle, naphtalène, 1,2-dihydronapthalényle, 1,2,3,4-tétrahydronaphtényle, pyridinyle, pyrazolyle, pyrazinyle, furyle, thiényle, isoxazolyle, oxazolyle, pyrrolyle, quinolinyle, isoquinolinyle, indolyle, pipéridinyle, morpholinyle, pyrrolidinyle, benzimidazolyle, benzofuranyle, benzopyranyle, coumarinyle, pyridazinyle, l'isoindolyle, le benzothiophényle, benzooxazolyle, cyclopentyle, cyclopentényle, cyclohexyle, cyclohexényle, l'adamantanyle ; chacun étant éventuellement substitué par un alkyle en C₁-C₄, un halogène, de préférence le fluor, CF₃, OR₁₀, NR₁₁R₁₂; R₁₀, R₁₁, R₁₂ représentant indépendamment à chaque occurrence H, un alkyle en C₁-C₃ ; et R₂₁ étant choisi parmi H, un alkyle en C₁-C₄ ; et où
la flèche indique la liaison au groupement NH représenté dans la formule (I) ;
R₂ est choisi parmi 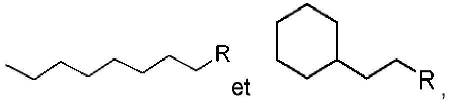 où R indique la liaison au groupement CH représenté dans la formule (I) ;
R₃ est choisi parmi 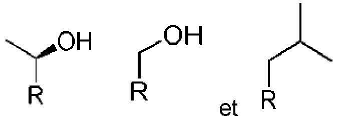 où R indique la liaison au groupement CH représenté dans la formule (I)
R₄ représente 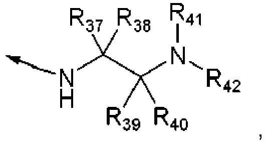 où
R₃₇, R₃₈, R₃₉ et R₄₀ représentent indépendamment à chaque occurrence H ou un alkyle en C₁-C₃, de préférence H ou un méthyle, ou indépendamment à chaque occurrence, deux desdits R₃₇, R₃₈, R₃₉ et R₄₀ ensemble avec l'atome de carbone auquel ils sont liés forment un cycle carbocyclique ou hétérocyclique, de préférence un cycle carbocyclique, et où
R₄₁ et R₄₂ représentent indépendamment l'un de l'autre H ou un alkyle en C₁-C₄ éventuellement substitué par un halogène, un hydroxyle ou un cycloalkyle en C₃-C₆, ; ou conjointement avec l'atome d'azote auquel ils sont fixés, forment indépendamment à chaque occurrence un hétéroaryle ou un hétérocyclyle, chacun indépendamment éventuellement substitué par un halogène, un alkyle en C₁-C₄, OR₄₃, NR₄₄R₄₅ ; où
la flèche indique la liaison au groupement C(O) représenté dans la formule (I) ;
et des sels pharmaceutiquement acceptables dudit composé de formule (I).

2. Composé selon la revendication 1, ledit composé étant un composé de formule (I).

3. Composé selon la revendication 1 ou la revendication 2, ledit R₈ représentant un phényle indépendamment éventuellement substitué par un alkyle en C₁-C₄, un halogène, de préférence un fluor, CF₃, OR₁₀, NR₁₁R₁₂ ; R₁₀, R₁₁, R₁₂ représentant indépendamment à chaque occurrence H, un alkyle en C₁-C₃ ; et R₂₁ étant choisi parmi H, un alkyle en C₁-C₄.

4. Composé selon l'une quelconque des revendications 1 à 3, ledit R₂₁ représentant H.

5. Composé selon la revendication 1, **caractérisé en ce que** ledit R₁ étant choisi parmi 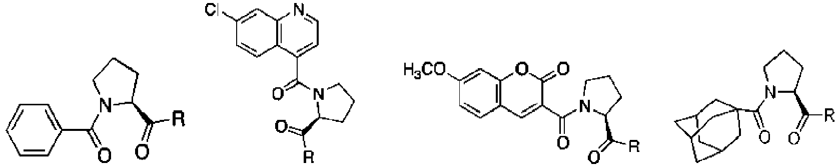 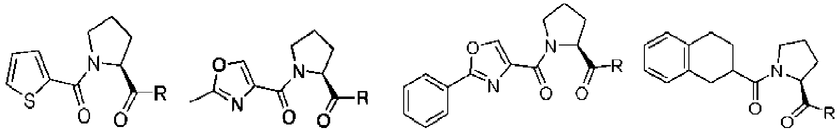 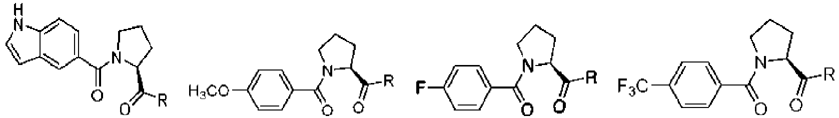 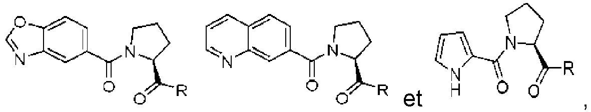 où R indique la liaison au groupement NH représenté dans la formule (I).

6. Composé selon l'une quelconque des revendications 1 à 5, ledit R₄ représentant 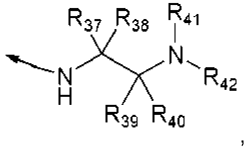 où
R₃₇, R₃₈, R₃₉ et R₄₀ représentent indépendamment à chaque occurrence un hydrogène ou un alkyle en C₁-C₃, de préférence un hydrogène ou un méthyle, ou indépendamment à chaque occurrence, deux desdits R₃₇, R₃₈, R₃₉ et R₄₀ ensemble avec l'atome de carbone auquel ils sont liés forment un cycle monocyclique carbocyclique ou monocyclique hétérocyclique, de préférence un cycle monocyclique carbocyclique, et où
R₄₁ et R₄₂ représentent indépendamment l'un de l'autre un alkyle en C₁-C₄, de préférence un méthyle ; ou conjointement avec l'atome d'azote auquel ils sont fixés, forment indépendamment à chaque occurrence un hétéroaryle monocyclique ou un hétérocyclyle monocyclique, chacun indépendamment éventuellement substitué par un halogène, un alkyle en C₁-C₄, OR₄₃, NR₄₄R₄₅, où R₄₃, R₄₄, R₄₅ représentent indépendamment à chaque occurrence H, un alkyle en C₁-C₄ ; où la flèche indique la liaison au groupement C(O) décrit dans la formule (I).

7. Composé selon l'une quelconque des revendications 1 à 6, ledit R₄ représentant 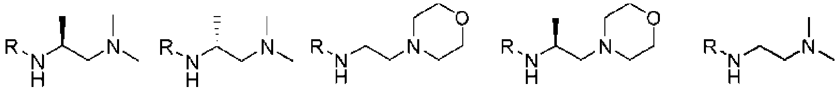 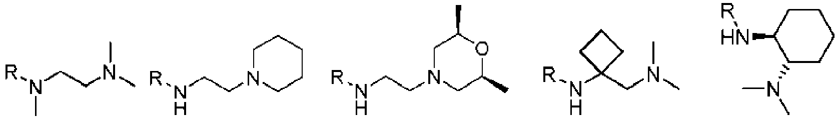 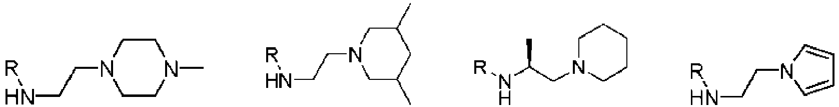 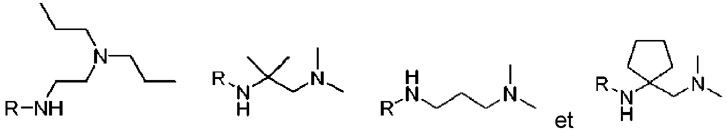 où R indique la liaison au groupement C(O) décrit dans la formule (I).

8. Composé de formule (I) selon la revendication 1, ledit composé étant choisi parmi
**5649** : 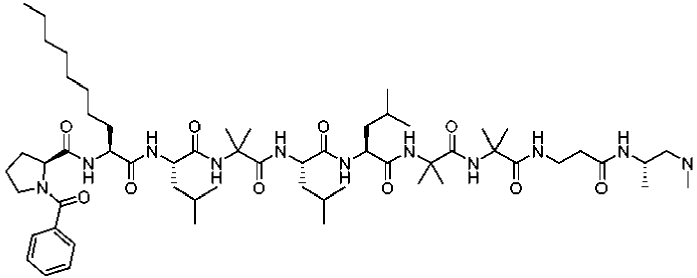
**5768** : 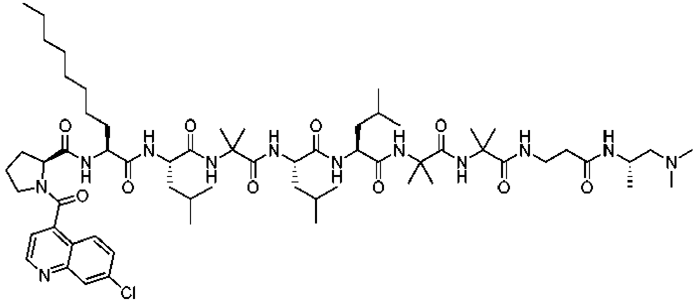
**5769** : 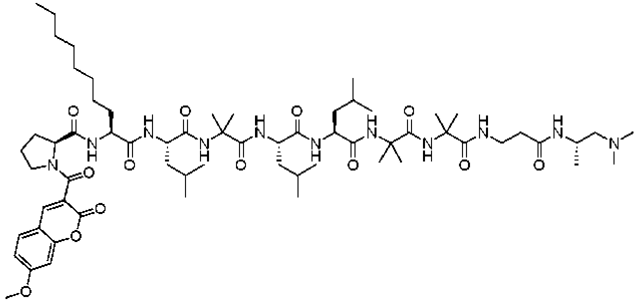
**5770** : 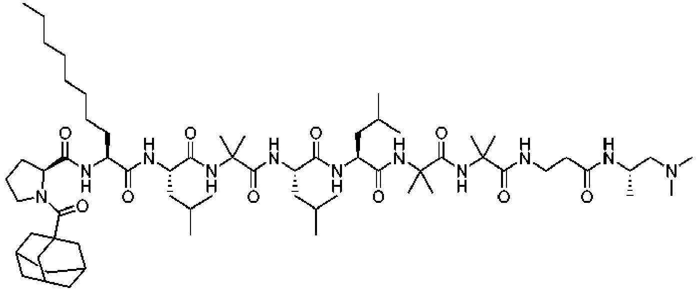
**5904** : 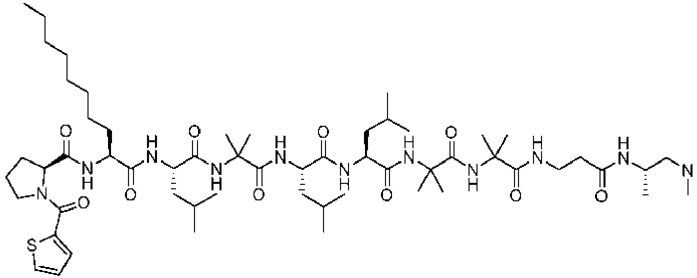
**5905** : 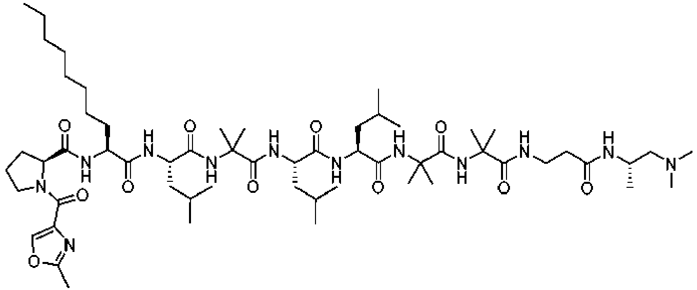
**5910** : 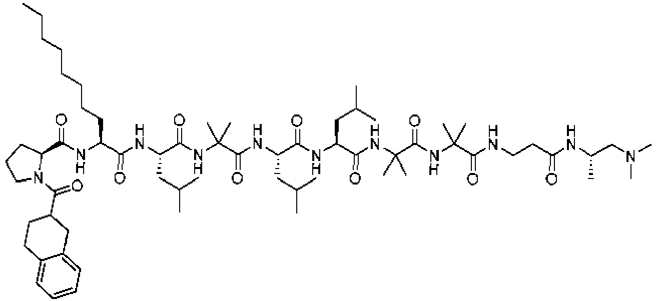
**5911** : 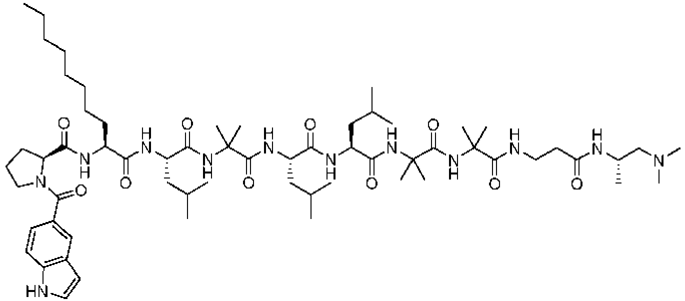
**5912** : 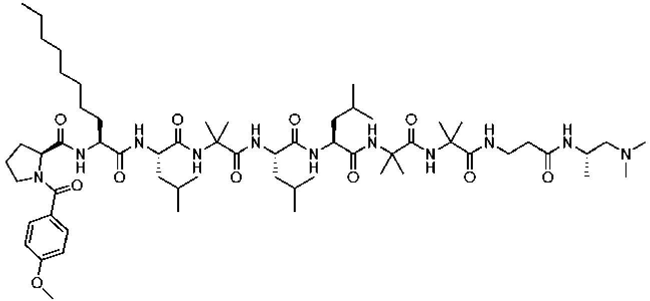 **5934** : 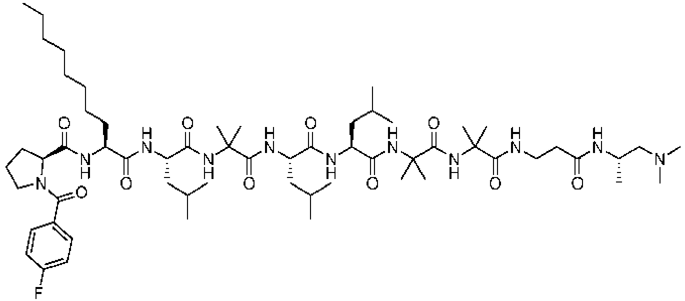
**5936** : 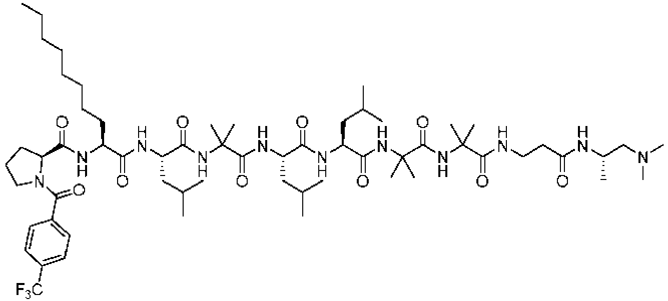
**5937** : 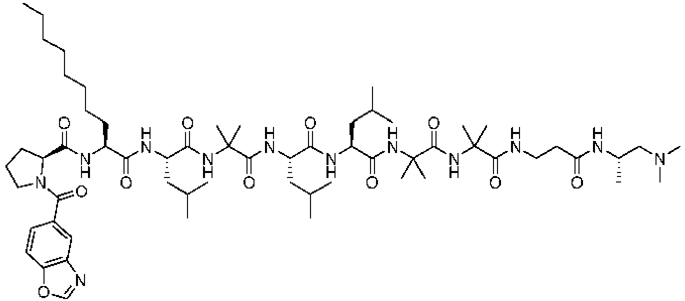
**5938** : 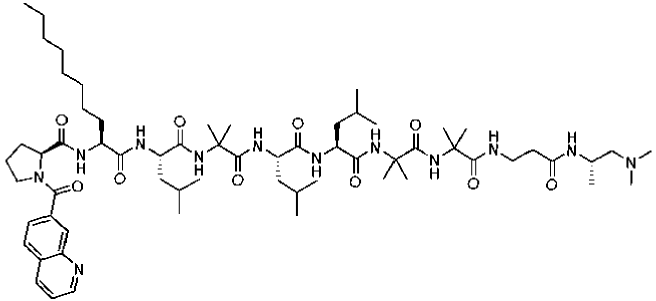
**5939** : 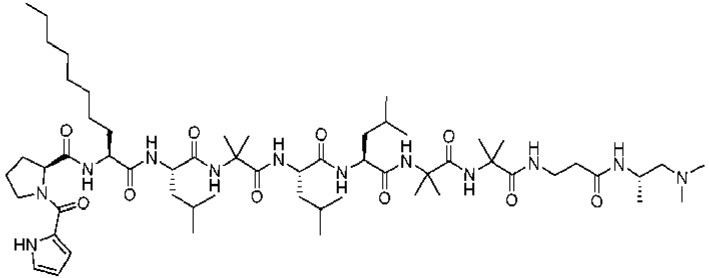
**6025** : 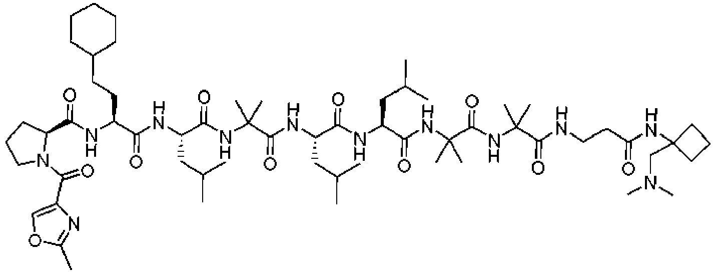
**6026** : 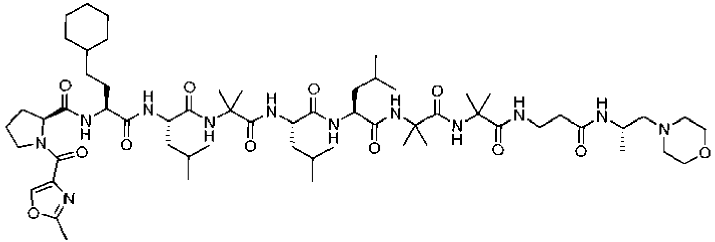
**6027** : 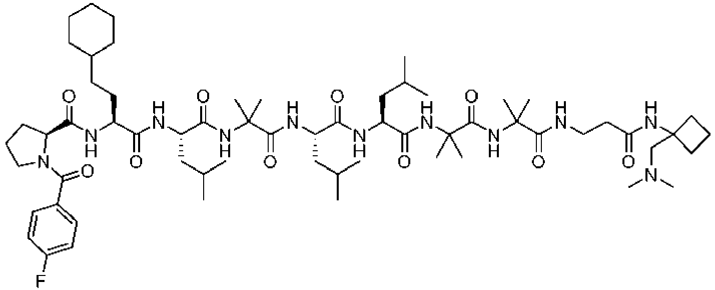
**6028** : 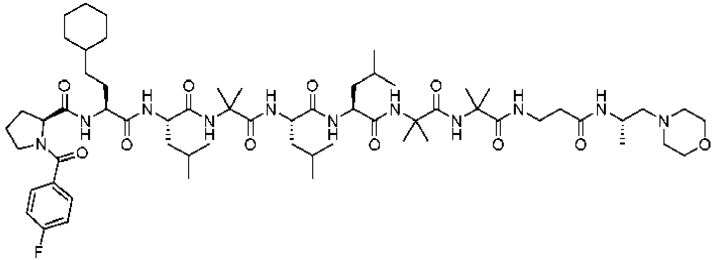
**6253** : 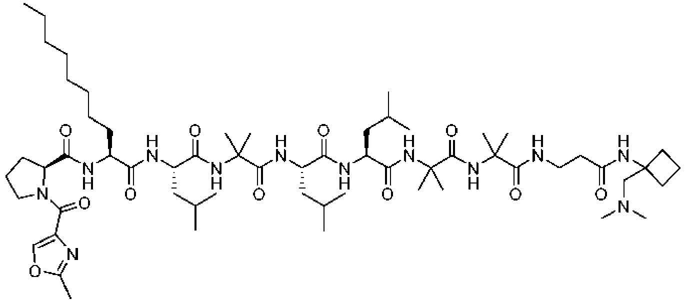
**6254** :

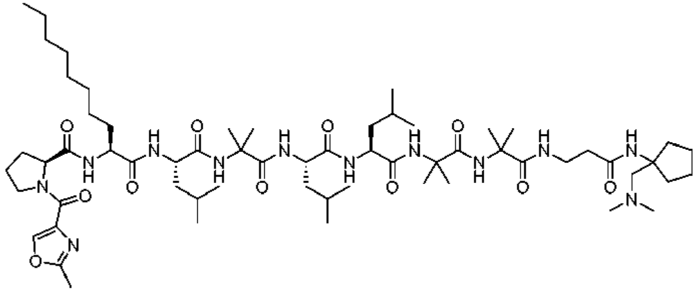

9. Composé selon l'une quelconque des revendications 1 à 8, destiné à être utilisé dans un procédé de traitement d'une protozooze, de préférence ladite protozooze étant choisie parmi le paludisme, la trypanosomiase humaine africaine, la maladie de Chagas et la leishmaniose, et de préférence en outre ladite maladie étant choisie parmi le paludisme aigu, l'accès pernicieux, la trypanosomiase humaine africaine, la maladie de Chagas, la leishmaniose cutanée (LC), la leishmaniose mucocutanée (LMC) et la leishmaniose viscérale (LV).

10. Composé destiné à être utilisé selon la revendication 9, ladite protozooze étant choisie parmi la maladie de Chagas ou la leishmaniose, et de préférence ladite protozooze étant choisie parmi la maladie de Chagas, la leishmaniose cutanée (LC), la leishmaniose mucocutanée (LMC) et la leishmaniose viscérale (LV), et de préférence en outre ladite protozooze étant choisie parmi la maladie de Chagas et la leishmaniose viscérale (LV)

11. Composé selon l'une quelconque des revendications 1 à 8, destiné à être utilisé dans le traitement d'une affection, maladie ou trouble provoqué par un parasite choisi parmi un parasite du genre *Plasmodium,* un parasite du genre *Trypanosoma* ou un parasite du genre *Leishmania,* de préférence ledit parasite étant choisi parmi *Trypanosoma cruzi, Leishmania donovani, Plasmodium falciparum, Plasmodium vivax, Plasmodium ovale, Plasmodium malaria,* et de préférence en outre ledit parasite étant choisi parmi *Trypanosoma cruzi* et *Leishmania donovani.*

12. Composition pharmaceutique comprenant un composé selon l'une quelconque des revendications 1 à 8 et un support ou adjuvant pharmaceutiquement acceptable, ladite composition pharmaceutique étant de préférence formulée pour être utilisée dans l'inhibition de la prolifération d'un parasite *Leishmania* chez un patient pour la prophylaxie ou pour un patient ayant besoin d'un traitement pour la leishmaniose.
